# EUROPEAN PATENT APPLICATION

(11) **EP 4 775 251 A2**
(43) Date of publication of application: **15.07.2026**
(21) Application number: 26181080.8
(22) Date of filing: 08.10.2020
(51) Int. Cl.: A61M 25/00

(54) **SYSTEMS AND METHODS FOR TRICUSPID VALVE TREATMENT**

(30) Priority: 23.10.2019 US 201962925027 P
(62) Divisional of application: 20799913.7
(71) Applicant: Edwards Lifesciences Corporation, Irvine, CA 92614 (US)
(72) Inventor: Landon, David Robert, Irvine, CA, 92614 (US); Scheinblum, Taylor Jacob, Irvine, CA, 92614 (US); Becerra, Matthew Michael, Irvine, CA, 92614 (US); Cooper, Alexander H., Irvine, CA, 92614 (US); Edwards, Jesse Robert, Irvine, CA, 92614 (US); Luong, Hieu Minh, Irvine, CA, 92614 (US); Hauser, David L., Irvine, CA, 92614 (US); Ho, Pui Tong, Irvine, CA, 92614 (US)
(74) Representative: Eisenführ Speiser

(57) **Abstract**

Devices, systems and methods are described herein to provide improved treatment of a tricuspid valve. Such treatment may include tricuspid valve replacement, which may include providing a prosthetic tricuspid valve within the tricuspid valve annulus. Delivery systems for delivering the prosthetic tricuspid valve to the tricuspid valve annulus are disclosed herein. Treatment may also include repair of the tricuspid valve.

## Description

### BACKGROUND

### Field

Certain embodiments disclosed herein relate generally to prostheses for implantation within a lumen or body cavity and delivery systems for a prosthesis. In particular, the prostheses and delivery systems relate in some embodiments to replacement heart valves, such as replacement tricuspid heart valves.

### Background

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life-threatening condition. For example, valve insufficiency can lead to conditions such as heart hypertrophy and dilation of the ventricle. Thus, extensive efforts have been made to develop methods and apparatuses to repair or replace impaired heart valves.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner.

Delivering a prosthesis to a desired location in the human body, for example delivering a replacement heart valve to the tricuspid valve, can also be challenging. Obtaining access to perform procedures in the heart or in other anatomical locations may require delivery of devices percutaneously through tortuous vasculature or through open or semi-open surgical procedures. The ability to control the deployment of the prosthesis at the desired location can also be challenging.

### SUMMARY

Embodiments of the present disclosure are directed to a prosthesis, such as but not limited to a replacement heart valve. Embodiments of the present disclosure may also be directed to delivery systems, devices and/or methods of use to deliver and/or controllably deploy a prosthesis, such as but not limited to a replacement heart valve, to a desired location within the body. In some embodiments, a replacement heart valve and methods for delivering a replacement heart valve to a native heart valve, such as a tricuspid valve, are provided.

In some embodiments, a delivery system and method are provided for delivering a replacement heart valve to a native tricuspid valve location. In some embodiments, components of the delivery system facilitate bending of the delivery system to steer a prosthesis within a right atrium to a location within the native tricuspid valve. In some embodiments, a capsule is provided for containing the prosthesis for delivery to the native tricuspid valve location. In other embodiments, the delivery system and method may be adapted for delivery of implants to locations other than the native tricuspid valve.

The present disclosure includes, but is not limited to, the following embodiments.

A delivery system for an implant, the delivery system including an elongate shaft having a distal end, an implant retention area for retaining the implant, a bend portion configured to deflect the distal end of the elongate shaft to a first direction, and a portion positioned proximal of the bend portion. A deflection mechanism is configured to deflect the portion that is positioned proximal of the bend portion to deflect the bend portion towards a second direction that is opposed to the first direction.

A delivery system for an implant, the delivery system including an elongate shaft having a distal end, an implant retention area for retaining the implant, a bend portion configured to deflect the distal end of the elongate shaft in a first plane, and a portion positioned proximal of the bend portion. A deflection mechanism is configured to deflect the portion that is positioned proximal of the bend portion in one or more planes that are not perpendicular to the first plane.

A delivery system for an implant, the delivery system including an elongate shaft having a distal end, an implant retention area for retaining the implant, a first bend portion configured to deflect the distal end of the elongate shaft to a first direction, a second bend portion positioned proximate of the first bend portion and configured to deflect the distal end of the elongate shaft to a second direction, and a portion positioned proximal of the second bend portion. A deflection mechanism may be configured to deflect the first bend portion and the second bend portion and the portion that is positioned proximal of the second bend portion.

A delivery system for an implant, the delivery system including an elongate shaft having an implant retention area for retaining the implant, and a capsule having a distal end and surrounding the implant retention area, and the distal end of the capsule forming a distal tip of the elongate shaft.

A delivery system for an implant, the delivery system including an elongate shaft having an implant retention area for retaining the implant, and a distal tip including a flexible sheath extending distally and configured to bend about a portion of a guide wire.

A delivery system for an implant, the delivery system including an elongate shaft having an implant retention area for retaining the implant, and a distal tip having a dome shape or a parabolic shape.

A delivery system for an implant, the delivery system including an elongate shaft having a wall surrounding a channel for the implant to be passed through for deployment of the implant, the wall configured to have a bend defining a bend in the channel during deployment of the implant.

A delivery system for an implant, the delivery system including an elongate shaft having an axial dimension and having an implant retention area for retaining the implant, and a port for the implant to be deployed from the elongate shaft in a direction transverse to the axial dimension.

A delivery system for an implant, the delivery system including an elongate shaft having an implant retention area for retaining the implant, the elongate shaft configured to bend more than 180 degrees to form a loop.

A delivery system for an implant, the delivery system including an elongate shaft having a capsule surrounding an implant retention area for retaining the implant, and a hinge coupling the capsule to a portion of the elongate shaft.

A delivery system for an implant, the delivery system including an elongate shaft extending along an axis and having an outer surface and an implant retention area for retaining the implant. One or more support bodies may be configured to extend radially outward from the outer surface of the elongate shaft and contact an external surface to resist deflection of the elongate shaft transverse to the axis.

A system including a prosthetic heart valve configured for implantation within a patient's valve annulus. The system includes an anchor configured to be secured within a portion of the patient's body. The system includes a tether configured to couple the prosthetic heart valve to the anchor.

A prosthetic valve for replacement of a patient's native valve, the prosthetic valve including a prosthetic heart valve body configured to be anchored within an annulus of the patient's native valve and forming a prosthetic valve annulus. The system includes a port coupled to the prosthetic heart valve body and configured to receive a diagnostic or therapeutic device.

A method for treating a patient's tricuspid valve, the method including passing a delivery apparatus for an implant into the patient's right atrium. The method including deploying the implant to the patient's tricuspid valve.

A method for treating a patient's tricuspid valve, the method including deploying a prosthetic heart valve within a patient's tricuspid valve annulus. The method including deploying an anchor to a portion within a patient's body. The method including providing a tether coupling the prosthetic heart valve to the anchor.

A method including passing a diagnostic or therapeutic device through a port positioned on a prosthetic heart valve body, the prosthetic heart valve body forming a prosthetic valve annulus.

A method including coupling a pacemaker pacing lead to a prosthetic heart valve body positioned within a patient's heart valve annulus to provide electrical energy through the pacemaker pacing lead and through the prosthetic heart valve body to pace functioning of the patient's heart.

A method including delivering a delivery apparatus for an implant into a portion of a patient's heart, the delivery apparatus including an elongate shaft extending along an axis and having an outer surface. The method including expanding one or more support bodies radially outward from the outer surface of the elongate shaft. The method including contacting the one or more support bodies to a surface external of the delivery apparatus to resist deflection of the elongate shaft transverse to the axis.

### BRIEF DESCRIPTION OF THE DRAWINGS

FIG. 1 shows an embodiment of a delivery system.
FIG. 2A shows a partial cross-sectional view of the distal end of the delivery system of FIG. 1 loaded with the valve prosthesis of FIG. 3A.
FIG. 2B shows a partial cross-sectional view of the distal end of the delivery system of FIG. 1 without the valve prosthesis of FIG. 3A.
FIG. 2C shows a partial cross-sectional view of the distal end of the delivery system of FIG. 1 with certain shaft assemblies translated along the rail assembly.
FIG. 3A shows a side view of an embodiment of a valve prosthesis that may be delivered using the delivery systems described herein.
FIG. 3B shows a side view of an embodiment of an aortic valve prosthesis that may be delivered using the delivery systems described herein.
FIG. 4 shows a perspective view of the distal end of the delivery system of FIG. 1.
FIG. 5 shows components of the delivery system of FIG. 4 with the outer sheath assembly moved proximally and out of view.
FIG. 6A shows components of the delivery system of FIG. 5 with the mid shaft assembly moved proximally and out of view.
FIG. 6B illustrates a cross-section of the rail assembly.
FIG. 6C illustrates a cross-section of an embodiment of the rail assembly.
FIG. 7 shows components of a delivery system.
FIG. 8 shows components of the delivery system of FIG. 7 with the inner assembly moved proximally and out of view.
FIG. 9 illustrates an embodiment of a rail assembly.
FIG. 10 illustrates an embodiment of a delivery system handle.
FIG. 11 illustrates a cross-section of the delivery system handle of FIG. 10.
FIG. 12A illustrates a side view of a distal end of an elongate shaft.
FIG. 12B illustrates a side view of the distal end of the elongate shaft deflected from the position shown in FIG. 12A.
FIG. 12C illustrate a top view of the distal end of the elongate shaft deflected from the position shown in FIG. 12A.
FIG. 13A illustrates a side view of a distal end of an elongate shaft.
FIG. 13B illustrates a side view of the distal end of the elongate shaft deflected from the position shown in FIG. 13A.
FIG. 13C illustrate a top view of the distal end of the elongate shaft deflected from the position shown in FIG. 13A.
FIG. 13D illustrates a front view of the distal end of the elongate shaft deflected from the position shown in FIG. 13A.
FIG. 14A illustrates a perspective view of a deflection mechanism positioned upon an elongate shaft.
FIG. 14B illustrates a side view of a distal end of an elongate shaft.
FIG. 14C illustrates a side view of the distal end of the elongate shaft deflected from the position shown in FIG. 14B.
FIG. 14D illustrate a top view of the distal end of the elongate shaft deflected from the position shown in FIG. 14B.
FIG. 15A illustrates a side view of a distal end of an elongate shaft.
FIG. 15B illustrates a side view of the distal end of the elongate shaft deflected from the position shown in FIG. 15A.
FIG. 15C illustrate a top view of the distal end of the elongate shaft deflected from the position shown in FIG. 15A.
FIG. 16A illustrates a side view of a distal end of an elongate shaft.
FIG. 16B illustrates a side view of the distal end of the elongate shaft deflected from the position shown in FIG. 16A.
FIG. 16C illustrate a top view of the distal end of the elongate shaft deflected from the position shown in FIG. 16A.
FIG. 17 illustrates a perspective view of a rail assembly having a pull tether positioned thereon.
FIG. 18A illustrates a perspective view of a rail assembly having cuts positioned on a tube of the rail assembly.
FIG. 18B illustrates a cross sectional view of the rail assembly having stoppers positioned therein.
FIG. 19A illustrates a side view of the distal end of the elongate shaft.
FIG. 19B illustrate a top view of the distal end of the elongate shaft shown in FIG. 19A.
FIG. 20A illustrates a representation of an elongate shaft entering a right atrium of a patient's heart.
FIG. 20B illustrates a distal end of the elongate shaft shown in FIG. 20A being deflected from the position shown in FIG. 20A.
FIG. 20C illustrates a distal end of the elongate shaft shown in FIG. 20B being deflected from the position shown in FIG. 20B.
FIG. 21 illustrates a representation of an elongate shaft entering a right atrium of a patient's heart from the superior vena cava.
FIG. 22A illustrates a perspective view of an implant being deployed from an elongate shaft.
FIG. 22B illustrates a perspective view of an implant being deployed from an elongate shaft.
FIG. 22C illustrates a perspective view of an implant being deployed from an elongate shaft.
FIG. 23 illustrates an implant in position within a tricuspid valve annulus.
FIG. 24 illustrates an embodiment of a tip of an elongate shaft.
FIG. 25A illustrates an embodiment of a tip of an elongate shaft.
FIG. 25B illustrates a view of a capsule being positioned within a right ventricle of a patient's heart.
FIG. 26 illustrates an embodiment of a tip of an elongate shaft.
FIG. 27 illustrates an embodiment of a tip of an elongate shaft.
FIG. 28 illustrates an embodiment of a tip of an elongate shaft.
FIG. 29 illustrates a view of a flexible implant being positioned within an elongate shaft having a bend.
FIG. 30 illustrates a view of an implant configured to be deployed from a port in a side of an elongate shaft.
FIG. 31 illustrates a view of an elongate shaft having a loop.
FIG. 32A illustrates a view of an elongate shaft having a hinge.
FIG. 32B illustrates a view of the elongate shaft shown in FIG. 32A with a capsule rotated from the position shown in FIG. 32A.
FIG. 33A illustrates a view of an elongate shaft having a hinge.
FIG. 33B illustrates a view of the elongate shaft shown in FIG. 33A with a capsule rotated from the position shown in FIG. 33A.
FIG. 34A illustrates a view of an elongate shaft within a patient's right atrium.
FIG. 34B illustrates a view of the elongate shaft shown in FIG. 34A translated from the position shown in FIG. 34A.
FIG. 35 illustrates a view of an implant within a patient's tricuspid valve annulus with an anchor positioned in the inferior vena cava.
FIG. 36A illustrates a view of an implant within a patient's tricuspid valve annulus with an anchor coupled to the moderator band of the right ventricle.
FIG. 36B illustrates a perspective view of an anchor for coupling to the moderator band.
FIG. 36C illustrates a perspective view of an anchor for coupling to the moderator band.
FIG. 36D illustrates a perspective view of an anchor for coupling to the moderator band.
FIG. 36E illustrates a perspective view of an anchor for coupling to the moderator band.
FIG. 36F illustrates a perspective view of an anchor for coupling to the moderator band.
FIG. 37 illustrates a view of an implant within a patient's tricuspid valve annulus with an anchor coupled to a wall of the right ventricle.
FIG. 38A illustrates a view of an implant within a patient's right atrium with an anchor coupled to a wall of the right ventricle.
FIG. 38B illustrates a view of the implant of FIG. 38A within the patient's tricuspid valve annulus.
FIG. 39A illustrates a side schematic view of an implant including a port for receiving a diagnostic or therapeutic device.
FIG. 39B illustrates a side schematic view of an implant including a port for receiving a diagnostic or therapeutic device.
FIG. 39C illustrates a side perspective view of an implant including a port for receiving a diagnostic or therapeutic device.
FIG. 39D illustrates a bottom view of an implant including a port for receiving a diagnostic or therapeutic device.
FIG. 39E illustrates a side perspective view of a port for receiving a diagnostic or therapeutic device.
FIG. 39F illustrates a side perspective view of a port for receiving a diagnostic or therapeutic device.
FIG. 39G illustrates a side perspective view of a port for receiving a diagnostic or therapeutic device.
FIG. 40A illustrates a side schematic view of an implant including a port for receiving a diagnostic or therapeutic device.
FIG. 40B illustrates a side perspective view of an implant including a port for receiving a diagnostic or therapeutic device.
FIG. 41 illustrates a side perspective view of an implant including a port for receiving a diagnostic or therapeutic device.
FIG. 42 illustrates a view of an implant including a port for receiving a diagnostic or therapeutic device in position within the tricuspid valve annulus.
FIG. 43 illustrates a view of an implant including a port for receiving a diagnostic or therapeutic device in position within the tricuspid valve annulus.
FIG.44 illustrates a view of an implant including a port for coupling to a pacemaker pacing lead.
FIG. 45 illustrates a perspective view of a delivery system.
FIG. 46 illustrates a schematic view of the handle of the delivery system shown in FIG. 45.
FIG. 47 illustrates a front plan view of an embodiment of an adaptor.
FIG. 48 illustrates a side perspective view of an embodiment of an adaptor and drive rods.
FIG. 49 illustrates a perspective view of the handle shown in FIG. 45.
FIG. 50 illustrates a perspective view of a proximal portion of the handle shown in FIG. 45.
FIG. 51 illustrates a side perspective view of insertion of a delivery apparatus into a patient's body.
FIG. 52 illustrates a perspective view of an embodiment of a distal end of an elongate sheath.
FIG. 53 illustrates a perspective view of an embodiment of a distal end of an elongate sheath.
FIG. 54 illustrates a cross sectional view of a capsule of the elongate sheath shown in FIG. 53.
FIG. 55 illustrates a side schematic view of the elongate sheath shown in FIG. 53 deploying an implant to a tricuspid heart valve.
FIG. 56 illustrates a view of an elongate shaft approaching a tricuspid valve.
FIG. 57 illustrates a view of the elongate shaft shown in FIG. 56 deflected in position.
FIG. 58 illustrates a view of an implant deployed to the tricuspid valve.
FIG. 59 illustrates a perspective view of an embodiment of a control device and an output device.
FIG. 60 illustrates a perspective view of an embodiment of a control device and an output device.
FIG. 61 illustrates a cross section view of an embodiment of a delivery system handle.
FIG. 62A illustrates a side view of a distal end of an elongate shaft.
FIG. 62B illustrates a side view of the elongate shaft shown in FIG. 62A, with support bodies deployed.
FIG. 62C illustrates a view of the elongate shaft shown in FIG. 62A approaching a mitral valve.
FIG. 62D illustrates a view of the elongate shaft shown in FIG. 62A approaching a mitral valve.
FIG. 62E illustrates a view of the elongate shaft shown in FIG. 62A approaching a mitral valve, with support bodies deployed.
FIG. 62F illustrates a view of an elongate shaft approaching a tricuspid valve, with support bodies deployed.
FIG. 63A illustrates a view of an elongate shaft approaching a mitral valve.
FIG. 63B illustrates a view of the elongate shaft shown in FIG. 63A approaching a mitral valve, with a support body deployed.
FIG. 64A illustrates a perspective view of a support body.
FIG. 64B illustrates a view of an elongate shaft approaching a mitral valve.
FIG. 64C illustrates a view of the elongate shaft shown in FIG. 64B approaching a mitral valve, with a support body deployed.

### DETAILED DESCRIPTION

The present specification and drawings provide aspects and features of the disclosure in the context of several embodiments of replacement heart valves, delivery systems and methods that are configured for use in the vasculature of a patient, such as for replacement or repair of natural heart valves in a patient. These embodiments may be discussed in connection with replacing specific valves such as the patient's aortic, tricuspid, mitral, or pulmonary valve. However, it is to be understood that the features and concepts discussed herein can be applied to devices other than heart valve implants. For example, the delivery systems, replacement heart valves, and methods can be applied to medical implants, for example other types of expandable prostheses, for use elsewhere in the body, such as within an artery, a vein, or other body cavities or locations. In addition, specific features of a valve, delivery system, method, etc. should not be taken as limiting, and features of any one embodiment discussed herein can be combined with features of other embodiments as desired and when appropriate. While certain of the embodiments described herein are described in connection with a transfemoral delivery approach, it should be understood that these embodiments can be used for other delivery approaches such as, for example, transapical, transatrial, or transjugular approaches. Moreover, it should be understood that certain of the features described in connection with certain embodiments can be incorporated with other embodiments, including those that are described in connection with different delivery approaches.

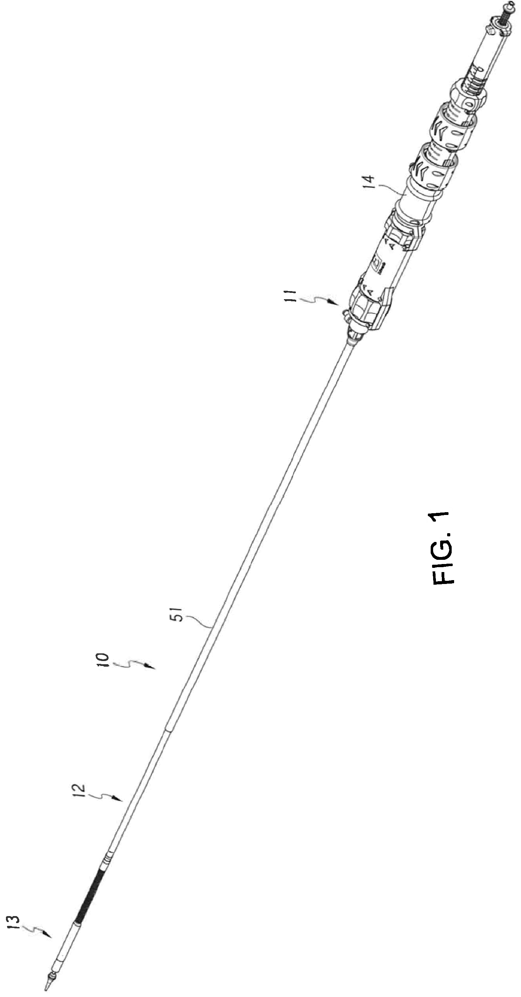

FIG. 1 illustrates an embodiment of a delivery device, assembly, or system **10.** The delivery system **10** can be used to deploy a prosthesis, such as a replacement heart valve, within the body. In some embodiments, the delivery system **10** can use a dual plane deflection approach to properly deliver the prosthesis. Replacement heart valves can be delivered to a patient's tricuspid heart valve annulus or other heart valve location in various manners, such as by open surgery, minimally-invasive surgery, and percutaneous or transcatheter delivery through the patient's vasculature. Example transfemoral approaches may be found in U.S. Pat. Pub. No. 2015/0238315, filed February 20, 2015, the entirety of which is hereby incorporated by reference in its entirety. While the delivery system **10** is described in connection with a percutaneous delivery approach, and more specifically a transfemoral delivery approach, it should be understood that features of delivery system **10** can be applied to other delivery system, including delivery systems for a transapical, transatrial, or transjugular delivery approach.

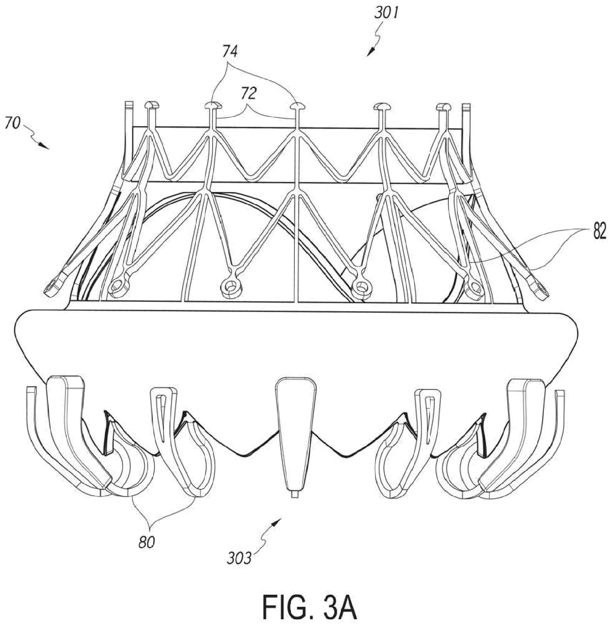

The delivery system **10** may be used to deploy a prosthesis, such as a replacement heart valve as described elsewhere in this specification, within the body. The delivery system **10** can receive and/or cover portions of the prosthesis such as a first end **301** and second end **303** of the prosthesis or implant **70** illustrated in FIG. 3A. For example, the delivery system **10** may be used to deliver an expandable prosthesis or implant **70,** where the implant **70** includes the first end **301** and the second end **303,** and wherein the second end **303** is configured to be deployed or expanded before the first end **301.**

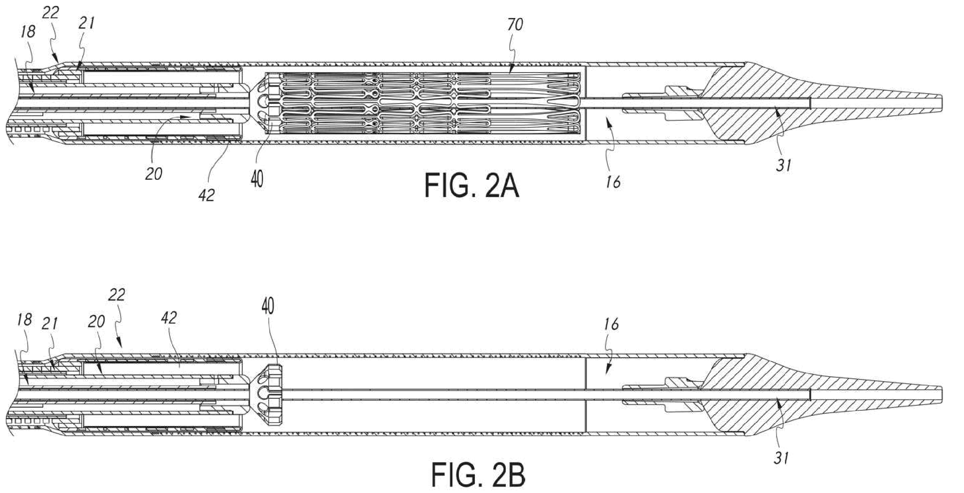

FIG. 2A further shows an example of the implant **70** that can be inserted into the delivery system **10,** specifically into the implant retention area **16.** For ease of understanding, in FIG. 2A, the prosthesis is shown with only the bare metal frame illustrated. The prosthesis or implant **70** can take any number of different forms. A particular example of frame for a prosthesis is shown in FIG. 3A, though it will be understood that other designs and frame configurations may also be used, including those disclosed in this application. The implant **70** can include one or more sets of anchors, such as distal (or ventricular) anchors **80** extending proximally when the prosthesis frame is in an expanded configuration and proximal (or atrial) anchors **82** extending distally when the prosthesis frame is in an expanded configuration. The prosthesis can further include struts **72** which may end in mushroom-shaped tabs **74** at the first end **301.** Further discussion can be found in U.S. Publication No. 2015/0328000A1, published November 19, 2015, hereby incorporated by reference in its entirety.

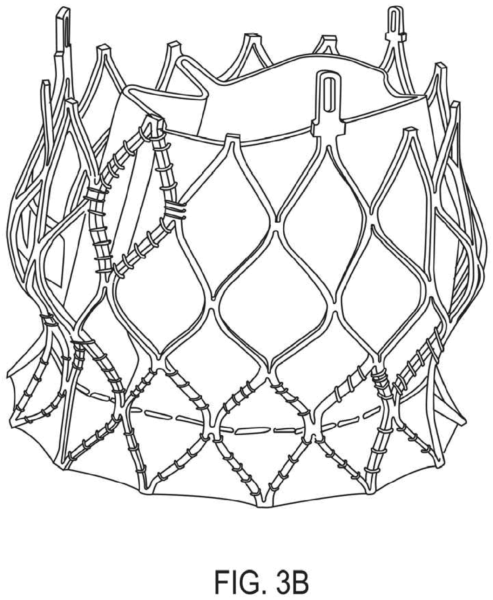

In some embodiments, the delivery system **10** can be used in conjunction with a replacement aortic valve, such as shown in FIG. 3B. In some embodiments the delivery system **10** can be modified to support and delivery the replacement aortic valve. However, the procedures and structures discussed below can similarly be used for a replacement tricuspid and replacement aortic valve.

Additional details and example designs for a prosthesis are described in U.S. Patent Nos. 8,403,983, 8,414,644, 8,652,203 and U.S. Patent Publication Nos. 2011/0313515, 2012/0215303, 2014/0277390, 2014/0277422, 2014/0277427, 2018/0021129, and 2018/0055629, the entirety of these patents and publications are hereby incorporated by reference and made a part of this specification. Further details and embodiments of a replacement heart valve or prosthesis and its method of implantation are described in U.S. Publication Nos. 2015/0328000 and 2016/0317301 the entirety of each of which is hereby incorporated by reference and made a part of this specification.

The delivery system **10** can be relatively flexible. In some embodiments, the delivery system 10 is particularly suitable for delivering a replacement heart valve to a mitral valve location through a transseptal approach (e.g., between the right atrium and left atrium via a transseptal puncture). The delivery system **10,** however, may be suitable for delivering a replacement heart valve to a tricuspid valve location, among other locations.

As shown in FIG. 1, the delivery system **10** can include a shaft assembly or elongate shaft **12** comprising a proximal end **11** and a distal end **13,** wherein a handle **14** is coupled to the proximal end of the elongate shaft **12.** The elongate shaft **12** can be used to hold the implant **70** for advancement of the same through the vasculature to a treatment location. The delivery system **10** can further comprise a relatively rigid live-on (or integrated) sheath **51** surrounding the elongate shaft **12** that can prevent unwanted motion of the elongate shaft **12.** The live-on sheath **51** can be attached at a proximal end of elongate shaft **12** proximal to the handle **14,** for example at a sheath hub. The elongate shaft **12** can include an implant retention area **16** (shown in FIGS. 2A-2B with FIG. 2A showing the implant **70** and FIG. 2B with the implant **70** removed) at its distal end that can be used for this purpose. In some embodiments, the elongate shaft **12** can hold an expandable prosthesis in a compressed state at implant retention area **16** for advancement of the implant **70** within the body. The elongate shaft **12** may then be used to allow controlled expansion of the implant **70** at the treatment location. In some embodiments, the elongate shaft **12** may be used to allow for sequential controlled expansion of the implant **70** as discussed in detail below. The implant retention area **16** is shown in FIGS. 2A-B at the distal end of the delivery system **10,** but may also be at other locations. In some embodiments, the implant **70** may be rotated in the implant retention area **16,** such as through the rotation of the inner shaft assembly **18** discussed herein.

As shown in cross-sectional view of FIGS. 2A-2B, the distal end of the delivery system **10** can include one or more subassemblies such as an outer sheath assembly **22¸** a mid shaft assembly **21,** a rail assembly **20,** an inner shaft assembly **18,** and a nose cone assembly **31** as will be described in more detail below. In some embodiments, the delivery system **10** may not have all of the assemblies disclosed herein. For example, in some embodiments a full mid shaft assembly may not be incorporated into the delivery system **10.** In some embodiments, the assemblies disclosed below may be in a different radial order than is discussed.

In particular, embodiments of the disclosed delivery system **10** can utilize a steerable rail in the rail assembly **20** for steering the distal end of the delivery system **10,** allowing the implant to be properly located in a patient's body. As discussed in detail below, the steerable rail can be, for example, a rail shaft that extends through the delivery system **10** from the handle **14** generally to the distal end. In some embodiments, the steerable rail has a distal end that ends proximal to the implant retention area **16.** A user can manipulate the bending of the distal end of the rail, thereby bending the rail in a particular direction. In preferred embodiments, the rail has more than one bend along its length, thereby providing multiple directions of bending. As the rail is bent, it presses against the other assemblies to bend them as well, and thus the other assemblies of the delivery system **10** can be configured to steer along with the rail as a cooperating single unit, thus providing for full steerability of the distal end of the delivery system.

Once the rail is steered into a particular location in a patient's body, the implant **70** can be advanced along or relative to the rail through the movement of the other sheaths/shafts relative to the rail and released into the body. For example, the rail can be bent into a desired position within the body, such as to direct the implant **70** towards the native mitral valve. The other assemblies (e.g., the outer sheath assembly **22,** the mid shaft assembly **21,** the inner assembly **18,** and the nose cone assembly **31**) can passively follow the bends of the rail. Further, the other assemblies (e.g., the outer sheath assembly **22,** the mid shaft assembly **21,** the inner assembly **18,** and the nose cone assembly **31**) can be advanced together (e.g., relatively together, sequentially with one actuator, simultaneously, almost simultaneously, at the same time, closely at the same time) relative to the rail while maintaining the implant **70** in the compressed position without releasing or expanding the implant **70** (e.g., within the implant retention area **16**)**.** The other assemblies (e.g., the outer sheath assembly **22,** the mid shaft assembly **21,** the inner assembly **18,** and the nose cone assembly **31**) can be advanced distally or proximally together relative to the rail. In some embodiments, only the outer sheath assembly **22,** mid shaft assembly **21,** and inner assembly **18** are advanced together over the rail. Thus, the nose cone assembly **31** may remain in the same position. The assemblies can be individually, sequentially, or simultaneously, translated relative to the inner assembly **18** in order to release the implant **70** from the implant retention area **16.**

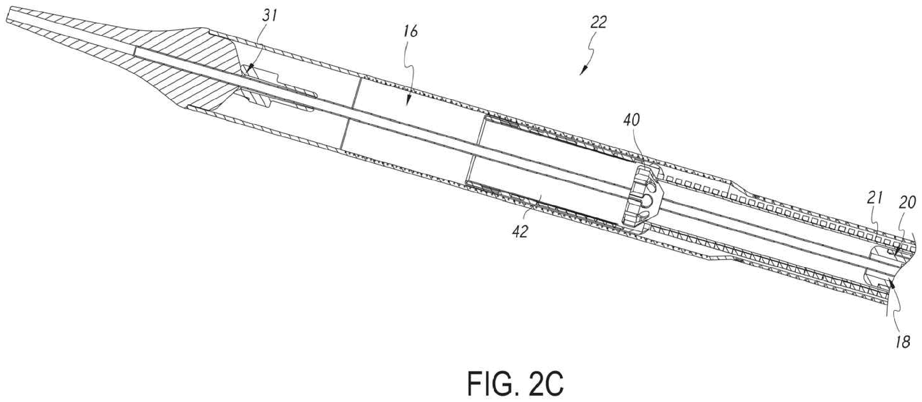

FIG. 2C illustrates the sheath assemblies, specifically the outer sheath assembly **22,** the mid shaft assembly **21,** the inner shaft assembly **18,** and the nose cone assembly **31** having translated distally together along the rail assembly **20,** further details on the assemblies are below. In some embodiments, the outer sheath assembly **22,** the mid shaft assembly **21,** the inner shaft assembly **18,** and the nose cone assembly **31** translate together (e.g., relatively together, sequentially with one actuator, simultaneously, almost simultaneously, at the same time, closely at the same time). This distal translation can occur while the implant **70** remains in a compressed configuration within the implant retention area **16.**

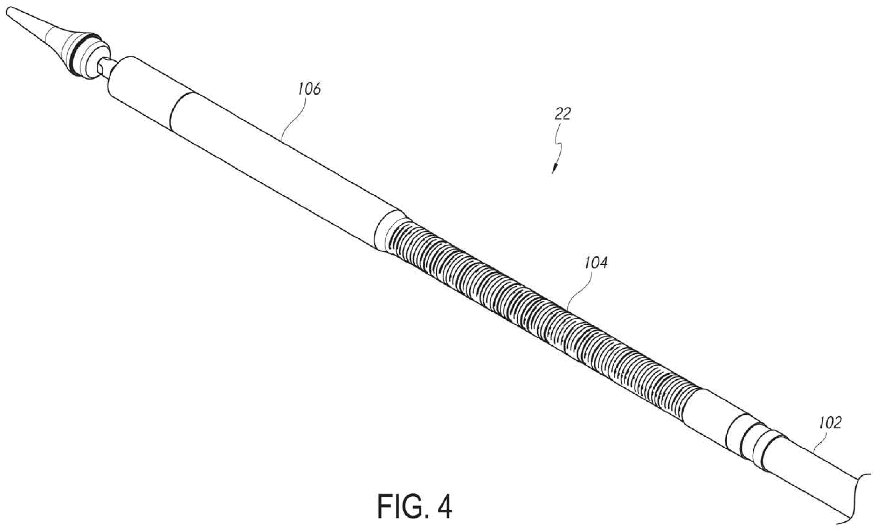

As shown in FIGS. 2A-2C and as further shown in FIGS. 4-8, starting with the outermost assembly, the delivery system can include an outer sheath assembly **22** forming a radially outer covering, or sheath, to surround an implant retention area **16** and prevent the implant from radially expanding. Specifically, the outer sheath assembly **22** can prevent radial expansion of the distal end of the implant from radially expanding. Moving radially inward, the mid shaft assembly **21** can be composed of a mid shaft hypotube **43** with its distal end attached to an outer retention member or outer retention ring **42** for radially retaining a portion of the prosthesis in a compacted configuration, such as a proximal end of the implant **70.** The mid shaft assembly **21** can be located within a lumen of the outer sheath assembly **22.** Moving further inwards, the rail assembly **20** can be configured for steerability, as mentioned above and further described below. The rail assembly **20** can be located within a lumen of the mid shaft assembly **21.** Moving further inwards, the inner shaft assembly **18** can be composed of an inner shaft with its distal end attached to inner retention member or inner retention ring **40** (such as a PEEK ring) for axially retaining the prosthesis, for example the proximal end of the prosthesis. The inner shaft assembly **18** can be located within a lumen of the rail assembly **20.** Further, the most radially-inward assembly is the nose cone assembly **31** which includes the nose cone shaft **27** having its distal end connected to the nose cone **28.** The nose cone **28** can have a tapered tip. The nose cone assembly **31** is preferably located within a lumen of the inner shaft assembly **18.** The nose cone assembly **31** can include a lumen for a guide wire to pass therethrough.

The elongate shaft **12,** and more specifically the nose cone assembly **31,** inner assembly **18,** rail assembly **20,** mid shaft assembly **21,** and outer sheath assembly **22,** can be collectively configured to deliver an implant **70** positioned within the implant retention area **16** (shown in FIG. 2A) to a treatment location. One or more of the subassemblies can then be moved to allow the implant **70** to be released at the treatment location. For example, one or more of the subassemblies may be movable with respect to one or more of the other subassemblies. The handle **14** can include various control mechanisms that can be used to control the movement of the various subassemblies as will also be described in more detail below. In this way, the implant **70** can be controllably loaded onto the delivery system **10** and then later deployed within the body. Further, the handle **14** can provide steering to the rail assembly **20,** providing for bending/flexing/steering of the distal end of the delivery system **10.**

As will be discussed below, the inner retention member **40,** the outer retention ring **42,** and the outer sheath assembly **22** can cooperate to hold the implant **70** in a compacted configuration. The inner retention member **40** is shown engaging struts **72** at the proximal end **301** of the implant **70** in FIG. 2A. For example, slots located between radially extending teeth on the inner retention member **40** can receive and engage the struts **72** which may end in mushroom-shaped tabs **74** on the proximal end of the implant **70.** The mid shaft assembly **21** can be positioned over the inner retention member **40** so that the first end **301** of the implant **70** is trapped between the inner retention member **40** and the outer retention ring **42,** thereby securely attaching it to the delivery system **10** between the mid shaft assembly **21** and the inner retention member **40.** The outer sheath assembly **22** can be positioned to cover the second end **303** of the implant **70.**

The outer retention member **42** may be attached to a distal end of the mid shaft hypotube **43** which can in turn be attached to a proximal tube **44** at a proximal end, which in turn can be attached at a proximal end to the handle **14.** The outer retention member **42** can provide further stability to the implant **70** when in the compressed position. The outer retention member **42** can be positioned over the inner retention member **40** so that the proximal end of the implant **70** is trapped therebetween, securely attaching it to the delivery system **10.** The outer retention member **42** can encircle a portion of the implant **70,** in particular the first end **301,** thus preventing the implant **70** from expanding. Further, the mid shaft assembly **21** can be translated proximally with respect to the inner assembly **18** into the outer sheath assembly **22,** thus exposing a first end **301** of the implant **70** held within the outer retention member **42.** In this way the outer retention member **42** can be used to help secure an implant **70** to or release it from the delivery system **10.** The outer retention member **42** can have a cylindrical or elongate tubular shape, and may be referred to as an outer retention ring, though the particular shape is not limiting.

As shown in FIG. 2A, the distal anchors **80** can be located in a delivered configuration where the distal anchors **80** point generally distally (as illustrated, axially away from the main body of the prosthesis frame and away from the handle of the delivery system). The distal anchors **80** can be restrained in this delivered configuration by the outer sheath assembly **22.** Accordingly, when the outer sheath **22** is withdrawn proximally, the distal anchors **80** can flip positions (e.g., bend approximately 180 degrees) to a deployed configuration (e.g., pointing generally proximally). FIG. 2A also shows the proximal anchors **82** extending distally in their delivered configuration within the outer sheath assembly **22.** In other embodiments, the distal anchors **80** can be held to point generally proximally in the delivered configuration and compressed against the body of the prosthesis frame.

The delivery system **10** may be provided to users with an implant **70** preinstalled. In other embodiments, the implant **70** can be loaded onto the delivery system shortly before use, such as by a physician or nurse.

FIGS. 4-8 illustrate further views of delivery system **10** with different assemblies translated proximally and described in detail.

Starting with the outermost assembly shown in FIG. 4, the outer sheath assembly **22** can include an outer proximal shaft **102** directly attached to the handle **14** at its proximal end and an outer hypotube **104** attached at its distal end. A capsule **106** can then be attached generally at the distal end of the outer hypotube **104.** In some embodiments, the capsule **106** can be 28 French or less in size. These components of the outer sheath assembly **22** can form a lumen for the other subassemblies to pass through.

A capsule **106** can be located at a distal end of the outer proximal shaft **102.** The capsule **106** can be a tube formed of a plastic or metal material. In some embodiments, the capsule **106** is formed of ePTFE or PTFE. In some embodiments, this capsule **106** is relatively thick to prevent tearing and to help maintain a self-expanding implant in a compacted configuration. In some embodiments the material of the capsule **106** is the same material as the coating on the outer hypotube **104.** As shown, the capsule **106** can have a diameter larger than the outer hypotube **104,** though in some embodiments the capsule **106** may have a similar diameter as the hypotube **104.** In some embodiments, the capsule **106** may include a larger diameter distal portion and a smaller diameter proximal portion. In some embodiments, there may be a step or a taper between the two portions. The capsule **106** can be configured to retain the implant **70** in the compressed position within the capsule **106.** Further construction details of the capsule **106** are discussed below.

The outer sheath assembly **22** is configured to be individually slidable with respect to the other assemblies. Further, the outer sheath assembly **22** can slide distally and proximally relative to the rail assembly **20** together with the mid shaft assembly **21,** inner assembly **18,** and nose cone assembly **31.**

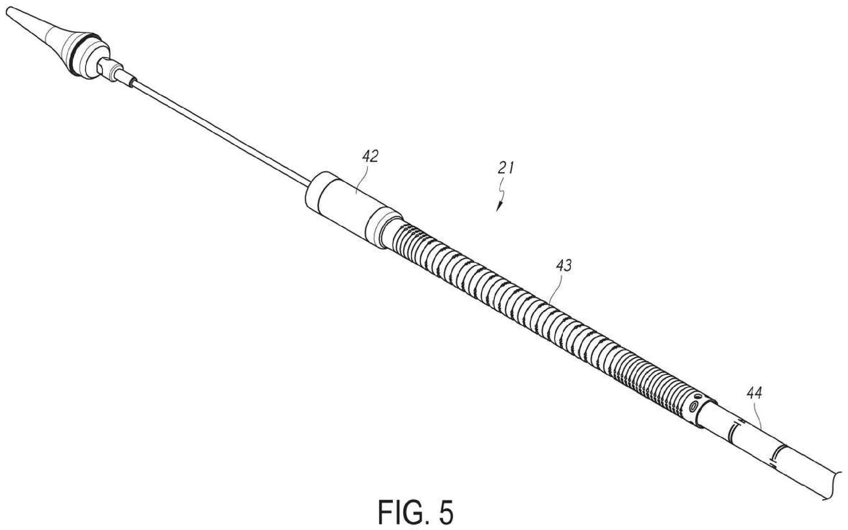

Moving radially inwardly, the next assembly is the mid shaft assembly **21.** FIG. 5 shows a similar view as FIG. 4, but with the outer sheath assembly **22** removed, thereby exposing the mid shaft assembly **21.**

The mid shaft assembly **21** can include a mid shaft hypotube **43** generally attached at its proximal end to a mid shaft proximal tube **44,** which in turn can be attached at its proximal end to the handle **14,** and an outer retention ring **42** located at the distal end of the mid shaft hypotube **43.** Thus, the outer retention ring **42** can be attached generally at the distal end of the mid shaft hypotube **43.** These components of the mid shaft assembly **21** can form a lumen for other subassemblies to pass through.

The outer retention ring **42** can be configured as a prosthesis retention mechanism that can be used to engage with the implant **70,** as discussed with respect to FIG. 2A. For example, the outer retention ring **42** may be a ring or covering that is configured to radially cover the struts **72** on the implant **70.** The outer retention ring **42** can also be considered to be part of the implant retention area **16,** and may be at the proximal end of the implant retention area **16.** With struts or other parts of an implant **70** engaged with the inner retention member **40,** discussed below the outer retention ring **42** can cover both the implant **70** and the inner retention member **40** to secure the implant **70** on the delivery system **10.** Thus, the implant **70** can be sandwiched between the inner retention member **40** of the inner shaft assembly **18** and the outer retention ring **42** of the mid shaft assembly **21.**

The mid shaft assembly **21** is disposed so as to be individually slidable with respect to the other assemblies. Further, mid shaft assembly **21** can slide distally and proximally relative to the rail assembly **20** together with the outer sheath assembly **22,** the inner assembly **18,** and nose cone assembly **31.**

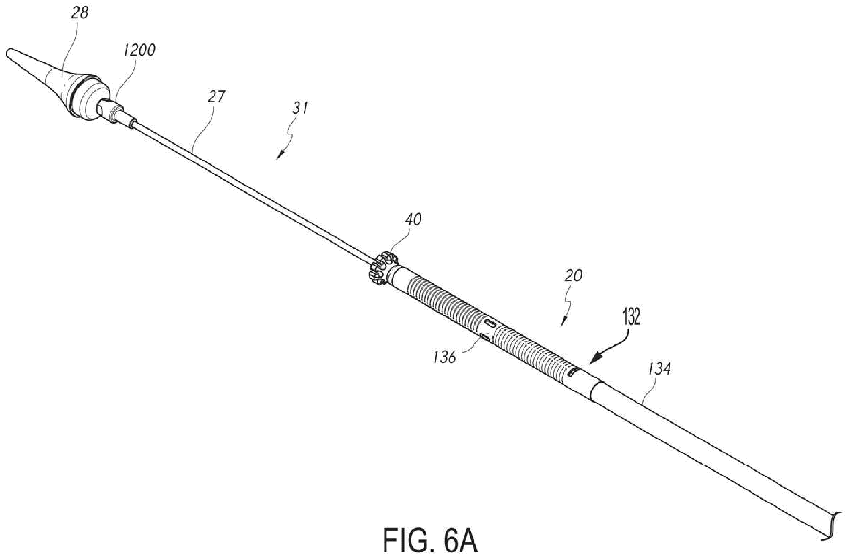

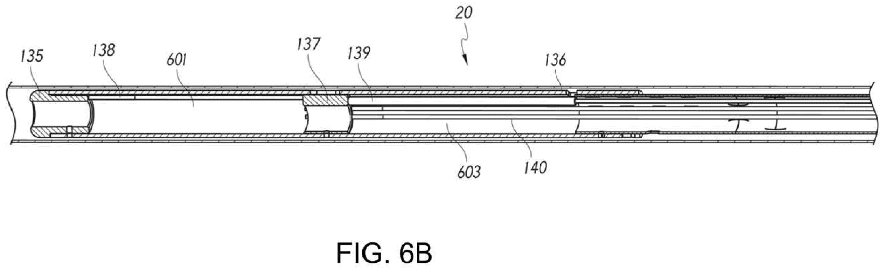

Next, radially inwardly of the mid shaft assembly **21** is the rail assembly **20.** FIG. 6A shows approximately the same view as FIG. 5, but with the mid shaft assembly **21** removed, thereby exposing the rail assembly **20.** FIG. 6B further shows a cross-section of the rail assembly **20** to view the pull wires. The rail assembly **20** can include a rail shaft **132** (or rail) generally attached at its proximal end to the handle **14.** The rail shaft **132** can be made up of a rail proximal shaft **134** directly attached to the handle at a proximal end and a rail hypotube **136** attached to the distal end of the rail proximal shaft **134.** The rail shaft **132** may include a proximal rail shaft portion **603** and a distal rail shaft portion **601.** The rail hypotube **136** can further include an atraumatic rail tip at its distal end. Further, the distal end of the rail hypotube **136** can abut a proximal end of the inner retention member **40,** as shown in FIG. 6A. In some embodiments, the distal end of the rail hypotube **136** can be spaced away from the inner retention member **40.** These components of the rail shaft assembly **20** can form a lumen for the other subassemblies to pass through.

As shown in FIG. 6B, attached to an inner surface of the rail hypotube **136** are one or more pull wires which can be used apply forces to the rail hypotube **136** and steer the rail assembly **20.** The pull wires can extend distally from the knobs in the handle **14,** discussed below, to the rail hypotube **136.** In some embodiments, pull wires can be attached at different longitudinal locations on the rail hypotube **136,** thus providing for multiple bending locations in the rail hypotube **136,** allowing for multidimensional steering.

In some embodiments, a distal pull wire **138** can extend to a distal section of the rail hypotube **136** and two proximal pull wires **140** can extend to a proximal section of the rail hypotube **136,** however, other numbers of pull wires can be used, and the particular amount of pull wires is not limiting. For example, a two pull wires can extend to a distal location and a single pull wire can extend to a proximal location. In some embodiments, ring-like structures attached inside the rail hypotube **136,** known as pull wire connectors, can be used as attachment locations for the pull wires, such as proximal ring **137** and distal ring **135.** In some embodiments, the rail assembly **20** can include a distal pull wire connector **135** and a proximal pull wire connector **137.** In some embodiments, the pull wires can directly connect to an inner surface of the rail hypotube **136.**

The distal pull wire **138** can be connected (either on its own or through a connector **135**) generally at the distal end of the rail hypotube **136.** The proximal pull wires **140** can connect (either on its own or through a connector **137**) at a location approximately one quarter, one third, or one half of the length up the rail hypotube **136** from the proximal end. In some embodiments, the distal pull wire **138** can pass through a small diameter pull wire lumen **139** (e.g., tube, hypotube, cylinder) attached on the inside of the rail hypotube **136.** This can prevent the wires **138** from pulling on the rail hypotube **136** at a location proximal to the distal connection. Further, the lumen **139** can act as compression coils to strengthen the proximal portion of the rail hypotube **136** and prevent unwanted bending. Thus, in some embodiments the lumen **139** is only located on the proximal half of the rail hypotube **136.** In some embodiments, multiple lumens **139,** such as spaced longitudinally apart or adjacent, can be used per distal wire **138.** In some embodiments, a single lumen **139** is used per distal wire **138.** In some embodiments, the lumen **139** can extend into the distal half of the rail hypotube **136.** In some embodiments, the lumen **139** is attached on an outer surface of the rail hypotube **136.** In some embodiments, the lumen **139** is not used.

For the pair of proximal pull wires **140,** the wires can be spaced approximately 180° from one another to allow for steering in both directions. Similarly, if a pair of distal pull wires **138** is used, the wires can be spaced approximately 180° from one another to allow for steering in both directions. In some embodiments, the pair of distal pull wires **138** and the pair of proximal pull wires **140** can be spaced approximately 90° from each other. In some embodiments, the pair of distal pull wires **138** and the pair of proximal pull wires **140** can be spaced approximately 0° from each other. However, other locations for the pull wires can be used as well, and the particular location of the pull wires is not limiting. In some embodiments, the distal pull wire **138** can pass through a lumen **139** attached within the lumen of the rail hypotube **136.** This can prevent an axial force on the distal pull wire **138** from creating a bend in a proximal section of the rail hypotube **136.**

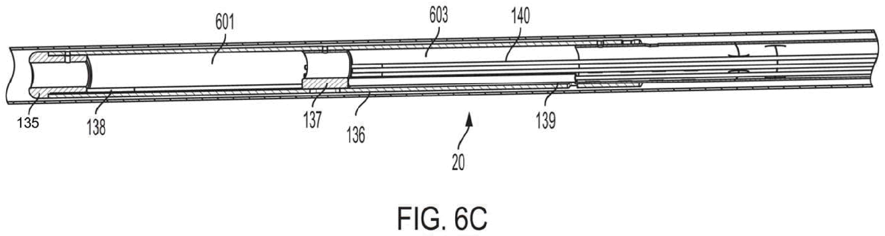

FIG. 6C illustrates an embodiment in which the position of the proximal pull wires **140** has been moved 180° from the position shown in FIG. 6B. The position of the proximal pull wires **140** shown in FIG. 6C may allow the proximal portion of the rail hypotube **136** to bend in an opposite direction than the direction possible in FIG. 6B. For example, in the embodiment of FIG. 6B, when the distal portion of the rail hypotube **136** is deflected in a downward direction by the pull of the distal pull wires **138,** the proximal portion of the rail hypotube **136** may be deflected leftward relative to the downward direction (viewing from the proximal end of the rail hypotube **136** toward the distal end of the rail hypotube **136**). In the embodiment of FIG. 6C, however, when the distal portion of the rail hypotube **136** is deflected in a downward direction by the pull of the distal pull wires **138,** the proximal portion of the rail hypotube **136** may be deflected rightward relative to the downward direction (viewing from the proximal end of the rail hypotube **136** toward the distal end of the rail hypotube **136**). Such a variation may allow the proximal portion of the rail hypotube **136,** and accordingly the elongate shaft **12** to deflect in an opposite direction than possible in the embodiment shown in FIG. 6B. The thickness of cuts on the rail shaft **132** may also be varied to allow for the opposite direction of deflection.

The rail assembly **20** is disposed so as to be slidable over the inner shaft assembly **18** and the nose cone assembly **31.** In some embodiments, the outer sheath assembly **22,** the mid shaft assembly **21,** the inner shaft assembly **18,** and the nose cone assembly **31** can be configured to slide together along or relative to the rail assembly **20,** such as proximally and distally with or without any bending of the rail assembly **20.** In some embodiments, the outer sheath assembly **22,** the mid shaft assembly **21,** the inner shaft assembly **18,** and the nose cone assembly **31** can be configured to retain the implant **70** in a compressed position when they are simultaneously slid along or relative to the rail assembly **20.**

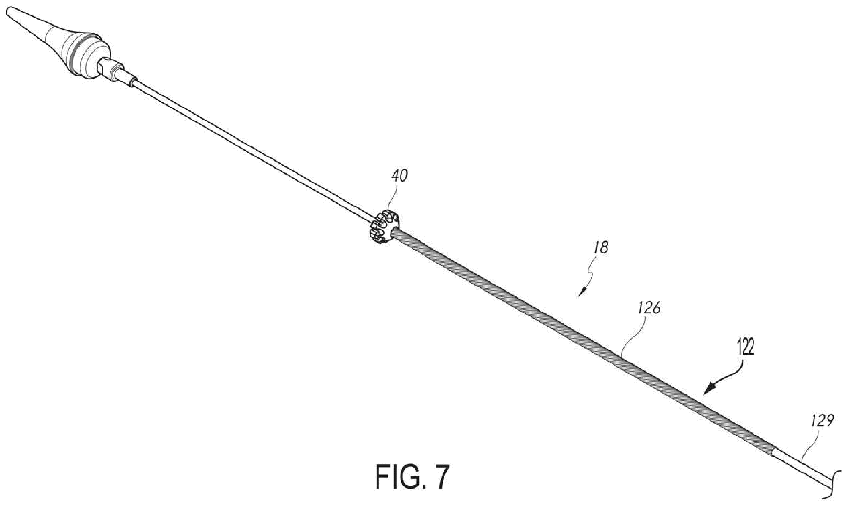

Moving radially inwards, the next assembly is the inner shaft assembly **18.** FIG. 7 shows approximately the same view as FIG. 6A, but with the rail assembly **20** removed, thereby exposing the inner shaft assembly **18.**

The inner shaft assembly **18** can include an inner shaft **122** generally attached at its proximal end to the handle **14,** and an inner retention ring **40** located at the distal end of the inner shaft **122.** The inner shaft **122** itself can be made up of an inner proximal shaft **129** directly attached to the handle **14** at a proximal end and a distal section **126** attached to the distal end of the inner proximal shaft **129.** Thus, the inner retention ring **40** can be attached generally at the distal end of the distal section **126.** These components of the inner shaft assembly **18** can form a lumen for the other subassemblies to pass through.

The inner retention member **40** can be configured as a prosthesis retention mechanism that can be used to engage with the implant **70,** as discussed with respect to FIG. 2A. For example, the inner retention member **40** may be a ring and can include a plurality of slots configured to engage with struts **72** on the implant **70.** The inner retention member **40** can also be considered to be part of the implant retention area **16,** and may be at the proximal end of the implant retention area **16.** With struts or other parts of an implant **70** engaged with the inner retention member **40,** the outer retention ring **42** can cover both the prosthesis and the inner retention member **40** to secure the prosthesis on the delivery system **10.** Thus, the implant **70** can be sandwiched between the inner retention member **40** of the inner shaft assembly **18** and the outer retention ring **42** of the mid shaft assembly **21.**

The inner shaft assembly **18** is disposed so as to be individually slidable with respect to the other assemblies. Further, the inner assembly **18** can slide distally and proximally relative to the rail assembly **20** together with the outer sheath assembly **22,** mid shaft assembly **21,** and nose cone assembly **31.**

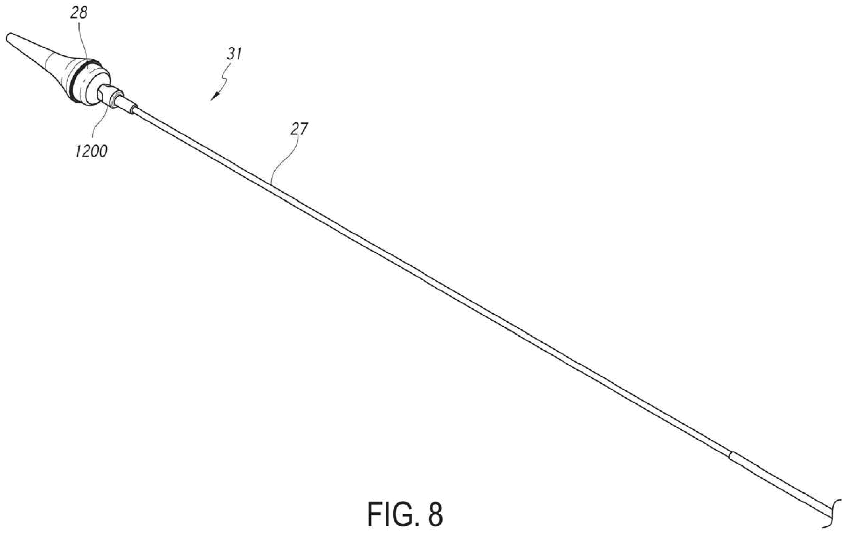

Moving further inwardly from the inner shaft assembly **18** is the nose cone assembly **31** also seen in FIG. 8. This may be a nose cone shaft **27,** and in some embodiments, may have a nose cone **28** on its distal end. The nose cone **28** can be made of polyurethane for atraumatic entry and to minimize injury to venous vasculature. The nose cone **28** can also be radiopaque to provide for visibility under fluoroscopy.

The nose cone shaft **27** may include a lumen sized and configured to slidably accommodate a guide wire so that the delivery system **10** can be advanced over the guide wire through the vasculature. However, embodiments of the system **10** discussed herein may not use a guide wire and thus the nose cone shaft **27** can be solid. The nose cone shaft **27** may be connected from the nose cone **28** to the handle, or may be formed of different segments such as the other assemblies. Further, the nose cone shaft **27** can be formed of different materials, such as plastic or metal, similar to those described in detail above.

In some embodiments, the nose cone shaft **27** includes a guide wire shield **1200** located on a portion of the nose cone shaft **27.**

The nose cone assembly **31** is disposed so as to be individually slidable with respect to the other assemblies. Further, the nose cone assembly **31** can slide distally and proximally relative to the rail assembly **20** together with the outer sheath assembly **22,** mid shaft assembly **21,** and inner assembly **18.**

In some embodiments, one or more spacer sleeves (not shown) can be used between different assemblies of the delivery system **10.** For example, a spacer sleeve can be located concentrically between the mid shaft assembly and the rail assembly **20,** generally between the mid **43** and rail hypotubes **136.** In some embodiments, the spacer sleeve can be generally embedded in the hypotube **43** of the mid shaft assembly **21,** such as on an inner surface of the mid shaft assembly **21.** In some embodiments, a spacer sleeve can be located concentrically between the rail assembly **20** and the inner assembly **18,** generally within the rail hypotube **136.** In some embodiments, a spacer sleeve can be used between the outer sheath assembly **22** and the mid shaft assembly **21.** In some embodiments, a spacer sleeve can be used between the inner assembly **18** and the nose cone assembly **31.** In some embodiments, 4, 3, 2, or 1 of the above-mentioned spacer sleeves can be used. The spacer sleeves can be used in any of the above positions.

As discussed above, the outer sheath assembly **22,** the mid shaft assembly **21,** the inner assembly **18,** and the rail assembly **20** can contain an outer hypotube **104,** a mid shaft hypotube, a distal section **126,** and a rail hypotube **136,** respectively. Each of these hypotubes/sections/shafts can be laser cut to include a number of slots, thereby creating a bending pathway for the delivery system to follow.

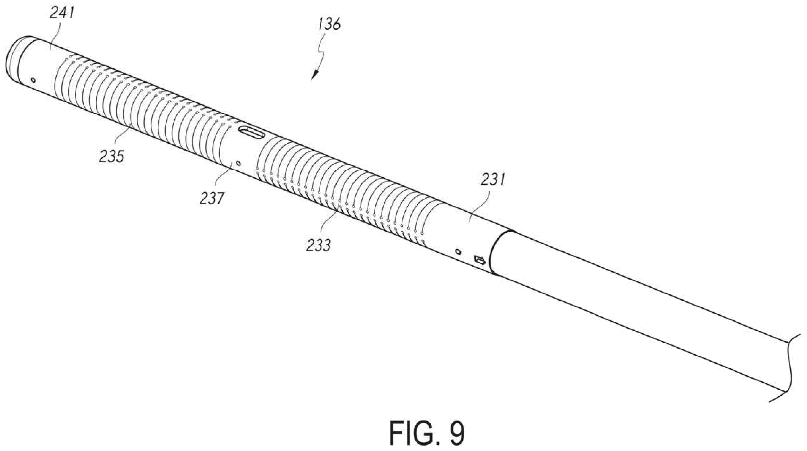

For example, FIG. 9 shows an embodiment of the rail hypotube **136.** The rail hypotube **136** can also contain a number of circumferential slots. The rail hypotube **136** can generally be broken into a number of different sections. At the most proximal end is an uncut (or unslotted) hypotube section **231.** Moving distally, the next section is the proximal slotted hypotube section **233.** This section includes a number of circumferential slots cut into the rail hypotube **136.** Generally, two slots are cut around each circumferential location forming almost half of the circumference. Accordingly, two backbones are formed between the slots extending up the length of the hypotube **136.** This is the section that can be guided by the proximal pull wires **140.** Moving further distally is the location **237** where the proximal pull wires **140** connect, and thus slots can be avoided. Thus section is just distal of the proximally slotted section.

Distally following the proximal pull wire connection area is the distal slotted hypotube section **235.** This section is similar to the proximal slotted hypotube section **233,** but has significantly more slots cut out in an equivalent length. Thus, the distally slotted hypotube section **235** provides easier bending than the proximally slotted hypotube section **233.** The proximal and distal slotted hypotube sections **233, 235** may comprise bend portions of the rail shaft. In some embodiments, the proximal slotted section **233** can be configured to experience a bend of approximately 90 degrees with a half inch radius whereas the distal slotted section **235** can bend at approximately 180 degrees within a half inch. Further, as shown in FIG. 9, the spines of the distally slotted hypotube section **235** are offset from the spines of the proximally slotted hypotube section **233.** Accordingly, the two sections will achieve different bend patterns, allowing for three-dimensional steering of the rail assembly **20.** In some embodiments, the spines can be offset 30, 45, or 90 degrees, though the particular offset is not limiting. In some embodiments, the proximally slotted hypotube section **233** can include compression coils. This allows for the proximally slotted hypotube section **233** to retain rigidity for specific bending of the distally slotted hypotube section **235.**

At the distalmost end of the distal slotted hypotube section **235** is the distal pull wire connection area **241** which is again a non-slotted section of the rail hypotube **136.**

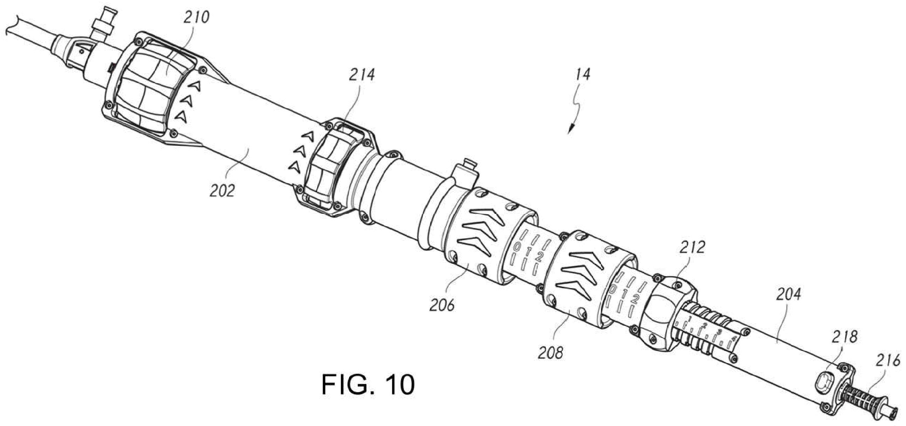

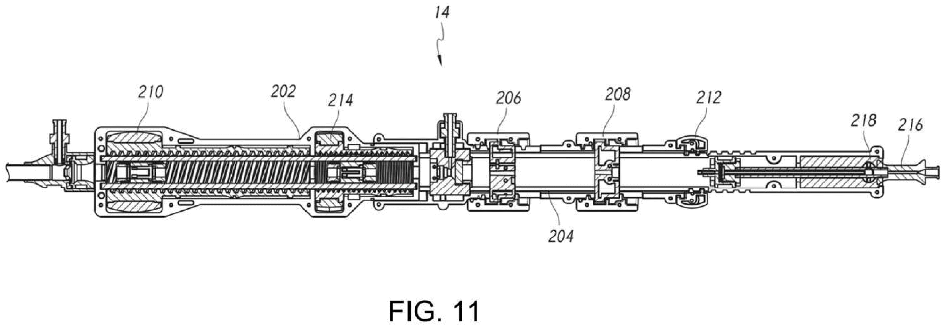

The handle **14** is located at the proximal end of the delivery system **10.** An embodiment of a handle **14** is shown in FIG. 10. A cross-section of the handle **14** is shown in FIG. 11. The handle **14** can include a number of actuators, such as rotatable knobs, that can manipulate different components of the delivery system **10.** The operation of the handle **14** is described with reference to delivery of a replacement valve prosthesis or implant **70,** though the handle **14** and delivery system **10** can be used to deliver other devices as well.

The handle **14** is generally composed of two housings, a rail housing **202** and a delivery housing **204,** the rail housing **202** being circumferentially disposed around the delivery housing **204.** The inner surface of the rail housing **202** can include a screwable section configured to mate with an outer surface of the delivery housing **204.** Thus, the delivery housing **204** is configured to slide (e.g., screw) within the rail housing **202,** as detailed below. The rail housing **202** generally surrounds about one half the length of the delivery housing **204,** and thus the delivery housing **204** extends both proximally and distally outside of the rail housing **202.**

The rail housing **202** can contain two rotatable knobs, a distal pull wire knob **206** and a proximal pull wire knob **208.** However, the number of rotatable knobs on the rail housing **202** can vary depending on the number of pull wires used. Rotation of the distal pull wire knob **206** can provide a proximal force, thereby providing axial tension on the distal pull wires **138** and causing the distal slotted section of the rail hypotube **136** to bend. The distal pull wire knob **206** can be rotated in either direction, allowing for bending in either direction, which can control anterior-posterior angles. Rotation of the proximal pull wire knob **208** can provide a proximal force, and thus axial tension, on the proximal pull wires **140,** thereby causing the proximal slotted section **133** of the rail hypotube **136** to bend, which can control the medial-lateral angle. The proximal pull wire knob **208** can be rotated in either direction, allowing for bending in either direction. Thus, when both knobs are actuated, there can be two bends in the rail hypotube **136,** thereby allowing for three-dimensional steering of the rail shaft **132,** and thus the distal end of the delivery system **10.** Further, the proximal end of the rail shaft **132** is connected on an internal surface of the rail housing **202.**

The bending of the rail shaft **132** can be used to position the system, in particular the distal end, at the desired patient location, such as at the native tricuspid valve. In some embodiments, rotation of the pull wire knobs **206/208** can help steer the distal end of the delivery system **10** to a desired position proximal a valve to be treated, for example a tricuspid or mitral valve.

Moving to the delivery housing **204,** the proximal ends of the inner shaft assembly **18,** outer sheath assembly **22,** mid shaft assembly **21,** and nose cone shaft assembly **31** can be connected to an inner surface of the delivery housing **204** of the handle **14.** Thus, they can move axially relative to the rail assembly **20** and rail housing **202.**

A rotatable outer sheath knob **210** can be located on the distal end of the delivery housing **204,** being distal to the rail housing **202.** Rotation of the outer sheath knob **210** will pull the outer sheath assembly **22** in an axial direction proximally, thus pulling the capsule **106** away from the implant **70** and releasing the distal end **303** of implant **70.** Thus the outer sheath assembly **22** is individually translated with respect to the other shafts in the delivery system **10.** The distal end **303** of the implant **70** can be released first, while the proximal end **301** of the implant **70** can remain radially compressed between the inner retention member **40** and the outer retention member **42.**

A rotatable mid shaft knob **214** can be located on the delivery housing **204,** in some embodiments proximal to the rotatable outer sheath knob **210,** being distal to the rail housing **202.** Rotation of the mid shaft knob **212** will pull the mid shaft assembly **21** in an axial direction proximally, thus pulling the outer retention ring **42** away from the implant **70** and uncovering the inner retention member **40** and the proximal end **301** of the implant **70,** thereby releasing the implant **70.** Thus, the mid shaft assembly **21** is individually translated with respect to the other shafts in the delivery system **10.**

Located on the proximal end of the delivery housing **204,** and thus proximal to the rail housing **202,** can be a rotatable depth knob **212.** As the depth knob **212** is rotated, the entirety of the delivery housing **204** moves distally or proximally with respect to the rail housing **202** which will remain in the same location. Thus, at the distal end of the delivery system **10,** the inner shaft assembly **18,** outer sheath assembly **22,** mid shaft assembly **21,** and nose cone shaft assembly **31** together (e.g., simultaneously) move proximally or distally with respect to the rail assembly **20** while the implant **70** remains in the compressed configuration. In some embodiments, actuation of the depth knob **212** can sequentially move the inner shaft assembly **18,** outer sheath assembly **22,** mid shaft assembly **21,** and nose cone shaft assembly **31** relative to the rail assembly **20.** In some embodiments, actuation of the depth knob **212** can together move the inner shaft assembly **18,** outer sheath assembly **22,** and mid shaft assembly **21** relative to the rail assembly **20.** Accordingly, the rail shaft **132** can be aligned at a particular direction, and the other assemblies can move distally or proximally with respect to the rail shaft **132** for final positioning while not releasing the implant **70.** The components can be advanced approximately 1, 2, 3, 5, 6, 7, 8, 9, or 10 cm along the rail shaft **132.** The components can be advanced more than approximately 1, 2, 3, 5, 6, 7, 8, 9, or 10 cm along the rail shaft **132.** An example of this is shown in FIG. 2C. The capsule **106** and outer retention ring **42** can then be individually withdrawn with respect to the inner assembly **18** as discussed above, in some embodiments sequentially, releasing the implant **70.** The assemblies other than the rail assembly **20** can then be withdrawn back over the rail shaft **132** by rotating the depth knob **212** in the opposite direction.

The handle **14** can further include a mechanism (knob, button, handle) **216** for moving the nose cone shaft **27,** and thus the nose cone **28.** For example, a knob **216** can be a portion of the nose cone assembly **31** that extends from a proximal end of the handle **14.** Thus, a user can pull or push on the knob **216** to translate the nose cone shaft **27** distally or proximally individually with respect to the other shafts. This can be advantageous for proximally translating the nose cone **28** into the outer sheath assembly **22** /capsule **106,** thus facilitating withdraw of the delivery system **10** from the patient.

In some embodiments, the handle **14** can provide a lock **218,** such as a spring lock, for preventing translation of the nose cone shaft **27** by the knob **216** discussed above. In some embodiments, the lock **218** can be always active, and thus the nose cone shaft **27** will not move without a user disengaging the lock **218.** The lock can be, for example, a spring lock that is always engaged until a button **218** on the handle **14** is pressed, thereby releasing the spring lock and allowing the nose cone shaft **27** to translate proximally/distally. In some embodiments, the spring lock **218** allows one-way motion, either proximal or distal motion, of the nose cone shaft **27** but prevents motion in the opposite direction.

The handle **14** can further include a communicative flush port for flushing out different lumens of the delivery system **10.** In some embodiments, a single flush port on the handle **14** can provide fluid connection to multiple assemblies. In some embodiments, the flush port can provide fluid connection to the outer sheath assembly **22.** In some embodiments, the flush port can provide fluid connection to the outer sheath assembly **22** and the mid shaft assembly **21.** In some embodiments, the flush port can provide fluid connection to the outer sheath assembly **22,** the mid shaft assembly **21,** and the rail assembly **20.** In some embodiments, the flush port can provide fluid connection to the outer sheath assembly **22,** the mid shaft assembly **21,** the rail assembly **20,** and the inner assembly **18.** Thus, in some embodiments, the rail shaft **132,** the outer retention ring **42,** and the capsule **106** can all be flushed by a single flush port.

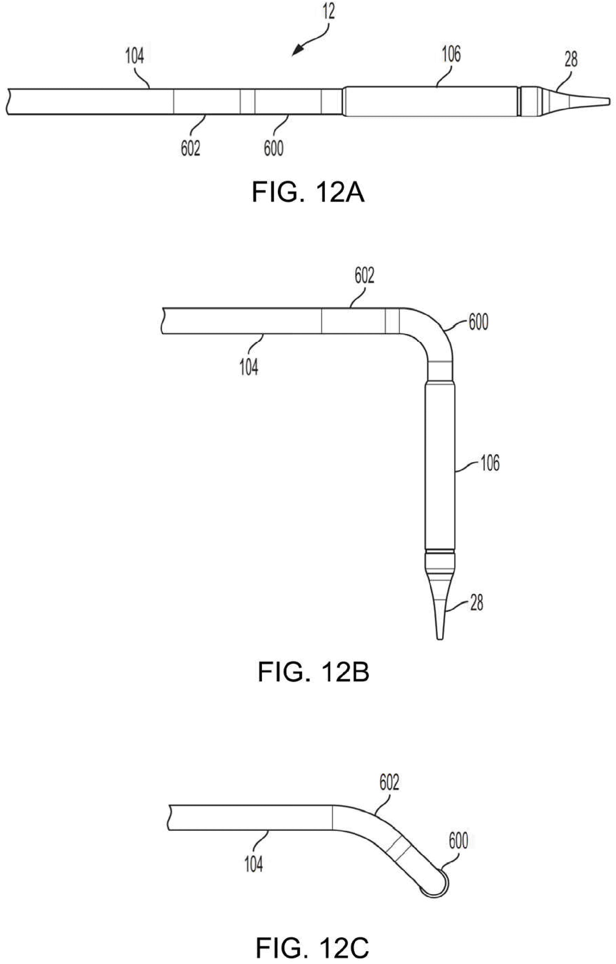

FIG. 12A illustrates a side view of a distal portion of the elongate shaft **12** with the elongate shaft **12** in a straightened configuration. The capsule **106** is shown positioned between the outer hypotube **104** and the nose cone **28.**

The elongate shaft **12** may include one or more bend portions, which may allow the elongate shaft **12** to bend at the bend portions. In the embodiment shown in FIG. 12A, for example, the elongate shaft **12** includes two bend portions **600, 602.** The bend portion **600** may correspond to the distal rail portion **601** shown in FIGS. 6B and 6C, and the bend portion **602** may correspond to the proximal rail portion **603** shown in FIGS. 6B and 6C. As such, the bend portions **600, 602** may be configured to bend the elongate shaft **12** in planes that are perpendicular from each other, with the bend portion **600** able to bend in what may be called a vertical plane, and the bend portion **602** able to bend in what may accordingly be called a horizontal plane. The bend portions **600, 602** may be configured to bend to orient the capsule 106 in the desired position for deployment of the implant **70** contained therein.

The capsule **106** (and the implant retention area **16** contained therein) may be configured to slide relative to the bend portions **600, 602** in the manners disclosed herein. For example, the outer sheath assembly **22,** mid shaft assembly **21,** inner shaft assembly **18,** and nose cone assembly **31** may be configured to slide relative to the bend portions **600, 602** (as part of the rail assembly **20**) to vary a distance or depth of the capsule **106** from the rail assembly **20.** The outer sheath assembly **22** may be configured to slide relative to the rail assembly **20** to vary a distance of the implant retention area from the patient's tricuspid valve.

Referring to FIG. 12B, the bend portion **600,** which is positioned proximal of the capsule **106,** and is positioned between the capsule **106** and the bend portion **602,** is shown to deflect the distal end of the elongate shaft **12** to a direction (which may be referred to as a downward direction as shown in FIG. 12B). The bend portion **600** deflects the distal end of the elongate shaft **12** in a plane (which may be referred to as a vertical plane). The bend portion **600** accordingly has varied the orientation of the capsule **106,** the distal end of the elongate shaft **12,** and the implant retention area **16** positioned within the capsule **106.**

FIG. 12C illustrates a top view of the elongate shaft **12** shown in FIGS. 12A and 12B, with the bend portion **602** bent. In FIG. 12C, the bend portion **602,** which is positioned proximal the bend portion **600** is shown to deflect the distal end of the elongate shaft **12** to a direction (which may be referred to as a rightward direction as shown in FIG. 12C). The bend portion **602** deflects the distal end of the elongate shaft **12** in a plane (which may be referred to as a horizontal plane). The bend portion **600** accordingly has varied the orientation of the capsule **106,** the distal end of the elongate shaft **12,** and the implant retention area **16** positioned within the capsule **106.**

The bend portion **602** accordingly may deflect the bend portion **600** and the capsule **106** in a plane that is perpendicular to the plane that the bend portion **600** may deflect the capsule **106.** The orthogonal planes of deflection may allow for three-dimensional steering of the capsule **106.**

The bend portion **602** as shown in FIG. 12C may be configured to deflect the distal end of the elongate shaft **12** to a rightward direction. Such direction of deflection may be provided by the configuration of pull wires shown in FIG. 6C.

Additional or varied movement of the elongate shaft **12** may be desired. Such additional or varied movement may be desired for a variety of reasons, which may include a variety of patient anatomies to be navigated with the distal end of the elongate shaft **12** or varied uses of the elongate shaft **12.**

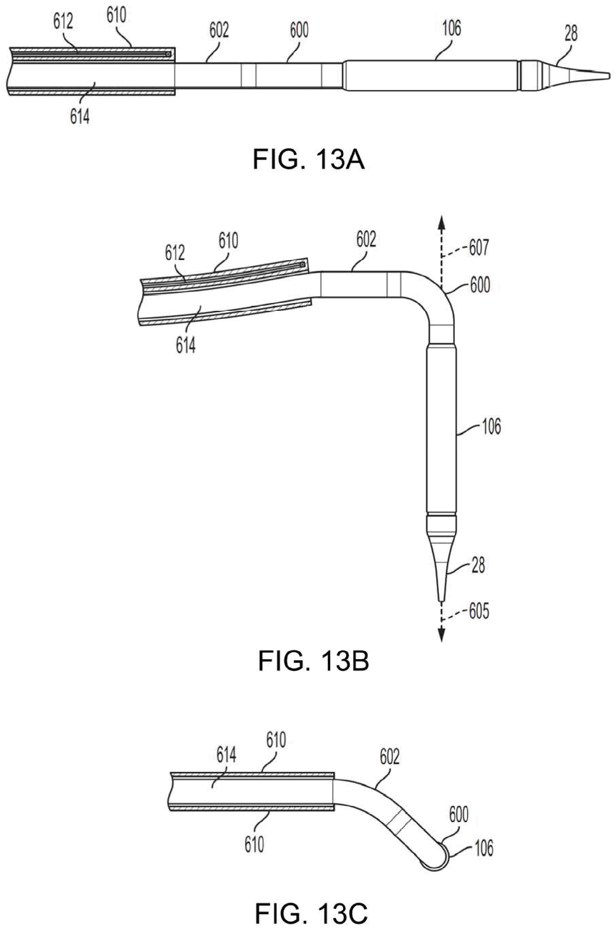

FIGS. 13A-D illustrate an embodiment in which a deflection mechanism may be utilized to provide deflection of a portion of the elongate shaft **12.** Referring to FIG. 13A, the deflection mechanism may include a sheath **610** that extends over a portion **614** of the elongate shaft **12.** The portion **614** of the elongate shaft **12** may comprise a portion that is positioned proximal of the bend portion **602** and the bend portion **600.** However, in other embodiments the sheath **610** may extend over other portions of the elongate shaft **12,** possibly extending to the distal end of the elongate shaft **12.**

The sheath **610** is shown in cross section in FIG. 13A and may be configured to deflect to provide the deflection of the elongate shaft **12.** The sheath may include a control device that is utilized to control deflection of the sheath **610.** The control device may comprise a pull tether **612** as shown in FIG. 13A, which may comprise a pull wire or other forms of tethers. In other embodiments, other forms of control devices may be utilized such as gears, rails, or other forms of control devices. The pull tether **612** may be oriented on the elongate shaft **12** such that retraction of the pull tether **612** may deflect the elongate shaft **12** in a direction towards the pull tether **612.**

Referring to FIG. 13B, the bend portion **600** has deflected the distal end of the elongate shaft 12 to a direction **605** (which may be referred to as a downward direction as shown in FIG. 13B). The deflection mechanism, however, has deflected the portion **614** of the elongate shaft **12** that is positioned proximal of the bend portion **600** and bend portion **602** to deflect the bend portions **600, 602** towards a direction **607** that is opposed from the direction **605** that the bend portion **600** has deflected the distal end of the elongate shaft **12.** The deflection mechanism has also deflected the bend portion **602,** bend portion **600,** capsule **106,** implant retention area **16** contained within the capsule **106,** and the nose cone **28** towards the direction that is opposed from the direction that the bend portion **600** has deflected the distal end of the elongate shaft **12.** The deflection mechanism accordingly may be utilized to deflect the elongate shaft **12** in order to create height or distance from the distal end of the elongate shaft **12** to a desired implantation location.

The deflection mechanism has deflected a portion **614** of the elongate shaft **12** in the same plane (coplanar) that the bend portion **600** has deflected the distal end of the elongate shaft **12.**

The deflection mechanism may be utilized to allow the bend portions **600, 602** to bend the respective distal portions of the elongate shaft **12,** in a similar manner as shown in FIGS. 12A-C. Referring to FIG. 13C, for example, the deflection mechanism is deflecting the proximal portion **614** of the elongate shaft **12,** however, the bend portion **600** continues to deflect the distal end of the elongate shaft **12** in the direction shown in FIG. 13B, and the bend portion **602** deflects the bend portion **600** in a perpendicular direction as described with respect to FIG. 12C. The deflection mechanism in the form of the elongate sheath **610** continues to deflect a portion **614** of the elongate shaft **12** that is proximal the bend portion **600** and bend portion **602** to deflect the bend portions **600, 602** towards a direction that is away from the direction that the bend portion **600** has deflected the distal end of the elongate shaft **12.**

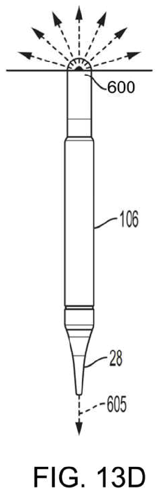

The deflection mechanism may be configured to provide multiple directions of deflection of the portion **614** of the elongate shaft **12** that is proximal the bend portion **600** and bend portion **602.** The deflection mechanism, in the form of the sheath **610,** for example, may be configured to rotate about the portion of the elongate shaft **12** that the sheath **610** extends over. Such rotation may move the position of the pull tether **612** relative to the elongate shaft **12** to cause the elongate shaft **12** to deflect towards the varied position of the pull tether **612.** As such, a variety of directions of deflection of the elongate shaft **12** may result. FIG. 13D for example, illustrates a front view of the elongate sheath showing multiple directions (via the arrows) that are opposed to the direction **605** that the bend portion **600** may be deflected towards.

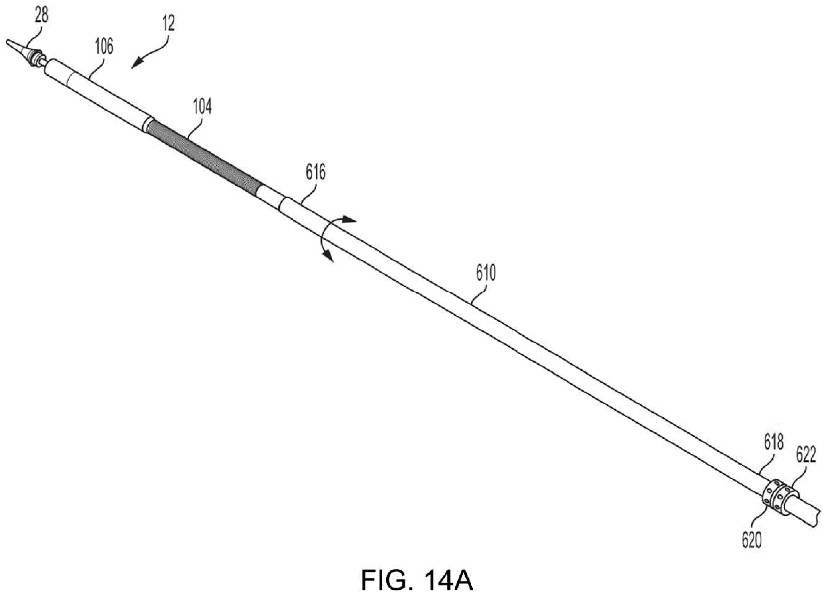

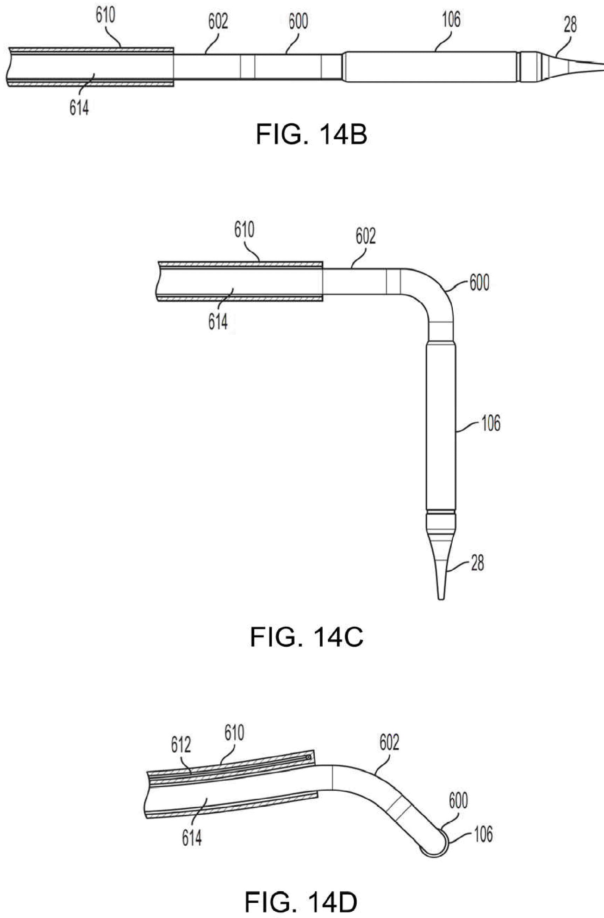

FIG. 14A illustrates a perspective view of the sheath **610** extending over the elongate shaft **12.** The sheath **610** may be utilized in lieu of, or in combination with the sheath **51** shown in FIG. 1. The sheath **610** may have a distal end **616** and a proximal end **618.** The proximal end **618** of the sheath **610** may be coupled to a rotation control housing **620** that may be utilized to control rotation of the sheath **610** about the elongate shaft **12.** A user, such as a surgeon or another user, may grasp the rotation control housing **620** to control rotation of the sheath **610** about the elongate shaft **12,** to thereby control the direction of deflection of the elongate shaft **12** caused by the sheath **610.** The proximal end **618** of the sheath **610** may alternatively or additionally couple to a deflection control housing **622,** which may be utilized to draw the pull tether **612** proximally to deflect the sheath **610,** and may be utilized to release the pull tether **612** in a distal direction to straighten the sheath **610.** The deflection control housing **622** may be configured for a user, such as a surgeon or another user, to grasp, to control deflection of the sheath **610.**

The control housings **620, 622** may be integrated to form a single control housing as desired. In one embodiment, the controls of the control housings **620, 622** may be integrated in the handle **14,** or may remain separate from the handle **14** as desired.

FIGS. 14B-D illustrate the deflection mechanism in the form of the sheath **610** rotated 90° about the elongate shaft **12** relative to the position shown in FIG. 13A. The sheath **610** may be rotated through use of the rotation control housing **620,** or through another method as desired. The relative position of the pull tether **612** has rotated 90° as shown in FIG. 14D. Referring to FIG. 14D, the sheath **610** may deflect the elongate shaft **12** towards a direction that is perpendicular to the direction that the bend portion **600** has deflected the distal end of the elongate shaft **12.** The sheath **610** may deflect the elongate shaft **12** in the same plane that the bend portion **602** deflects a portion of the elongate shaft **12** that is distal to the bend portion **602.**

The deflection mechanism in the form of the sheath **610** may have a variety of orientations relative to the elongate shaft **12,** at any angular position relative to the elongate shaft **12** as desired. As such, the deflection mechanism in the form of the sheath **610** may be configured to deflect the portion **614** of the elongate shaft **12** in multiple directions, which may or may not be perpendicular to the direction that the bend portion **600** has deflected the distal end of the elongate shaft **12.** The deflection mechanism in the form of the sheath **610** may deflect the portion **614** of the elongate shaft **12** towards a variety of directions that are opposed to the direction that the bend portion **600** has deflected the distal end of the elongate shaft **12,** which may include a direction that is directly opposite the direction that the bend portion **600** has deflected the distal end of the elongate shaft **12** (at 180° degrees) and a variety of other directions that are in between direct opposition (at 180° degrees) and a perpendicular direction (at 90°) (e.g., 135°, among others).

The deflection mechanism in the form of the sheath **610** may be configured to deflect the portion **614** of the elongate shaft **12** in a direction that is towards the direction that the bend portion **600** has deflected the distal end of the elongate shaft **12,** if the sheath **610** is rotated to provide such deflection.

The deflection mechanism in the form of the sheath **610** may be configured to vary the direction of deflection of the portion **614** not only via rotation of the sheath **610** but in embodiments may be configured with multiple pull tethers or other control devices that allow for varied directions of deflection of the sheath **610** without rotation of the sheath **610.** For example, if four equally spaced pull tethers (spaced 90° from each other) are utilized with the sheath **610,** then a combination of movement of the pull tethers may provide a variety of directions of deflection of the sheath **610.** Other configurations may be utilized to vary the direction of deflection of the sheath **610.** At least one pull tether may be utilized in embodiments.

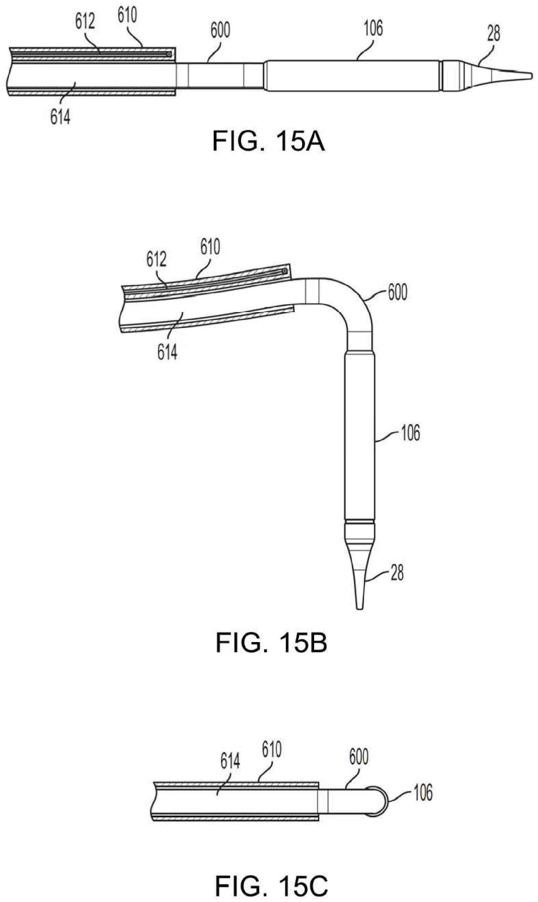

The embodiments of FIGS. 13A-14D illustrate an elongate shaft **12** having two bend portions **600, 602** configured to bend in perpendicular planes. However, the configuration and use of the bend portions **600, 602** may be varied in other embodiments as desired. For example, FIGS. 15A-16C illustrate an embodiment in the bend portion **602** has been excluded, and where the sheath **610** controls deflection of the elongate shaft **12** in lieu of the bend portion **602.** The sheath **610** accordingly may be configured to deflect the elongate shaft **12** towards a direction that is opposed to the direction that the bend portion **600** has deflected the distal end of the elongate shaft **12,** which may include a direction that is directly opposite the direction that the bend portion **600** has deflected the distal end of the elongate shaft **12** (at 180° degrees) and a variety of other directions that are in between direct opposition (at 180° degrees) and a perpendicular direction (at 90°) (e.g., 135°, among others). FIGS. 15A-C illustrate the sheath **610** deflecting the portion of the elongate shaft **12** to deflect the bend portion **600** towards a direction that is opposed to the direction that the bend portion **600** has deflected the distal end of the elongate shaft **12.**

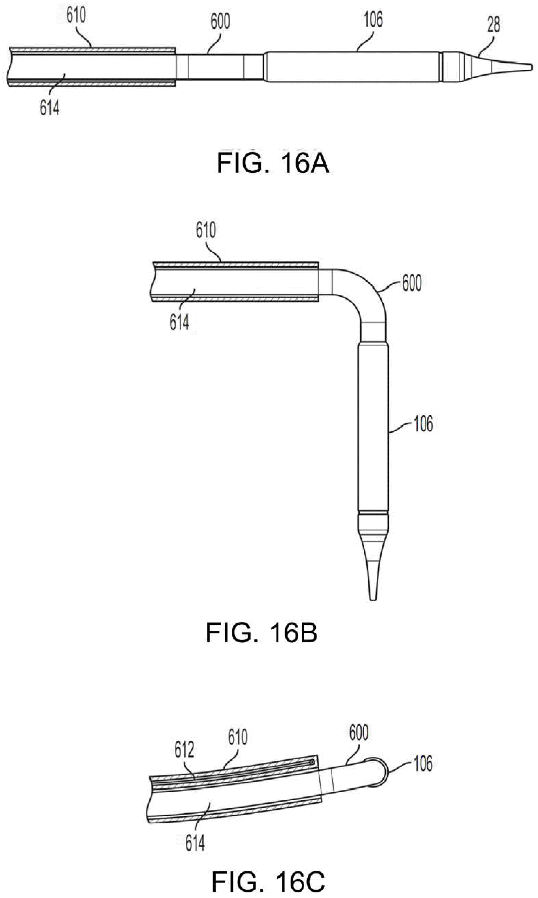

The sheath **610** may be rotated from the orientation shown in FIGS. 15A-C, to vary the direction of deflection of the portion **614.** FIGS. 16A-C illustrate the sheath **610** rotated 90° from the orientation shown in FIGS. 15A-C, to deflect the portion **614** in a plane that is perpendicular to the plane of deflection of the bend portion **600.** As discussed with respect to FIGS. 13A-14D, the sheath **610** may in other embodiments be configured with multiple pull tethers or other control devices that allow for varied directions of deflection of the sheath **610** without rotation of the sheath **610.**

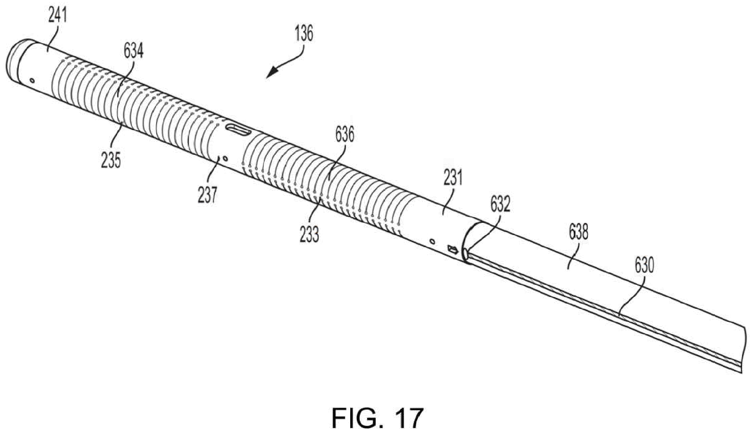

Other forms of deflection mechanisms may be utilized. For example, FIG. 17 illustrates an embodiment of a deflection mechanism in the form of a pull tether **630.** The pull tether **630** may have a distal end **632** that is coupled to a portion of the elongate shaft **12,** for example, the rail shaft **132.** The rail shaft **132** may extend over an inner shaft as disclosed herein, and may have an outer sheath extending over the rail shaft **132** as disclosed herein. The distal end **632** may couple to the rail proximal shaft **134** or another portion of the rail shaft **132** that is proximal the rail hypotube **136** or the bend portions **634, 636** of the rail shaft **132.** For example, as shown in FIG. 17, the distal end **632** may couple to a portion that is proximal the uncut (or unslotted) hypotube section **231.**

The pull tether **630** may be configured to be retracted to deflect the portion **638** of the rail shaft **132,** and thus the elongate shaft **12,** that is proximal the bend portions **634, 636.** As such, the bend portion **634** may be configured to deflect the distal end of the elongate shaft **12** in a direction, and the pull tether **630** may be configured to deflect the elongate shaft **12** to deflect the bend portions **634, 636** towards a direction that is opposed to the direction that the bend portion **634** has deflected the distal end of the elongate shaft **12.** The pull tether **630** may be coupled to the rail shaft **132** at a position and with an orientation that opposes the direction that the bend portion **634** has deflected the distal end of the elongate shaft **12** when the pull tether **630** is retracted.

A single pull tether **630** is shown in FIG. 17, however multiple pull tethers may be utilized in other embodiments as desired. For example, if four equally spaced pull tethers (spaced 90° from each other) are coupled to the rail shaft **132,** then a combination of movement of the pull tethers may provide a variety of directions of deflection of the elongate shaft **12.** Other configurations may be utilized to vary the direction of deflection of the elongate shaft **12.** The one or more pull tethers accordingly may be configured to deflect the elongate shaft **12** towards a direction that is opposed to the direction that the bend portion **634** has deflected the distal end of the elongate shaft **12,** which may include a direction that is directly opposite the direction that the bend portion **634** has deflected the distal end of the elongate shaft **12** (at 180° degrees) and a variety of other directions that are in between and include direct opposition (at 180° degrees) and a perpendicular direction (at 90°) (e.g., 135°, among others).

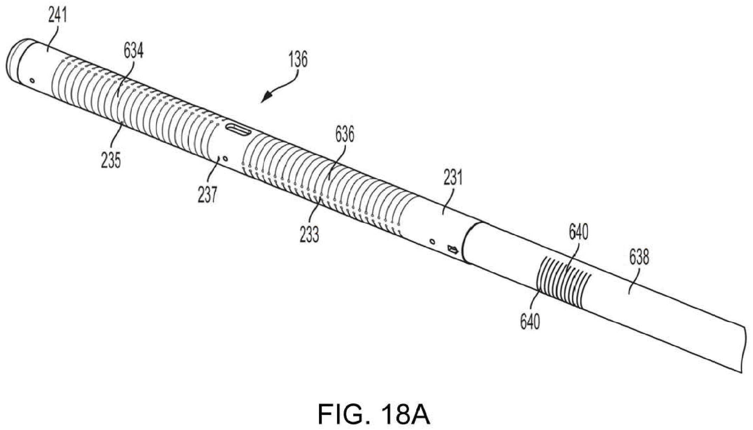

FIGS. 18A-B illustrate an embodiment of a deflection mechanism including cuts **640** in a portion of the elongate shaft **12** and a pull shaft **642** that may be retracted to cause the elongate shaft **12** to deflect at the location of the cuts **640.** Referring to FIG. 18A, the cuts **640** may be positioned on the rail shaft **132** at a desired location. Such a location may be proximal the rail hypotube **136** or the bend portions **634, 636** of the rail shaft **132.** For example, as shown in FIG. 18A, the cuts **640** may be proximal the uncut (or unslotted) hypotube section **231.**

The cuts **640** may have a configuration that biases the rail shaft **132** to deflect at the cuts **640** and in a direction that is away from the direction that the bend portion **634** has deflected the distal end of the elongate shaft **12.**

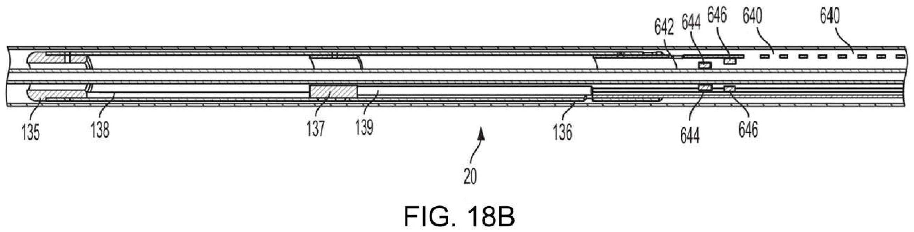

Referring to FIG. 18B, a cross sectional view of the rail shaft **132** is shown. The deflection mechanism may include the inner shaft or pull shaft **642,** which may be positioned within the rail shaft **132.** The pull shaft **642** may be positioned between the rail shaft **132** and an inner shaft such as the inner shaft assembly **18** or the nose cone assembly **31.** In other embodiments, the inner shaft or pull shaft **642** may be provided in other locations.

The inner shaft or pull shaft **642** may include a stopper **644** coupled thereto. The rail shaft **132,** and particularly the portion of the rail shaft **132** distal the cuts **640** may include a stopper **646.** The deflection mechanism may be configured that as the pull shaft **642** is drawn proximally, the stopper **644** contacts the stopper **646** and applies a proximal force to the rail shaft **132** and particularly the portion of the rail shaft **132** including the cuts **640.** The cuts **640,** providing a biased direction of deflection, may cause the rail shaft **132** and accordingly the elongate shaft **12** to deflect in this direction of deflection, which is in a direction that is opposed to the direction that the bend portion **634** has deflected the distal end of the elongate shaft **12.** The pull shaft **642** may then be moved distally to reduce the force between the stoppers **644, 646** to cause the rail shaft **132** to straighten. FIG. 18B shows the stoppers **644, 646** separate from each other, however, the inner shaft or pull shaft **642** may be drawn proximally for the stoppers **644, 646** to contact each other.

A single pull shaft **642** is shown in FIG. 18B, however multiple pull shafts may be utilized in other embodiments as desired. For example, if four equally spaced pull shafts (spaced 90° from each other) with corresponding stoppers are utilized, then a combination of movement of the pull shafts may provide a variety of directions of deflection of the elongate shaft **12.** The cut pattern may be provided such that a variety of directions of deflection are possible. Other configurations may be utilized to vary the direction of deflection of the elongate shaft **12.** The one or more pull shafts accordingly may be configured to deflect the elongate shaft **12** to deflect the bend portions **634, 636** towards a direction that is opposed to the direction that the bend portion **634** has deflected the distal end of the elongate shaft **12,** which may include a direction that is directly opposite the direction that the bend portion **634** has deflected the distal end of the elongate shaft **12** (at 180° degrees) and a variety of other directions that are in between and include direct opposition (at 180° degrees) and a perpendicular direction (at 90°) (e.g., 135°, among others).

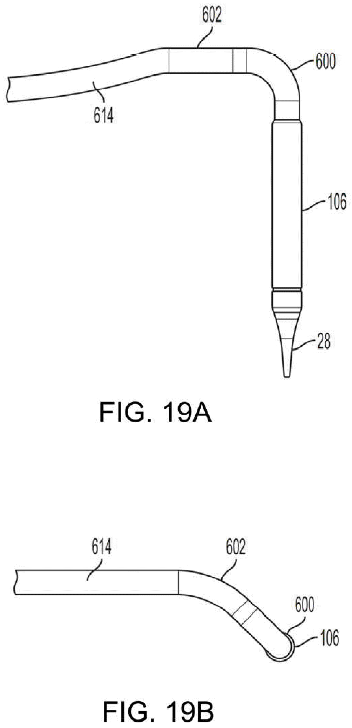

FIGS. 19A-B illustrate an external view of the embodiments of FIGS. 17-18B. The sheath 610 may or may not be utilized with the deflection mechanisms shown in FIGS. 17-18B. As such, the outer sheath assembly **22** may comprise the outer surface of the elongate shaft **12,** with the deflection mechanisms contained within the outer sheath assembly **22.**

As shown in FIG. 19A, the bend portion **600** may deflect the distal end of the elongate shaft **12** to a direction. The deflection mechanism may deflect the proximal portion **614** of the elongate shaft **12** to deflect the bend portion **600** towards a direction that is opposed to the direction of the distal end of the elongate shaft **12.** FIG. 19B illustrates that the bend portions **600, 602** may continue to operate to deflect the respective distal portions of the elongate shaft **12.**

The deflection mechanisms may be utilized to provide for additional or varied movement of the elongate shaft **12.** Such additional or varied movement may be desired for a variety of reasons, which may include a variety of patient anatomies to be navigated with the distal end of the elongate shaft **12** or varied uses of the elongate shaft **12.**

The deflection mechanisms may be utilized to move the elongate shaft **12** for delivery of a replacement heart valve, which may include a replacement tricuspid valve. Although many of the embodiments herein are discussed with respect to a replacement tricuspid valve, the deflection mechanisms may be utilized for a variety of other implementations including delivery of mitral replacement valves, or aortic or pulmonary valves, or for valve repair procedures, including tricuspid or mitral valve repair or aortic or pulmonary valve repair.

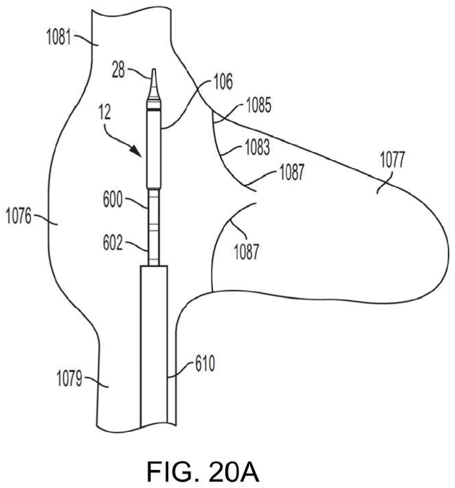

FIGS. 20A-21 illustrate a use of the elongate shaft **12** to treat a patient's tricuspid valve. The elongate shaft **12** may be passed into the patient's body in an endovascular manner, which may include percutaneous entry of the patient's vasculature. For example, the elongate shaft **12** may be entered into the ipsilateral femoral vein and advanced toward the right atrium **1076.** Other entry methods may be utilized in other embodiments, including a transjugular approach, or other approaches including transapical approaches.

As shown in FIG. 20A, the elongate shaft **12** may be advanced through the inferior vena cava **1079** to approach or reach the right atrium **1076** of the patient's heart. The right ventricle **1077,** the tricuspid valve **1083** including tricuspid valve leaflets **1087,** the tricuspid valve annulus **1085,** and the superior vena cava **1081** are also shown.

The delivery system may include use of the deflection mechanisms discussed herein. As shown in FIG. 20A, the deflection mechanism in the form of the sheath **610** may be utilized, however it is understood that other forms of deflection mechanisms may be utilized, including the deflection mechanisms shown in FIGS. 17-19B.

The elongate shaft **12** may be advanced towards the right atrium **1076,** with the distal end of the elongate shaft **12** to be deflected such that the capsule **106** and thus the implant retention area **16** are oriented to deploy the implant contained therein to the tricuspid valve **1083** in the desired manner. As represented in FIG. 20A, the distal end of the elongate shaft **12** may require deflection to a direction towards the tricuspid valve **1083,** to align the distal end of the elongate shaft **12** and the capsule **106** (and the deployment port at the distal end of the capsule for the implant to be deployed from) with the central axis of the tricuspid valve **1083.** For other methods of deployment, other directions of deflection may be desired.

The bend portions **600, 602** may be utilized to deflect the distal end of the elongate shaft **12** to the desired direction. The bend portions **600, 602** may be configured to deflect the distal end of the elongate shaft in perpendicular planes, to provide two planes of deflection. The bend portions **600, 602** may be configured similarly as shown in FIG. 6C, with the proximal bend portion **602** configured to deflect the distal portions of the elongate shaft **12** in a rightward (or anterior) direction relative to a downward (or ventricular) direction of deflection of the distal bend portion **600.** Such a configuration may account for the position of the tricuspid valve **1083** relative to the inferior vena cava **1079** within a human heart.

Additional movement, however, may be provided by the deflection mechanisms disclosed herein. The deflection mechanism in the form of the sheath **610** may be utilized to deflect a proximal portion of the elongate shaft **12** to deflect the bend portions **600, 602** in a direction opposed to the direction that the bend portion **600** has deflected the distal end of the elongate shaft **12.** Such a deflection may include deflecting the proximal portion of the elongate shaft **12** and the bend portions **600, 602** in an atrial direction (or providing a height from the tricuspid valve **1083**)**.** The capsule **106** and distal end of the elongate shaft **12** may also be deflected in an atrial direction (or providing a height from the tricuspid valve 1083).

The deflection mechanism may be utilized to account for a geometry of the patient's anatomy, which may include the geometry of the right atrium **1076,** the size and relative position of the tricuspid valve **1083,** and the geometry of the inferior vena cava **1079.** For example, as shown in FIG. 20A, the distance of the bend portion **600** to the distal end of the elongate shaft **12** may be such that the bending radius of the elongate shaft **12** distal the bend portion **600** is too large to properly direct the distal end of the elongate shaft **12** to the tricuspid valve **1083,** depending on the geometry of the patient's right atrium **1076.** The deflection mechanism accordingly may be utilized to deflect the bend portion **600** towards a direction opposed to the direction that the bend portion **600** deflects the distal end of the elongate shaft **12.**

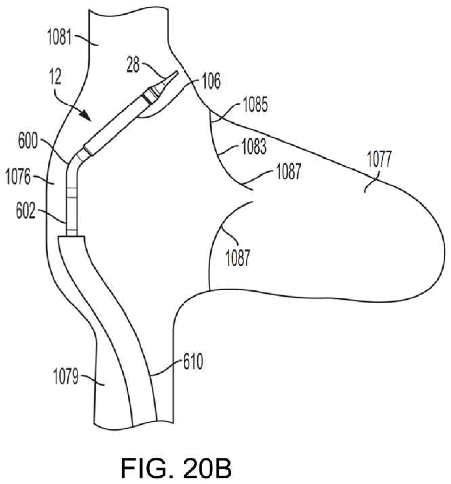

Referring to FIG. 20B, the deflection mechanism in the form of the sheath **610** may deflect the proximal portion of the elongate shaft **12,** as discussed herein. The deflection of the proximal portion of the elongate shaft **12** may occur wholly or partially (at least partially) within the patient's inferior vena cava **1079.** The deflection may move the bend portions **600, 602** to create height from the tricuspid valve **1083** in a direction away from the tricuspid valve. As such, the distal end of the elongate shaft **12** may have greater clearance space for the bend portion **600** to deflect the distal end of the elongate shaft **12** towards the tricuspid valve **1083.** As shown in FIG. 20B, the deflection mechanism may form a curve of the proximal portion of the sheath, although other forms of deflection may result. The bend portion **600** has begun deflection of the distal end of the elongate shaft **12** in FIG. 20B.

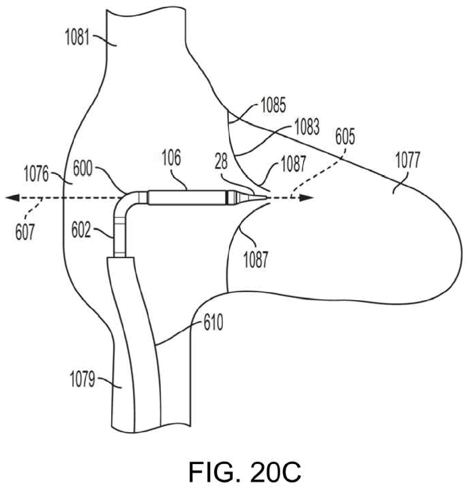

Referring to FIG. 20C, the bend portion **600** has deflected the distal end of the elongate shaft **12** to a direction **605.** The direction may be aligned with the axis of the tricuspid valve **1083** or otherwise may be directed in a desired orientation. The deflection mechanism in the form of the sheath **610** has deflected the proximal portion of the elongate shaft **12** to deflect the bend portion **600** in a direction **607** that is opposed to the direction **605.** As such, the capsule **106** has increased height from the tricuspid valve 1083, to allow for deployment of the implant contained therein.

The deflection mechanism in the form of the sheath **610** may provide various directions of deflection of the proximal portion of the elongate shaft **12,** and correspondingly various directions of deflection of the bend portions **600, 602,** the capsule **106,** and the distal end of the elongate shaft **12.** As discussed with respect to FIGS. 14A-D, for example, the sheath **610** may provide for various directions of deflection, including perpendicular to the direction of deflection provided by the bend portion **600,** and towards the direction of deflection provided by the bend portion **600.** Such various directions of deflection may allow for additional maneuverability and variation of trajectory of the distal end of the elongate shaft **12** within the right atrium, and within the inferior vena cava **1079** or other area that the elongate shaft **12** is positioned within. The deflection mechanism in the form of the sheath **610** may provide for deflection in both the atrial and ventricular directions, and for various other directions.

The operation of the deflection mechanism shown in FIGS. 20A-C is not limited to the sheath **610** shown in FIGS. 13A-14D, but includes use of the deflection mechanisms shown in FIGS. 15A-19B as well. For example, the proximal bend portion **602** may be excluded, with the sheath **610** providing deflection of this portion of the elongate shaft **12** as discussed with respect to FIGS. 15A-16C. Further, the deflection mechanism may be positioned within the outer sheath assembly **22** as discussed with respect to the embodiments of FIGS. 17-19B. Various directions of deflection in both the atrial and ventricular directions, and various other directions may result.

The deflection mechanisms may be utilized to deflect the proximal portion of the elongate shaft **12** in one or more planes that are not perpendicular to the plane that the bend portion **600** deflects the distal end of the elongate shaft **12.**

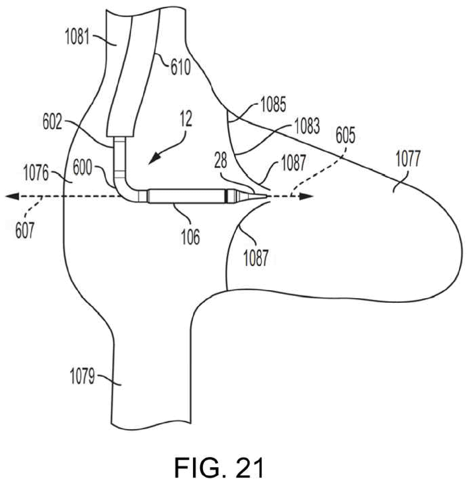

FIG. 21 illustrates use of the deflection mechanism in an approach from the superior vena cava **1081.** The approach may be a transjugular approach, or via another entry point into the patient's body. The bend portions **600, 602** may be configured similarly as shown in FIG. 6B, with the proximal bend portion **602** configured to deflect the distal portions of the elongate shaft **12** in a leftward (or posterior) direction relative to a downward (or ventricular) direction of deflection of the distal bend portion **600.** Such a configuration may account for the position of the tricuspid valve **1083** relative to the superior vena cava **1081** within a human heart. A method may include passing a delivery apparatus for an implant into a patient's right atrium.

The deflection mechanism, similarly as shown in FIGS. 20A-C, may deflect the proximal portion of the elongate shaft **12** to deflect the bend portion **600** in a direction **607** that is opposed to the direction **605** that the bend portion **600** has deflected the distal end of the elongate shaft **12.** Similarly, as discussed with respect to FIGS. 13A-19B, other forms of deflection mechanisms may be utilized and other directions of deflection may result.

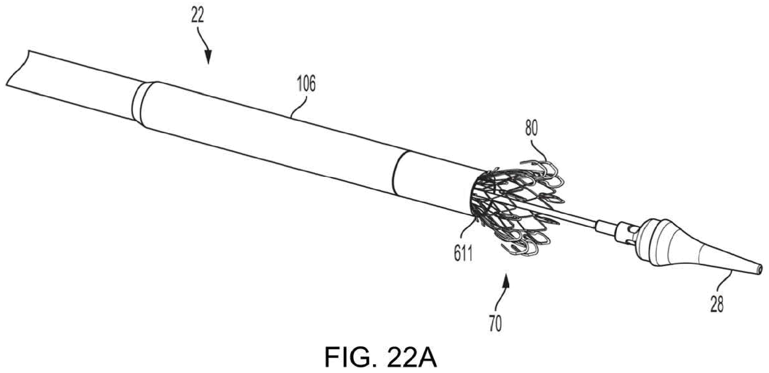

The implant **70** contained within the capsule **106** may be deployed to be positioned within the tricuspid valve annulus **1085,** to replace the native tricuspid valve **1083.** Upon the distal end of the elongate shaft **12** being oriented as desired relative to the native tricuspid valve **1083,** a release mechanism may be utilized to deploy the implant **70** from the deployment port **611** at the distal end of the capsule **106.** A height of the deployment port **611** relative to the valve may be varied by deflecting the delivery apparatus within an inferior vena cava or a superior vena cava. FIGS. 22A-C illustrate the release mechanism of the delivery system **10.** During the initial insertion of the implant **70** and the delivery system **10** into the body, the implant **70** can be located within the system **10,** similar to as shown in FIG. 2A. The distal end **303** of the implant **70,** and specifically the distal anchors **80,** are restrained within the capsule **106** of the outer sheath assembly **22,** thus preventing expansion of the implant **70.** Similar to what is shown in FIG. 2A, the distal anchors **80** can extend distally when positioned in the capsule. The proximal end **301** of the implant **70** is restrained within the capsule **106** and within a portion of the inner retention member **40** and thus is generally constrained between the capsule **106** and the inner retention member **40.**

Once the implant **70** is loaded into the delivery system **10,** a user can thread a guide wire into a patient to the desired location. The guide wire passes through the lumen of the nose cone assembly **31,** and thus the delivery system **10** can be generally advanced through the patient's body following the guide wire. The delivery system **10** can be advanced by the user manually moving the handle **14** in an axial direction. In some embodiments, the delivery system **10** can be placed into a stand while operating the handle **14** controls.

Once generally in heart, the user can begin the steering operation of the rail assembly **20,** and particularly the bend portions **600, 602** using the distal pull wire knob **206** and/or the proximal pull wire knob **208.** By turning either of the knobs, the user can provide flexing/bending of the rail assembly **20** (either on the distal end or the proximal end), thus bending the distal end of the delivery system **10** in one, two, or more locations into the desired configuration. As discussed above, the user can provide multiple bends in the rail assembly **20** to direct the delivery system **10** towards the tricuspid valve. In particular, the bends of the rail assembly **20** can direct a distal end of the delivery system **10,** and thus the capsule **106,** along the center axis passing through the native tricuspid valve and towards the tricuspid valve. Thus, when the outer sheath assembly **22,** mid shaft assembly **21,** inner assembly **18,** and nose cone assembly **31** are together advanced over the rail assembly **20** with the compressed implant **70,** the capsule **106** proceed directly in line with the axis for proper release of the implant **70.**

The user may utilize the deflection mechanisms as well, which may create height from the native tricuspid valve or may otherwise orient the distal end of the elongate shaft **12** as desired. The height of a bend portion of the elongate shaft **12** may be varied from the tricuspid valve.

The system **10** can be positioned to a particular location in a patient's body, such as at the native tricuspid valve, through the use of the bend portions and deflection mechanisms discussed herein or other techniques.

The user can also rotate and/or move the handle **14** itself in a stand for further fine tuning of the distal end of the delivery system **10.** The user can continually turn the proximal and/or distal pull wire knobs **208/206,** as well as moving the handle **14** itself, to orient the delivery system **10** for release of the implant **70** in the body. The user can also further move the other assemblies relative to the rail assembly **20,** such as proximally or distally.

The user may utilize control mechanisms such as the rotation control housing **620** or deflection control housing **622** as shown in FIG. 14A or other control mechanisms to control operation of the deflection mechanism.

Upon the distal end of the elongate shaft **12** being oriented as desired, the user may rotate the depth knob **212.** As discussed, rotation of this knob **212** together advances the inner shaft assembly **18,** mid shaft assembly **21,** outer sheath assembly **22,** and nose cone assembly **31** over/through the rail assembly **20** while the implant **70** remains in the compressed configuration within the implant retention area **16.** Due to the rigidity of, for example, either the inner shaft assembly **18,** the mid shaft assembly **21,** and/or the outer sheath assembly **22,** these assemblies proceed straight forward in the direction aligned by the rail assembly **20.**

Once in the release position, the user can rotate the outer sheath knob **210,** which individually translates the outer sheath assembly **22** (and thus the capsule **106**) with respect to the other assemblies, in particular the inner assembly **18,** in a proximal direction towards the handle **14** as shown in FIG. 22A. By doing so, the distal end **303** of implant **70** is uncovered in the body, allowing for the beginning of expansion. At this point, the distal anchors **80** can flip proximally and the distal end **303** begins to expand radially outwardly. For example, if the system **10** has been delivered to a native tricuspid valve location, the distal anchors **80** expand radially outwardly within the right ventricle. The distal anchors **80** can be located above the papillary heads, but below the tricuspid valve annulus and tricuspid valve leaflets.

In some embodiments, the distal anchors **80** may contact and/or extend between the chordae in the right ventricle, as well as contact the leaflets, as they expand radially. In some embodiments, the distal anchors **80** may not contact and/or extend between the chordae or contact the leaflets. Depending on the position of the implant **70,** the distal ends of the distal anchors **80** may be at or below where the chordae connect to the free edge of the native leaflets.

As shown in the illustrated embodiment, the distal end **303** of the implant **70** is expanded outwardly. It should be noted that the proximal end **301** of the implant **70** can remain covered by the outer retention ring during this step such that the proximal end **301** remains in a radially compacted state. At this time, the system **10** may be withdrawn proximally so that the distal anchors **80** capture and engage the leaflets of the tricuspid valve, or may be moved proximally to reposition the implant **70.** For example, the assemblies may be proximally moved relative to the rail assembly **20.** Further, the deflection mechanisms may be utilized to draw the elongate shaft **12** proximally relative to the tricuspid valve. Further, the system **10** may be torqued, which may cause the distal anchors **80** to put tension on the chordae through which at least some of the distal anchors may extend between. However, in some embodiments the distal anchors **80** may not put tension on the chordae. In some embodiments, the distal anchors **80** may capture the native leaflet and be between the chordae without any further movement of the system **10** after withdrawing the outer sheath assembly **22.**

During this step, the system **10** may be moved proximally or distally to cause the distal or ventricular anchors **80** to properly capture the native tricuspid valve leaflets. This can be done by moving the outer sheath assembly **22,** mid shaft assembly **21,** inner assembly **18,** and nose cone assembly **31** with respect to the rail assembly **20.** In particular, the tips of the ventricular anchors **80** may be moved proximally to engage a ventricular side of the native annulus, so that the native leaflets are positioned between the anchors **80** and the body of the implant **70.** When the implant **70** is in its final position, there may or may not be tension on the chordae, though the distal anchors **80** can be located between at least some of the chordae.

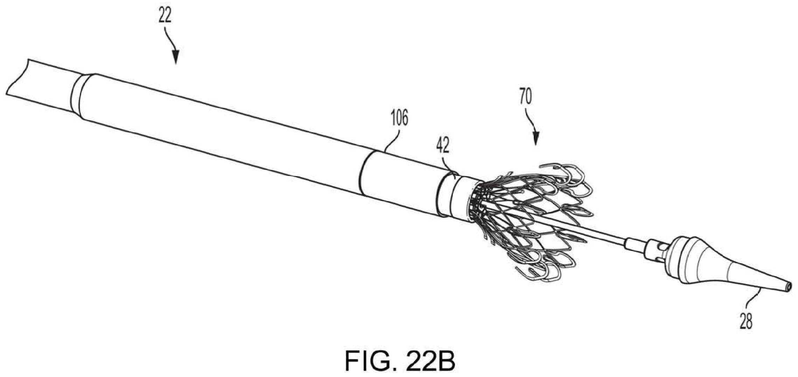

The proximal end **301** of the implant **70** will remain in the outer retention ring **42** after retraction of the capsule **106.** The capsule **106** may surround the implant retention area and be retracted proximally to deploy the implant. As shown in FIG. 22B, once the distal end **303** of the implant **70** is fully expanded (or as fully expanded as possible at this point), the outer retention ring **42** can be individually withdrawn proximally with respect to the other assemblies, in particular relative to the inner assembly **18,** to expose the inner retention member **40,** thus beginning the expansion of the proximal end **301** of the implant **70.** For example, in a tricuspid valve replacement procedure, after the distal or ventricular anchors **80** are positioned between at least some of the chordae tendineae and/or engage the native tricuspid valve annulus, the proximal end **301** of the implant **70** may be expanded within the right atrium.

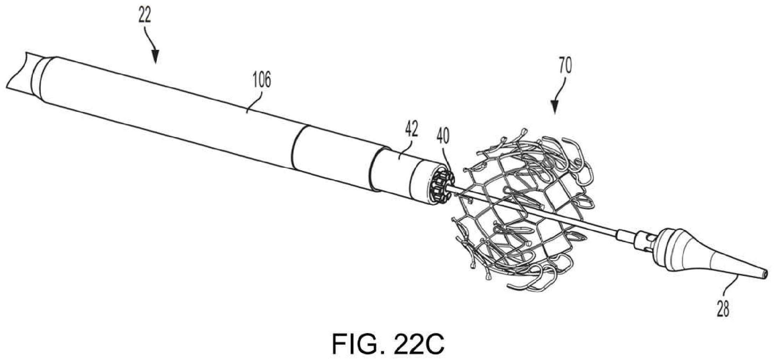

The outer retention ring **42** can be moved proximally such that the proximal end **310** of the implant **70** can radially expand to its fully expanded configuration as shown in FIG. 22C. The implant **70** may be deployed to the valve. After expansion and release of the implant **70,** the inner assembly **18,** nose cone assembly **31,** mid shaft assembly **21,** and outer sheath assembly **22** can be simultaneously withdrawn proximally along or relative to the rail assembly **20** back to their original position. In some embodiments, they are not withdrawn relative to the rail assembly **20** and remain in the extended position. Further, the nose cone **28** can be withdrawn through the center of the expanded implant **70** and into the outer sheath assembly **22,** such as by proximally translating the knob **216.** The system **10** can then be removed from the patient.

In some embodiments, the implant **70** can be delivered under fluoroscopy so that a user can view certain reference points for proper positioning of the implant **70.** Further, echocardiography can be used for proper positioning of the implant **70.**

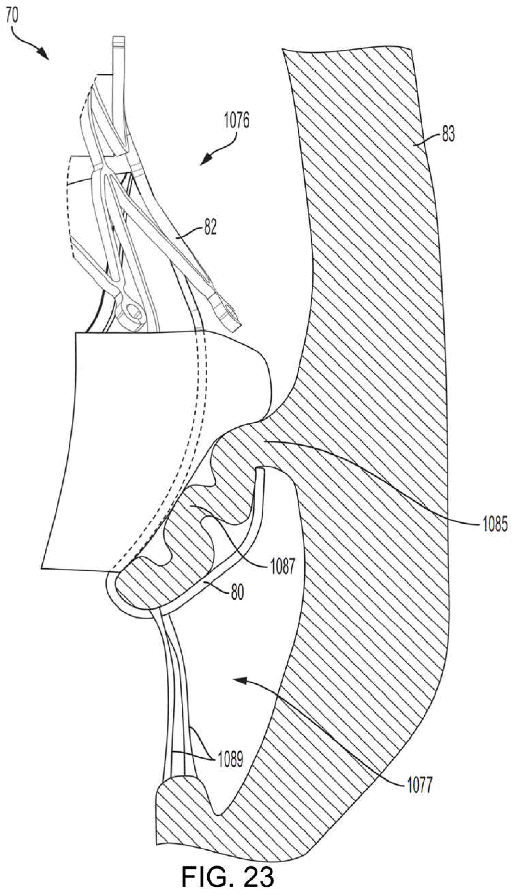

Reference is now made to FIG. 23 which illustrates a schematic representation of a portion of an embodiment of a replacement heart valve (implant **70**) positioned within a native tricuspid valve of a heart **83.** A portion of the native tricuspid valve is shown schematically and represents typical anatomy, including a right atrium **1076** positioned above an annulus **1085** and a right ventricle **1077** positioned below the annulus **1085.** The right atrium **1076** and right ventricle **1077** communicate with one another through a tricuspid annulus **1085.** Also shown schematically in FIG. 23 is a native tricuspid leaflet **1087** having chordae tendineae **1089** that connect a downstream end of the tricuspid leaflet **1087** to the papillary muscle of the right ventricle **1077.** The portion of the implant **70** disposed upstream of the annulus **1085** (toward the right atrium **1076**) can be referred to as being positioned supra-annularly. The portion generally within the annulus **1085** is referred to as positioned intra-annularly. The portion downstream of the annulus **1085** is referred to as being positioned sub-annularly (toward the right ventricle **1077**).

As shown in FIG. 23, the replacement heart valve (e.g., implant **70**) can be positioned so that the tricuspid annulus **1085** is located between the distal anchors **80** and the proximal anchors **82.** In some situations, the implant **70** can be positioned such that ends or tips of the distal anchors **80** contact the annulus **1085** as shown, for example, in FIG. 23. In some situations, the implant **70** can be positioned such that ends or tips of the distal anchors **80** do not contact the annulus **1085.** In some situations, the implant **70** can be positioned such that the distal anchors **80** do not extend around the leaflet **1087.**

As illustrated in FIG. 23, the replacement heart valve or implant **70** can be positioned so that the ends or tips of the distal anchors **80** are on a ventricular side of the tricuspid annulus **1085** and the ends or tips of the proximal anchors **82** are on an atrial side of the tricuspid annulus **1085.** The distal anchors **80** can be positioned such that the ends or tips of the distal anchors **80** are on a ventricular side of the native leaflets beyond a location where chordae tendineae **1089** connect to free ends of the native leaflets. The distal anchors **80** may extend between at least some of the chordae tendineae **1089** and, in some situations such as those shown in FIG. 23, can contact or engage a ventricular side of the annulus **1085.** It is also contemplated that in some situations, the distal anchors **80** may not contact the annulus **1085,** though the distal anchors **80** may still contact the native leaflet **1087.** In some situations, the distal anchors **80** can contact tissue of the right ventricle **1077** beyond the annulus **1085** and/or a ventricular side of the leaflets.

Upon deployment of the implant **70** as desired, the deflection mechanisms disclosed with respect to FIGS. 13A-19B may be utilized to deflect the elongate shaft **12** to allow for removal of the elongate shaft **12** from the patient's heart.

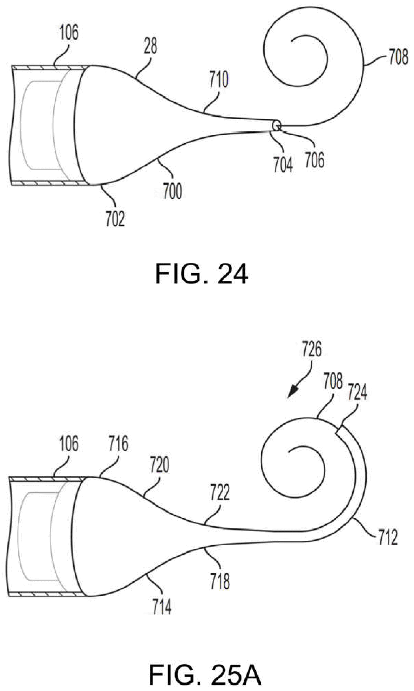

FIG. 24 illustrates a side perspective view of the nose cone **28** forming the tip of the elongate shaft **12.** The nose cone **28** includes a tip body **700** that closes the end of the capsule **106** (shown in partial cross section) and is positioned distal of the capsule **106.** The tip body **700** includes a proximal portion **702** and a distal portion **704** and tapers from the proximal portion **702** to the distal portion **704.** An opening **706** is positioned at the distal portion **704** of the tip body **700** for the guide wire **708** to pass through. The distal portion **704** of the tip body **700** may include a stiff protruding section **710** that is tapered. The tapered profile of the stiff protruding section may allow for ease of entry into the patient's vasculature and may assist to pass the tip of the elongate shaft **12** within the patient's vasculature.

Notably, however, the stiff protruding section **710** may interfere with or potentially damage a portion of the patient's body upon contact with the stiff protruding section **710.** For example, if the nose cone **28** is passed into the right ventricle of the patient's heart, potentially the stiff protruding section **710** may impact and potentially puncture or otherwise damage the interior of the right ventricle. Notably, there is also a possibility of kinking with the guide wire **708** at the opening **706.** The length of the stiff protruding section **710** may also inhibit maneuverability of the distal end of the elongate shaft **12.**

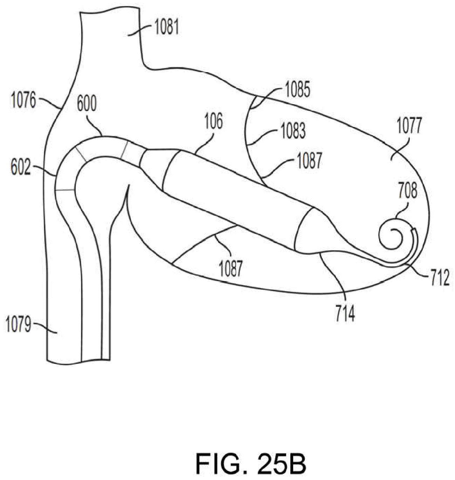

FIGS. 25A and 25B illustrate an embodiment of a distal tip of the elongate shaft **12** including a flexible sheath **712** that extends distally and is configured to bend about a portion of a guide wire **708.** The distal tip may include a tip body **714** having a proximal portion **716** and a distal portion **718** and the distal tip may have an outer surface **720** that tapers in a direction from the proximal portion **716** to the distal portion **718.** The tip body **714** may be positioned distal of the capsule **106** and may be positioned at and close the distal end of the capsule **106.** The tip body **714** may be movable relative to the capsule **106** to allow an implant **70** enclosed by the capsule **106** to be deployed from the capsule **106.**

The outer surface **720** may taper from a proximal portion **716** of the tip body **714** to a proximal portion **722** of the flexible sheath **712.** The flexible sheath **712** may extend from the proximal portion **722** of the flexible sheath **712** to the distal end **724** of the flexible sheath **712.** The flexible sheath **712** may have a cylindrical shape from the proximal portion **722** of the flexible sheath **712** to the distal end **724** of the flexible sheath **712.**

The flexible sheath **712** may have a length that is configured to extend over a leading curve of the guide wire **708** for a guide wire **708** having a curved configuration **726** at the end of the guide wire **708.** The flexible sheath **712** may thus cover the leading curve of the guide wire **708,** to reduce the possibility of injury due to contact between the guide wire and a portion of the patient's body. FIG. 25B, for example, illustrates the distal tip within the patient's right ventricle **1077.** The flexible sheath **712** is bent about the guide wire **708** when the guide wire **708** is positioned within the right ventricle. The flexible sheath **712** covers the portion of the guide wire **708** that may otherwise contact the interior wall of the patient's right ventricle **1077.** Further, the flexible sheath **712** is flexible, to reduce the possibility of puncture or other interference with the interior wall of the patient's right ventricle **1077.** The curvature of the flexible sheath **712** along the guide wire **708** additionally may reduce the possibility of kinking of the guide wire **708.**

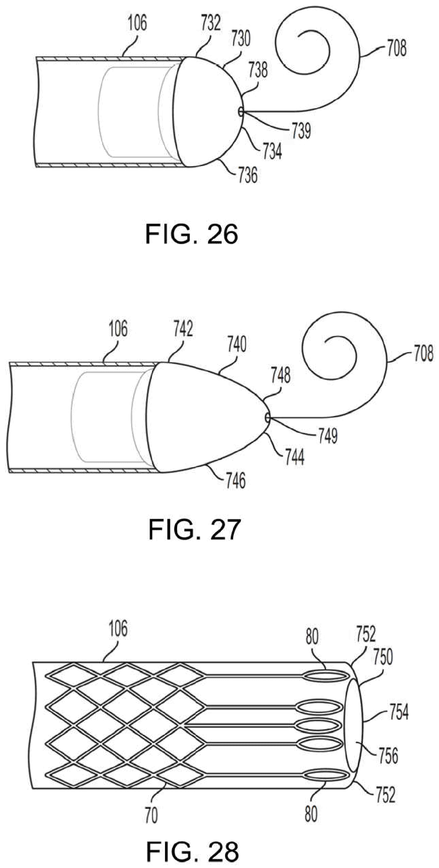

FIGS. 26-28 illustrate embodiments of distal tips of elongate shafts **12** that may reduce the distal profile of the elongate shafts **12.** Such features may be utilized to allow the elongate shafts **12** to more easily navigate or be deflected in a variety of vascular geometries. For example, in the methods shown in FIGS. 20A-21, a reduced distal profile of the elongate shaft **12** may allow for greater maneuverability of the elongate shaft **12** within and towards the right atrium **1076.**

FIG. 26 illustrates an embodiment of a distal tip of the elongate shaft **12** having a dome shape. The distal tip may include a tip body **730** having a proximal portion **732** and a distal portion **734** and may have an outer surface **736** that tapers in a direction from the proximal portion **732** to the distal portion **734.** The tip body **730** may be positioned distal of the capsule **106** and may be positioned at and close the distal end of the capsule **106.** The tip body **730** may be movable relative to the capsule **106** to allow an implant **70** enclosed by the capsule **106** to be deployed from the capsule **106.** The dome shaped tip body may form a convex profile of the distal end **738** of the distal tip. The outer surface **736** may be convex from the proximal portion **732** of the tip body **730** to the distal end **738** of the tip body **730.** The tip body **730** may include an opening **739** at its distal end **738** for a guide wire **708** to pass through.

FIG. 27 illustrates an embodiment of a distal tip of the elongate shaft **12** having a parabolic shape. The distal tip may include a tip body **740** having a proximal portion **742** and a distal portion **744** and may have an outer surface **746** that tapers in a direction from the proximal portion **742** to the distal portion **744.** The tip body **740** may be positioned distal of the capsule **106** and may be positioned at and close the distal end of the capsule **106.** The tip body **740** may be movable relative to the capsule **106** to allow an implant **70** enclosed by the capsule **106** to be deployed from the capsule **106.** The parabolic shaped tip body may form a convex profile of the distal end **748** of the distal tip. The outer surface **746** may be convex from the proximal portion **742** of the tip body **740** to the distal end **748** of the tip body **740.** The tip body **740** may include an opening **749** at its distal end **748** for a guide wire **708** to pass through.

FIG. 28 illustrates an embodiment in which the distal end **750** of the capsule **106** forms the distal tip of the elongate shaft **12.** The distal end **750** of the capsule may include a rounded portion **752** that extends over the distal ends (or anchors **80**) of the implant and may provide a smooth profile for the distal tip of the elongate shaft **12.** The distal tip accordingly may comprise an atraumatic rounded tip. The capsule may include a portion **754** having a planar profile at the leading edge of the capsule **106.** The portion **754** may include an opening or port **756** for the implant to be deployed from.

The capsule **106** may be configured to have a distal end **705** that is elastic, and may conform to the shape of the implant **70** positioned within the capsule **106.** A tie-layer or the like may be added to the capsule **106** to provide elasticity of the capsule **106** against the implant **70.** The capsule **106** may include an ePTFE tip with a low durometer elastic tie layer for example. Upon deployment of the implant **70,** the implant may be advanced distally from the capsule **106** through the port **756,** with the rounded portion **752** of the distal end **750** expanding to accommodate the distal movement of the implant **70.** The port **756** or an opening in the distal end **705** of the capsule **106** may be configured to allow a guide wire **708** to pass through.

In the embodiment shown in FIG. 28, a separate tip body may not be present at the distal tip of the elongate shaft **12,** thus reducing the distal profile of the elongate shaft **12.**

One or more features of the embodiments of distal tips of FIGS. 25A-28 may be utilized solely or with any other embodiment of delivery system or other system, or other methods, disclosed herein.

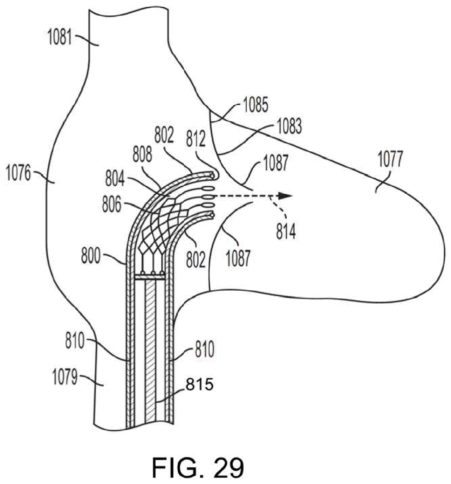

FIG. 29 illustrates an embodiment of an elongate shaft **800** that is configured with a wall **802** surrounding a channel **804** for an implant **806** to be passed through for deployment of the implant **806.** The wall **802** may be configured to have a bend **808** that defines a bend in the channel **804** during the deployment of the implant **806.**

The wall **802** may be configured to be steerable, and a control mechanism may be utilized to steer the wall **802.** For example, pull tethers **810** or other forms of control mechanisms may be utilized to steer the wall **802,** to control the direction of bend of the wall **802,** and particularly to direct an opening or port **812** for the implant **806** to be passed through to a desired orientation.

In one embodiment, the wall **802** may not be steerable, but the wall may have a bend preformed by the wall **802** in a desired orientation.

The channel **804** may be a deployment channel for the implant **806** to be deployed from. The channel **804** may be configured to retain the implant **806** and may comprise an implant retention area. The channel **804** may be configured to retain the implant **806** upon approach and entry of the right atrium **1706** or other portion of the patient's heart or vasculature.

The implant **806** may be configured to be a flexible implant, configured to bend in a direction transverse to an axial dimension **814** of the implant **806.** As such, the implant **806** may be configured to bend within the channel **804** in the direction transverse to the axial dimension **814** of the implant **806** for deployment of the implant **806.** A deployment device, such as a push shaft **815** may be utilized to push the implant **806** from the port **812** for deployment. Other forms of deployment devices, such as expandable balloons may be utilized as desired.

The implant **806** may be an expandable implant, and may be self-expanding, for deployment to the desired portion of the patient's body. The implant **806** may be configured similarly as the implant **70,** yet may be configured to bend in a direction transverse to an axial dimension **814** of the implant **806** when passing through the bent deployment channel. Such a configuration may be provided by the frame of the implant **70** being made thinner to allow for greater flexibility in a transverse direction.

Components of the elongate shaft **12** may be utilized with the elongate shaft **800,** including use of an outer sheath assembly, a mid shaft assembly, a rail assembly, an inner shaft assembly, and a nose cone assembly. Any or all of the assemblies may be utilized to perform or assist with deployment of the implant **806.** The deflection mechanisms disclosed herein may also be utilized. One or more features of the elongate shaft **800** may be utilized solely or with any other embodiment of delivery system or other system, or other methods, disclosed herein.

The use of the wall **802** having a bend **808** that defines a bend in the channel **804** during the deployment of the implant **806,** may provide benefits including a reduced transverse profile of the elongate shaft **800.** For example, as shown in FIGS. 20A-21, the capsule **106** of the elongate shaft **12** may form a relatively large turning radius for the elongate shaft **12** about the bend portion **600.** The use of a bend in the channel **804** may allow for a reduced transverse profile of the elongate shaft **800,** with a relatively smaller turning radius. The port **812** accordingly may be moved proximate the tricuspid valve **1083** for deployment of the flexible implant **806,** with the elongate shaft **800** having a reduced transverse profile. The implant may be passed through a bent deployment channel to deploy the implant (which may be a prosthetic tricuspid valve).

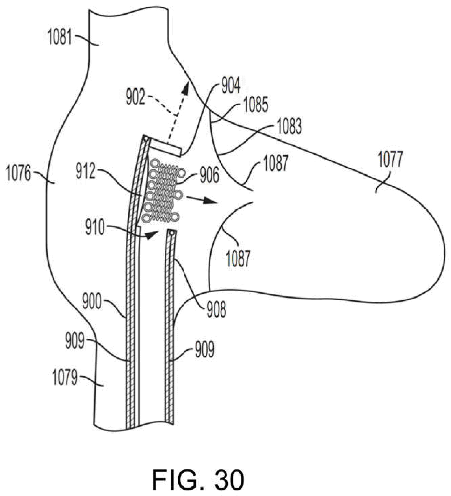

FIG. 30 illustrates an embodiment of an elongate shaft **900** having an axial dimension **902** and having a port **904** for an implant **906** to be deployed from in a direction transverse to the axial dimension **902.** The elongate shaft **900** may include a side wall **908** and the port **904** may be positioned on the side wall **908.**

The side wall **908** may be configured to be steerable, and a control mechanism may be utilized to steer the side wall **908.** For example, pull tethers **909** or other forms of control mechanisms may be utilized to steer the side wall **908,** to direct the port **904** to a desired orientation.

The elongate shaft **900** may include an implant retention area **910** for retaining the implant **906.** The implant **906** may be configured to be deployed in the axial dimension of the implant **906,** exiting through the port **904** in the axial dimension of the implant **906.** The implant **906** may be configured to be compressed in the axial dimension of the implant **906** prior to deployment.

A deployment mechanism may be utilized to deploy the implant **906** from the port **904.** The deployment mechanism may include an inflatable body **912** configured to push the implant **906** out of the port **904** as shown in FIG. 30, or in other embodiments, other forms of deployment mechanisms may be utilized. The implant may be deployed through the port **904** in a direction transverse to the axial dimension of the elongate shaft.

The implant **906** may be an expandable implant, and may be self-expanding, for deployment to the desired portion of the patient's body. The implant **906** may be configured similarly as the implant **70,** yet may be configured to be compressed in the axial dimension of the implant **906.**

Components of the elongate shaft **12** may be utilized with the elongate shaft **900,** including use of an outer sheath assembly, a mid shaft assembly, a rail assembly, an inner shaft assembly, and a nose cone assembly. Any or all of the assemblies may be utilized to perform or assist with deployment of the implant **906.** The deflection mechanisms disclosed herein may also be utilized. One or more features of the elongate shaft **900** may be utilized solely or with any other embodiment of delivery system or other system, or other methods, disclosed herein.

The use of the elongate shaft **900** having an axial dimension **902** and having a port **904** for an implant **906** to be deployed from in a direction transverse to the axial dimension **902,** may provide benefits including a reduced transverse profile of the elongate shaft **900.** For example, as shown in FIGS. 20A-21, the capsule **106** of the elongate shaft **12** may form a relatively large turning radius for the elongate shaft **12** about the bend portion **600.** The use of a port **904** for an implant **906** to be deployed from in a direction transverse to the axial dimension **902** may allow for a reduced transverse profile of the elongate shaft **900.** The port **904** accordingly may be moved proximate the tricuspid valve **1083** for deployment of the implant **906,** with the elongate shaft **900** having a reduced transverse profile.

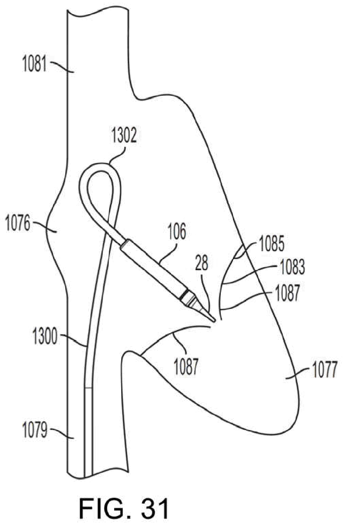

FIG. 31 illustrates an embodiment of an elongate shaft **1300** that is configured to bend more than 180 degrees to form a loop **1302.** The shaft **1300** may be configured similarly as the elongate shaft **12,** yet may be configured to bend more than 180 degrees to form the loop **1302.** Such a feature may be provided by a control mechanism that is configured to cause the bend of greater than 180 degrees, such as a push shaft that extends along the outer diameter of the shaft **1300** and applies a distal force to cause the shaft **1300** to bend more than 180 degrees. Other mechanisms may be utilized as well. The elongate shaft **1300** may thus form a loop **1302,** which may be positioned in a desired location within the patient's body.

For example, as shown in FIG. 31, the loop **1302** may be positioned within the right atrium **1076,** in an embodiment in which the implant **70** is to be deployed to the tricuspid valve **1083.** The loop **1302** may be positioned within the right atrium **1076** to allow for greater clearance of the distal end of the capsule **106** from the wall of the right atrium **1076.** The loop **1302** may be directed in the atrial direction, away from the tricuspid valve **1083.** The elongate shaft is configured to bend at a bend portion of the elongate shaft, with the implant retention area being positioned distal of the bend portion.

The elongate shaft is bent more than 180 degrees to form a loop at least partially within the patient's right atrium. The degree of bend of the elongate shaft **1300** may vary as desired, for example the degree of bend may be more than 200 degrees in one embodiment, may be more than 230 degrees in one embodiment, may be more than 250 degrees in one embodiment, and may be more than 270 degrees in one embodiment. Other degrees of bend may be utilized as desired. One or more features of the elongate shaft **1300** may be utilized solely or with any other embodiment of delivery system or other system, or other methods, disclosed herein.

Components of the elongate shaft **12** may be utilized with the elongate shaft **1300,** including use of an outer sheath assembly, a mid shaft assembly, a rail assembly, an inner shaft assembly, and a nose cone assembly. Any or all of the assemblies may be utilized to perform or assist with deployment of the implant **70** retained by the implant retention area. The deflection mechanisms disclosed herein may also be utilized.

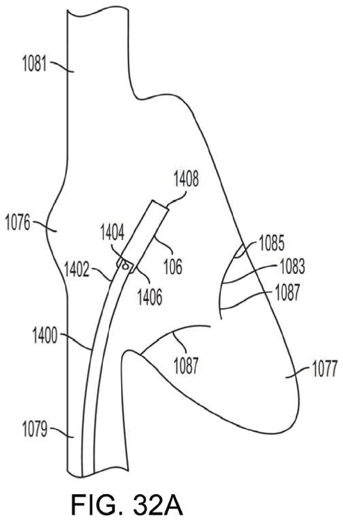

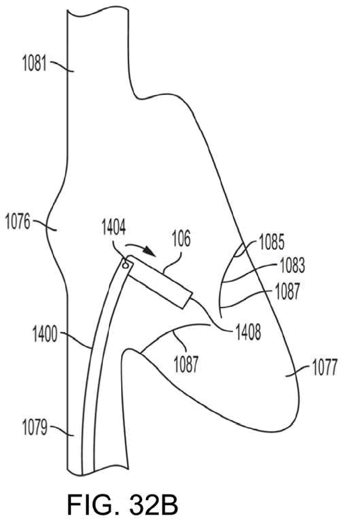

FIGS. 32A-33B illustrate embodiments of elongate shafts including a hinge that couples the capsule to a portion of the elongate shaft. FIG. 32A, for example, illustrates an elongate shaft **1400** having a distal portion **1402** with a hinge **1404.** The hinge **1404** couples to a proximal portion **1406** of a capsule **106.** The capsule **106** is configured to rotate about the hinge **1404** to place the port **1408** of the capsule **106** in the desired orientation relative to the tricuspid valve **1083.** FIG. 32B, for example, illustrates the capsule **106** rotated about the hinge **1404** with the port **1408** oriented towards the tricuspid valve **1083.**

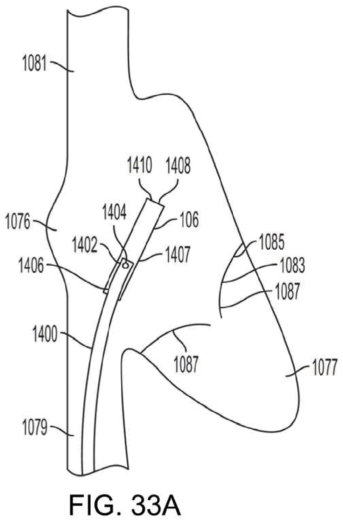

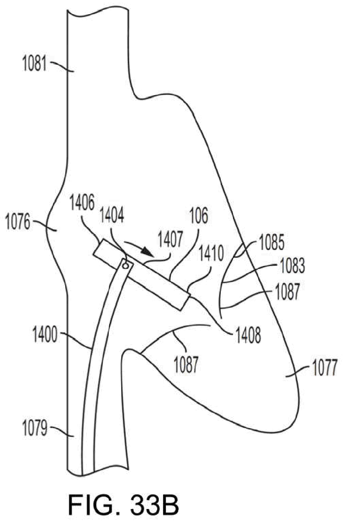

FIG. 33A illustrates an embodiment in which the hinge **1404** at the distal portion **1402** of the elongate shaft **1400** couples to the capsule **106** at a central portion **1407** of the capsule that is positioned between the proximal portion **1406** of the capsule **106** and the distal portion **1410** of the capsule **106.** The capsule accordingly may be pivoted about the hinge **1404** to place the port **1408** of the capsule **106** in the desired orientation relative to the tricuspid valve **1083.** FIG. 33B for example, illustrates the capsule **106** rotated about the hinge **1404** with the port **1408** oriented towards the tricuspid valve **1083.**

The capsules **106** shown in FIGS. 32A-33B may be rotated about the hinge **1404** through use of a control mechanism, which may comprise push or pull shafts or other devices configured to control rotation of the capsule **106.** The implant may be configured to be deployed from the capsule **106** by a deployment mechanism, which may comprise an inflatable body or the like for deploying the implant from the capsule **106.**

The capsules **106** may be configured to rotate about the hinge **1404** to a variety of angles, including between zero degrees and 360 degrees, or more, as desired. The capsules **106** may be rotated to provide the desired orientation of the port **1408** of the capsule **106,** for example in a desired orientation relative to a tricuspid valve **1083** or other delivery location.

The hinge **1404** may comprise a pin extending through an aperture, or may comprise other forms of hinges as desired.

Components of the elongate shaft **12** may be utilized with the elongate shaft **1400,** including use of an outer sheath assembly, a mid shaft assembly, a rail assembly, an inner shaft assembly, and a nose cone assembly. Any or all of the assemblies may be utilized to perform or assist with deployment of the implant **70** retained by the implant retention area. The deflection mechanisms disclosed herein may also be utilized. One or more features of the elongate shaft **1400** may be utilized solely or with any other embodiment of delivery system or other system, or other methods, disclosed herein.

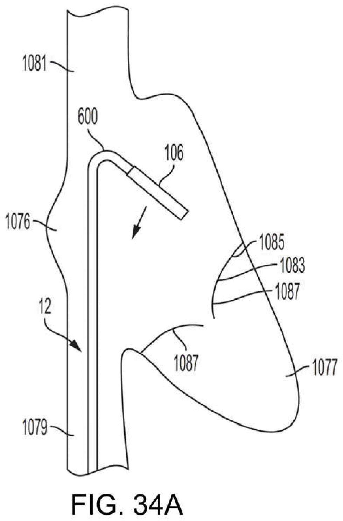

FIGS. 34A-B illustrate a method that may be utilized for deployment of an implant from the elongate shaft **12.** Referring to FIG. 34A, the method may include positioning the capsule **106** of the elongate shaft **12** within the right atrium **1076** of the patient's heart. The bend portion **600** may then be utilized to deflect the capsule **106** to a direction. The elongate shaft **12** may then be translated proximally, for example by retracting the elongate shaft **12** from the patient's heart, to position the capsule **106** in the desired orientation relative to the tricuspid valve **1083.**

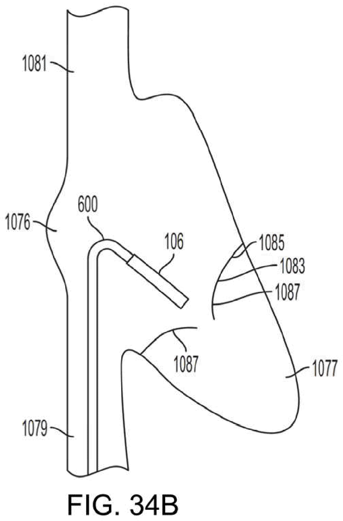

FIG. 34B for example, illustrates the elongate shaft **12** having been retracted in a proximal direction to place the capsule **106** in a desired position relative to the tricuspid valve **1083.** The implant may then be deployed from the capsule **106** for implantation to the tricuspid valve **1083** using methods disclosed herein. The method of FIGS. 34A-B may be utilized solely or with any other embodiment of delivery systems, other systems, or methods disclosed herein.

Various other methods of deploying implants or utilizing the systems and apparatuses disclosed herein may be utilized.

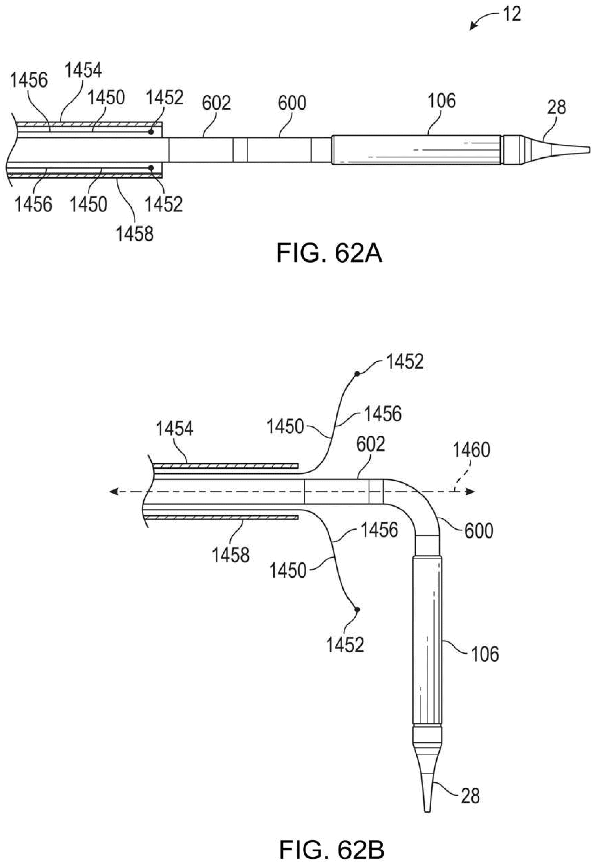

FIGS. 62A-64C illustrate embodiments utilizing one or more support bodies configured to extend radially outward from an outer surface of the elongate shaft **12** and contact an external surface to resist deflection of the elongate shaft transverse to an axis that the elongate shaft **12** extends along. The embodiments may be utilized with any other embodiment disclosed herein.

FIG. 62A, for example, illustrates an embodiment in which one or more support bodies **1450** in the form of arms may be utilized. The support bodies **1450** are shown in an undeployed, unexpanded, or linearized configuration in FIG. 62A in which the support bodies **1450** are collapsed against the outer surface of the elongate shaft **12.** The support bodies **1450** may extend from distal tips **1452** of the support bodies **1450** proximally to the position of the handle **14** or to another position for access exterior of the patient's body.

The support bodies **1450** may be held in the undeployed, unexpanded, or linearized configuration shown in FIG. 62A by a sheath **1454** that extends along the outer surface of the elongate shaft **12** and over the elongate shaft **12** and support bodies **1450.** The sheath **1454,** for example, may be configured similarly as the sheath **51** shown in FIG. 1 or shown as sheath **610** in FIG. 13A as examples. In embodiments, the sheath **1454** may be configured to slide proximally and/or distally along the outer surface of the elongate shaft **12** to uncover or cover the support bodies **1450** as desired. A proximal portion of the sheath **1454** for example may be controlled to move the sheath **1454** proximally or distally.

The support bodies **1450** may extend to the distal tips **1452** of the support bodies **1450.** Each support body **1450** may have an intermediate portion **1456** between the distal tip **1452** and a proximal portion of the support body **1450** that may be configured to extend radially outward from the sheath **1454** and the outer surface of the elongate shaft **12.** Each support body **1450** may be shaped to extend radially outward from the outer surface of the elongate shaft **12** and in embodiments may be biased to extend radially outward from the outer surface of the elongate shaft **12.** For example, the support bodies **1450** may comprise a shape memory material that may be pre-shaped to extend radially outward. The shape memory material may comprise nitinol or another form of shape memory material. In embodiments, the support bodies **1450** may be made from other materials such as stainless steel or another material.

The support bodies **1450** may each be configured to contact an external surface. The external surface may comprise a portion of a patient's vasculature, including a portion of a patient's heart. For example, in embodiments, the support bodies **1450** may be configured to contact atrial walls (which may include an interatrial septum) or other portions of the patient's heart. The support bodies **1450** may be configured to be atraumatic. The intermediate portions **1456** and distal tips **1452,** for example, may each be rounded or smooth to reduce the possibility of damage to a heart wall.

The support bodies **1450** may each be sufficiently stiff to reduce deflection of the elongate shaft 12 in a direction transverse to an axis that the elongate shaft **12** extends along. The support bodies **1450,** however, may be flexible to extend radially outward from an unexpanded configuration (as shown in FIG. 62A) to an expanded configuration (as shown in FIG. 62B). The support bodies **1450** may deflect outward by the sheath **1454** being retracted proximally or the support bodies **1450** being advanced distally relative to the distal end **1458** of the sheath **1454.**

FIG. 62B illustrates the support bodies **1450,** for example, having been advanced distally relative to the sheath **1454.** The support bodies **1450** extend outward radially in the expanded configuration shown in FIG. 62B. The intermediate portions **1456** of the support bodies **1450** protrude from the distal end **1458** of the sheath **1454** outward to the distal tips **1452** of the support bodies **1450.** The support bodies **1450** are positioned to resist deflection of the elongate shaft **12** transverse to an axis **1460** that the elongate shaft **12** extends along.

In embodiments, the support bodies **1450** may be advanced distally and/or the sheath **1454** may be retracted proximally to expand the support bodies **1450.**

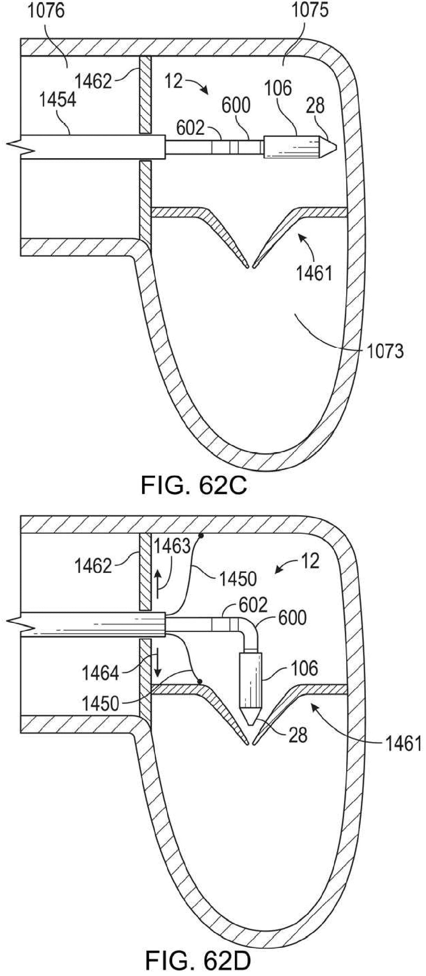

FIGS. 62C-E illustrate an exemplary method of utilizing a delivery apparatus utilizing the support bodies **1450.** FIG. 62C, for example, illustrates the delivery apparatus may be utilized to deliver an implant to a mitral valve **1461.** The elongate shaft **12** of the delivery apparatus for example, may be passed transseptally, through the interatrial septum **1462** between the right atrium **1076** and the left atrium **1075.** A puncture, for example, may be made at the interatrial septum **1462** that allows the elongate shaft **12** to pass through the interatrial septum **1462.** The support bodies **1450** may remain in the unexpanded configuration, covered by the sheath **1454** at this point. The delivery apparatus is positioned within the left atrium **1075.**

FIG. 62D illustrates that the capsule **106** surrounding the implant retention area may be deflected in the ventricular direction, towards the left ventricle **1073** via the bend portion **600** for example. One or more other bend portions, including bend portion **602** may be utilized to align the capsule **106** as desired with respect to the mitral valve **1461.** For example, the bend portion **602** may deflect the capsule in a plane extending transverse to the plane of bend of the bend portion **600** according to methods disclosed herein. Various directions of deflection may be utilized.

With the capsule **106** aligned in position, a depth of the capsule **106** may be increased in the ventricular direction utilizing methods disclosed herein. An increase in depth in the ventricular direction, however, may result in a force applied in an atrial direction **1463** (marked in FIG. 62D) upon the elongate shaft **12.** Further, a reduction of depth towards the atrial direction may result in a force applied in a ventricular direction **1464** (marked in FIG. 62D) upon the elongate shaft **12.** Such forces may result from the movement of the capsule **106** or may result from contact of the capsule **106** with a structure, such as chordae or the valve leaflets of the mitral valve **1461.** The force upon the elongate shaft **12** may provide stress upon the puncture of the interatrial septum **1462.** In embodiments, the stress may increase the size of the puncture of the interatrial septum **1462,** which may be undesirable, and may increase the time for the puncture to seal or may reduce the likelihood of the puncture sealing.

To reduce the deflection of the elongate shaft **12,** and the possible stress to the interatrial septum **1462,** the support bodies **1450** may be expanded radially outward to contact the walls of the patient's heart. FIG. 62D, for example, illustrates the support bodies **1450** expanded, in a configuration as shown in FIG. 62B. The support bodies **1450** move to the expanded state from the unexpanded state. The distal tips **1452** and/or intermediate portions **1456** of the support bodies **1450** may contact the atrial wall to support the elongate shaft **12.** The support bodies **1450** may be positioned to resist a force in the atrial direction **1463** and/or the ventricular direction **1464** as desired. In embodiments, other directions (e.g., transverse to the atrial direction **1463** and/or ventricular direction **1464**) may be utilized as desired. The atrial wall may comprise opposing portions of the atrial wall as shown in FIG. 62D, or may comprise a wall of the interatrial septum in embodiments.

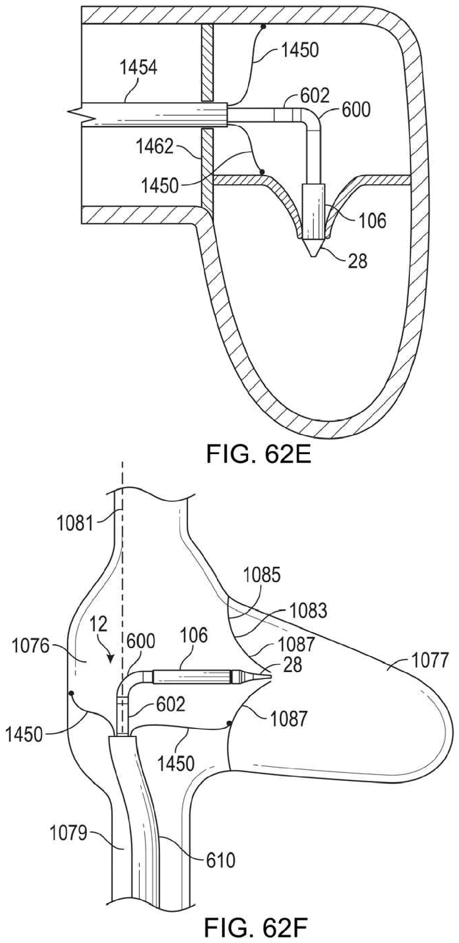

The support bodies **1450** may be positioned proximal of the bend portions **600, 602** or may be in another location as desired. The support bodies **1450** may remain in position during an increase in depth of the capsule **106** or another deployment procedure performed by the elongate shaft **12.** FIG. 62E, for example, illustrates the support bodies **1450** in position as the depth of the capsule **106** is increased in the ventricular direction.

Upon deployment of the implant, the support bodies **1450** may be retracted to the undeployed, unexpanded, or linearized configuration as shown in FIG. 62A and then withdrawn from the patient's body. The support bodies **1450** may be withdrawn along with the elongate shaft **12.**

In embodiments, the sheath **1454** may not be utilized and the support bodies **1450** may be coupled directly to the elongate shaft **12** and may extend radially outward from the elongate shaft **12.** A separate control mechanism may be utilized to control deployment of the support bodies **1450** in such an embodiment.

The support bodies **1450** may beneficially reduce deflection of the elongate shaft **12** and may reduce stress upon the interatrial septum **1462.** As such, the precision of the deflection and depth control of the capsule **106** may be improved due to the reduced possibility of undesired deflection of the elongate shaft **12.** Further, the reduced stress to the interatrial septum **1462** may reduce the possibility of an undesired increase in size of the puncture of the interatrial septum **1462.** Such a feature may reduce the time for the puncture to seal or enhance the likelihood of the puncture sealing. A smaller puncture of the interatrial septum may reduce the likelihood of requiring an occluder to be utilized to seal the puncture following the deployment of the heart valve implant. As such, reduced steps for an implant deployment procedure may result.

The support bodies **1450** may further be utilized in deployment to other locations within the patient's body. FIG. 62F, for example, illustrates an embodiment in which the support bodies **1450** are utilized for deployment to the tricuspid valve. The delivery apparatus is positioned within the right atrium **1076.** The support bodies **1450** may extend radially outward from the elongate shaft **12** and contact the right atrial wall. Other positions of contact of the patient's vasculature may be utilized, such as the inferior vena cava **1079** or the superior vena cava **1081** as desired.

The form of the support bodies may be varied in embodiments.

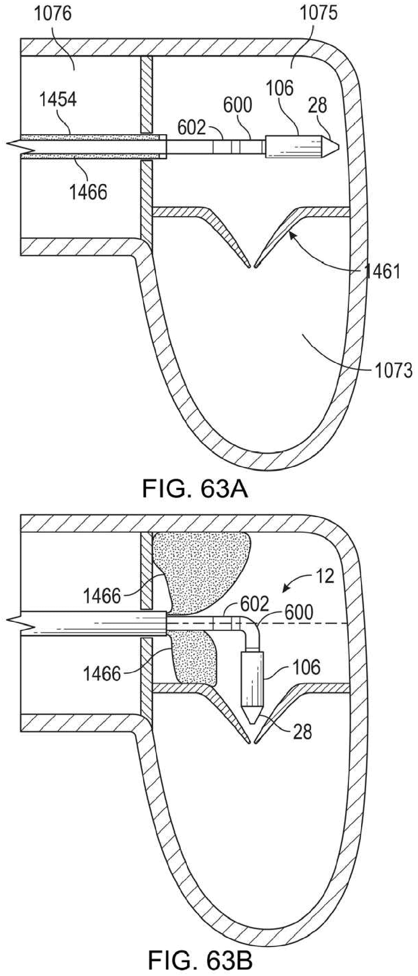

FIGS. 63A-B, for example, illustrate an embodiment in which the support body **1466** comprises an inflatable body. The support body **1466** may be configured to be inflated with fluid or the like to move from an unexpanded, undeployed, or linearized configuration as shown in FIG. 63A to an expanded or deployed configuration as shown in FIG. 63B. FIG. 63A illustrates the support body in an unexpanded or deflated configuration with the sheath **1454** extending over the support body **1466.** The support body **1466** may then be inflated via a fill lumen or the like to the expanded or inflated configuration as shown in FIG. 63B.

Referring to FIG. 63B, the support body **1466** may contact the atrial wall to resist deflection of the elongate shaft **12** in a similar manner as discussed regarding the support body **1450** shown in FIG. 62E or FIG. 62F. In embodiments, one or more inflatable bodies may be utilized. The inflatable bodies may have a rectangular shape, or other shape (e.g., spheroid) or may have another shape such as a disk in embodiments. Further, the configuration of the inflatable bodies may comprise membranes, or may comprise a mesh body, or may have another form in embodiments.

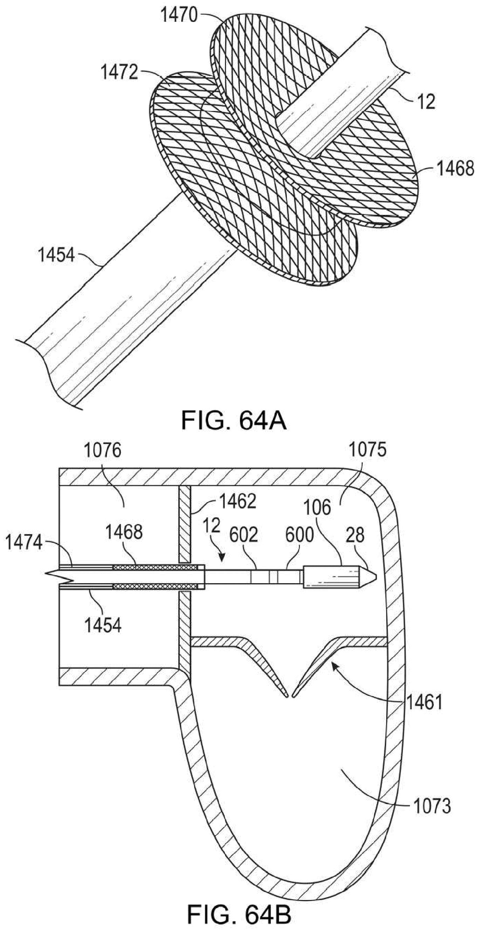

FIGS. 64A-B illustrate an embodiment in which the support body **1468** comprises a mesh configured to extend radially outward from the elongate shaft **12.** The mesh may be configured as a plurality of disks **1470, 1472** that each extend radially outward from the elongate shaft **12.** The disks **1470, 1472** may be configured to be positioned on opposite sides of a puncture of an interatrial septum, although other locations may be utilized in embodiments. In embodiments, a single disk may be utilized or a greater number of disks (e.g., three disks, or four disks) may be utilized as desired.

The support body **1468** may be configured as one or more occluders that are configured to seal a puncture of the interatrial septum. As such, the support body may reduce fluid flow between the atria and support the elongate shaft **12** from deflection in embodiments.

FIG. 64B, for example, illustrates the support body **1468** in an unexpanded, undeployed, or linearized configuration. A sheath **1454** extends over the support body **1468.** A tether **1474** may couple the support body **1468** to the sheath **1454** or the elongate shaft **12.** The support body **1468** may be placed in the desired position relative to the puncture in the interatrial septum and the deployment site.

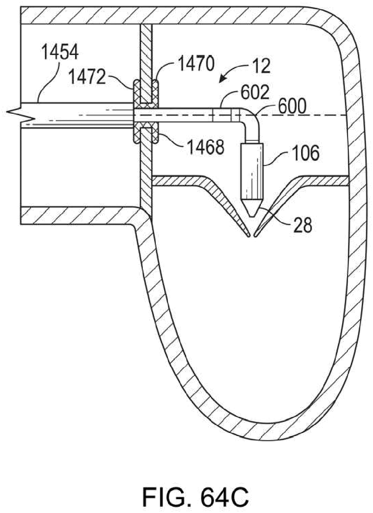

FIG. 64C illustrates the support body **1468** deployed and extending radially outward from the elongate shaft **12.** The surface area of the disks **1470, 1472** against the interatrial septum may reduce the possibility of deflection of the elongate shaft **12.** A disk **1470** may be positioned in the left atrium and another disk **1472** may be positioned in the right atrium. The position and configuration of the disks may be varied in embodiments. The disks **1470, 1472** for example, may be made of a mesh material configured to expand upon deployment. The material may comprise a shape memory material such as nitinol or another form of shape memory material. In embodiments, the disks may comprise inflatable bodies or may comprise other forms of occluders.

The support body **1468** may either be retracted and withdrawn upon deployment of the heart valve implant or may remain in place within the puncture of the interatrial septum. The support body **1468** may remain in place as an occluder following deployment.

In embodiments, various other forms of mesh bodies and disks may be utilized as support bodies herein. The support bodies may be used for deployment to a mitral valve or a tricuspid valve, or another valve as desired. One or more features of the embodiments of support bodies may be utilized with any other embodiment of delivery system, or other system, or methods, disclosed herein.

The implants disclosed herein may be utilized with anchors that are configured to be secured within a portion of a patient's body. The anchors may serve to further secure the implants to the desired implantation location within the patient's heart. The implants may comprise prosthetic heart valves, and particularly may comprise prosthetic heart valves configured for implantation within a patient's tricuspid valve annulus **1085.** The implants may comprise prosthetic tricuspid heart valves and may comprise implants that are disclosed herein.

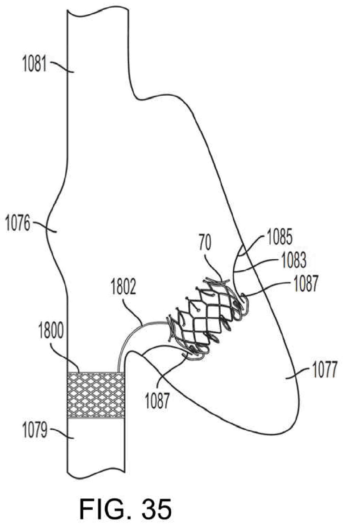

FIGS. 35-38B illustrate embodiments of anchors that may be utilized with implants disclosed herein. FIG. 35, for example, illustrates the implant **70** in position within the tricuspid valve annulus **1085** of the patient's heart. The implant **70** includes prosthetic valve flaps to replace the native valve flaps. An anchor **1800** may be utilized that is configured to be secured within the inferior vena cava **1079** of the patient's heart. The anchor **1800** may comprise a stent that is secured within the inferior vena cava **1079.** A tether **1802** may couple from the anchor **1800** to the implant **70** to secure the implant in position within the tricuspid valve annulus **1085.** The tether **1802** may be rigid, to resist a force in the atrial direction applied to the implant **70.** In one embodiment, the anchor **1800** may be positioned within the superior vena cava **1081** of the patient's heart, alternatively or in combination with an anchor in the inferior vena cava **1079.** An atrial ball anchor may be utilized in certain embodiments.

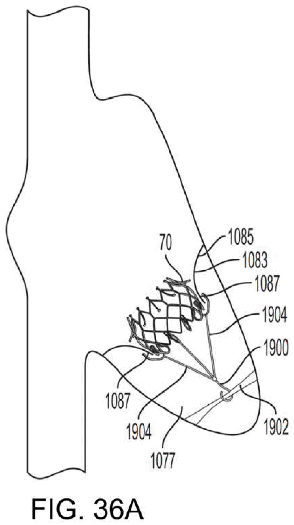

FIG. 36A illustrates an embodiment in which an anchor **1900** is configured to be secured to a moderator band **1902** of the patient's right ventricle **1077.** The anchor **1900** may couple to one or more tethers **1904** that couple to the implant **70.** The tethers **1904** may be configured to resist a force applied to the implant **70** in the atrial direction, to secure the implant **70** within the tricuspid valve annulus **1085.**

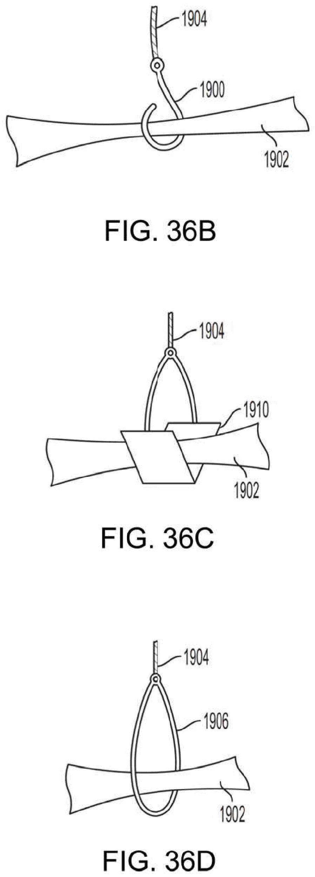

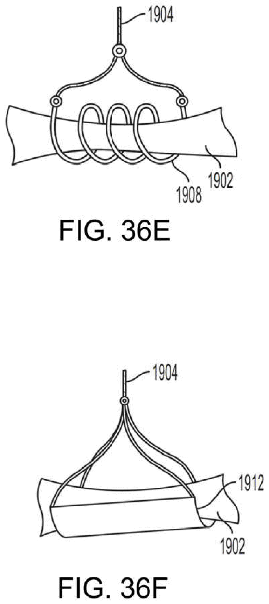

The anchor **1900** may have a variety of forms. The anchor **1900** may comprise a hook as shown in FIGS. 36A and 36B. In one embodiment, the anchor **1900** may have the form of a loop **1906** as shown in FIG. 36D, or multiple loops **1908** (one or more loops) as shown in FIG. 36E. In one embodiment, the anchor **1900** may have the form of a cover **1910** for covering a portion of the moderator band **1902** as shown in FIG. 36C. The cover **1910** may have a V-shaped configuration as shown in FIG. 36C or may have a U-shaped configuration as shown with the cover **1912** in FIG. 36F.

The anchors may be deployed to the moderator band **1902** during the process of implantation of the implant **70,** or in a separate process in which the tether **1904** is coupled between the anchor and the implant **70.** Additional forms of anchors may include barbs or expandable bodies that are expanded over a portion of the moderator band **1902** to secure the anchor to the moderator band **1902.**

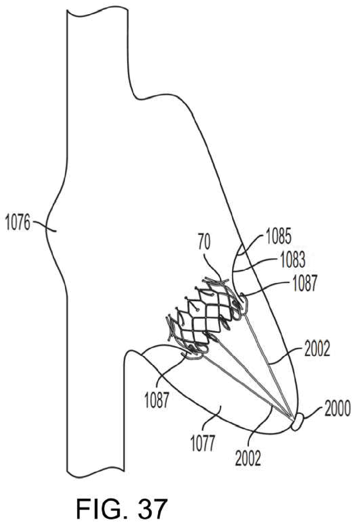

FIG. 37 illustrates an embodiment in which an anchor **2000** may be secured to a wall of the patient's right ventricle **1077.** For example, the anchor **2000** may comprise an expandable body that is passed through a puncture in the wall in a compressed or undeployed state and placed on an exterior surface of the wall of the right ventricle **1077.** The expandable body may be expanded to have a size larger than the size of the puncture to prevent the anchor **2000** from passing back through the puncture. One or more tethers **2002** may couple the anchor **2000** to the implant **70** to secure the implant in position within the tricuspid valve annulus **1085.** The anchor **2000** in other embodiments may have other forms, including barbs or hooks for coupling to the wall of the right ventricle **1077.** The anchor **2000** may be deployed and secured to the wall of the right ventricle **1077** during the process of implantation of the implant **70,** or in a separate process in which the tether **2002** is coupled between the anchor **2000** and the implant **70.**

The embodiments disclosed herein may be deployed within a patient's tricuspid valve annulus, and an anchor may be deployed to a portion within a patient's body. A tether may be provided coupling the prosthetic heart valve to the anchor. The anchor may be coupled to the prosthetic heart valve with the tether.

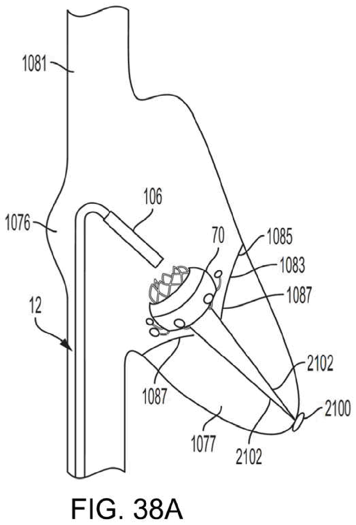

FIG. 38A illustrates an embodiment in which the implant **70** (shown with a cover present on the implant **70**) is deployed within the patient's right atrium **1076** and then moved in the ventricular direction to couple the implant **70** to leaflets **1087** of the tricuspid valve **1083.** The implant **70** may be deployed in the right atrium **1076** utilizing methods disclosed herein, including deploying the implant **70** from the capsule **106** into the right atrium **1076.** The implant **70** may be moved in the ventricular direction in a variety of methods. In one embodiment, as shown in FIG. 38A, the implant **70** may be coupled to one or more tethers **2102.** The tethers **2102** may be configured to be drawn away from the atrium **1076** to move the implant **70** in the ventricular direction to couple to the leaflets **1087.** The tethers **2102** may be coupled to an anchor **2100** positioned on a wall of the right ventricle **1077** and may be configured to be withdrawn through the anchor **2100** to draw the tethers **2102** away from the atrium **1076.** In other embodiments, other methods may be utilized to draw the tethers **2102** away from the atrium **1076.** In one embodiment, a rail structure may be utilized to guide the implant **70** in the ventricular direction to couple to the valve leaflets **1087.**

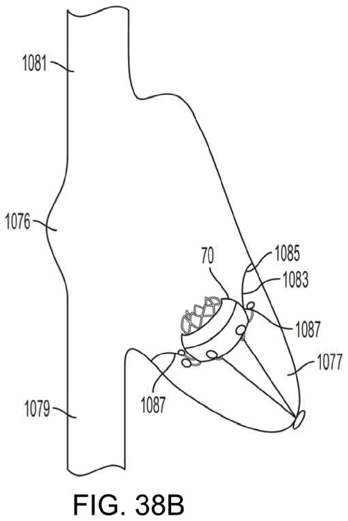

In one embodiment, the elongate shaft **12** may be utilized to push the implant **70** in the ventricular direction to couple to the valve leaflets **1087.** In one embodiment, another push device (such as a device that may be passed through the superior vena cava **1081**) may be utilized to push the implant **70** in the ventricular direction. A combination of methods may be utilized as desired. The implant **70** in position in the tricuspid annulus is shown in FIG. 38B.

In embodiments, the implants may include distal anchors for extending over and anchoring to heart valve flaps or leaflets **1087** as desired. The implant **70** may be anchored to the heart valve flaps or leaflet **1087.** In embodiments, such distal anchors may be excluded.

The systems, apparatuses, and methods disclosed with respect to FIGS. 13A-38B and 62A-64C may be utilized with any embodiment disclosed in this application in combination or in substitution, or any other variation as desired.

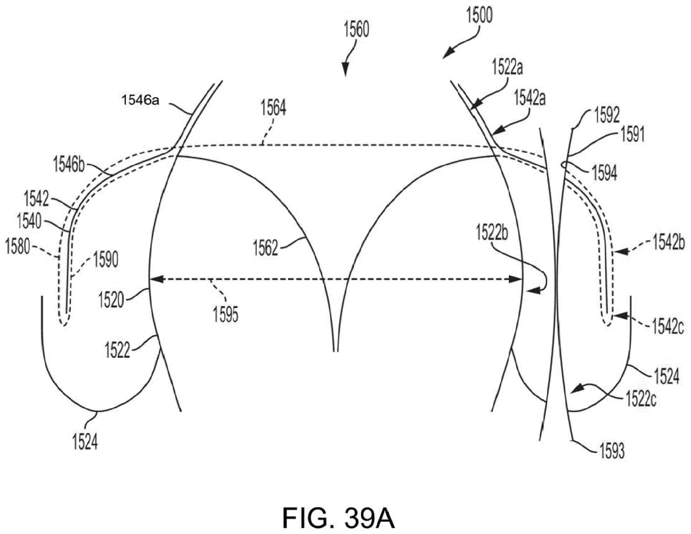

The implant to be utilized according to systems, apparatuses, and methods disclosed herein may include a port that may be configured to receive a diagnostic or therapeutic device. Such a diagnostic or therapeutic device may comprise a pacemaker pacing lead. Embodiments of such an implant are shown in FIGS. 39A-44.

Referring to FIG. 39A, an embodiment of an implant **1500** in an expanded configuration is illustrated. The implant **1500** can include an inner frame **1520,** an outer frame **1540,** a valve body **1560,** and one or more skirts, such as an outer skirt **1580** and an inner skirt **1590.**

With reference first to the inner frame **1520,** the inner frame **1520** can include an inner frame body **1522** and an inner frame anchoring feature **1524.** The inner frame body **1522** can have an upper region **1522a,** an intermediate region **1522b,** and a lower region **1522c.** As shown, the inner frame body **1522** can have a generally bulbous shape such that the diameters of the upper region **1522a** and the lower region **1522c** are less than the diameter of the intermediate region **1522b.**

While the illustrated inner frame body **1522** is bulbous, it is to be understood that the diameters of the upper region **1522a,** the intermediate region **1522b,** and/or the lower region **1522c** can be the same such that the inner frame body **1522** is generally cylindrical along one or more regions. Moreover, all or a portion of the inner frame body **1522** can have a non-circular cross-section such as, but not limited to, a D-shape, an oval or an otherwise ovoid cross-sectional shape.

With reference next to the outer frame **1540** illustrated in FIG. 39A, the outer frame **1540** can be attached to the inner frame **1520** using any suitable fastener and/or other technique. Although the outer frame **1540** is illustrated as a separate component from the inner frame **1520,** it is to be understood that the frames **1520, 1540** can be unitarily or monolithically formed.

As shown in the illustrated embodiment, the outer frame **1540** can include an outer frame body **1542.** The outer frame body **1542** can have an upper region **1542a,** an intermediate region **1542b,** and a lower region **1542c.**

The upper region **1542a** of the outer frame body **1542** can include a first section **1546a** and a second section **1546b.** The first section **1546a** can be sized and/or shaped to generally match the size and/or shape of the inner frame **1520.**

The intermediate region **1542b** of the outer frame body **1542** can extend generally downwardly from the outwardly-extending section **1546b** of the upper region **1542a.**

While the intermediate and lower regions **1542b, 1542c** have been described as cylindrical, it is to be understood that the diameters of the upper end, the lower end, and/or the portion therebetween can be different. For example, all or a portion of the outer frame body **1542** can be have a non-circular cross-section such as, but not limited to, a D-shape, an oval or an otherwise ovoid cross-sectional shape.

The outer frame **1540,** such as the outer frame body **1542** can be used to attach or secure the implant **1500** to a native valve, such as a native tricuspid valve. For example, the intermediate region **1542b** of the outer frame body **1542** and/or the anchoring feature **1524** can be positioned to contact or engage a native valve annulus, tissue beyond the native valve annulus, native leaflets, and/or other tissue at or around the implantation location during one or more phases of the cardiac cycle, such as systole and/or diastole.

With continued reference to the implant **1500** illustrated in FIG. 39A, the valve body **1560** is attached to the inner frame **1520** within an interior of the inner frame body **1522.** The valve body **1560** functions as a one-way valve to allow blood flow in a first direction through the valve body **1560** and inhibit blood flow in a second direction through the valve body **1560.**

The valve body **1560** can include a plurality of valve leaflets **1562,** for example three leaflets **1562,** which are joined at commissures. The valve body **1560** can include one or more intermediate components **1564.** The intermediate components **1564** can be positioned between a portion of, or the entirety of, the leaflets **1562** and the inner frame **1520** such that at least a portion of the leaflets **1562** are coupled to the frame **1520** via the intermediate component **1564.**

With reference next to the outer skirt **1580** illustrated in FIG. 39A, a cover in the form of the outer skirt **1580** can be attached to the inner frame **1520** and/or outer frame **1540.** As shown, the outer skirt **1580** can be positioned around and secured to a portion of, or the entirety of, the exterior of the outer frame **1540.**

With reference next to the inner skirt **1590** illustrated in FIG. 39A, a cover in the form of the inner skirt **1590** can be attached to the valve body **1560** and the outer skirt **1580.**

Although the implant **1500** has been described as including an inner frame **1520,** an outer frame **1540,** a valve body **1560,** and skirts **1580, 1590,** it is to be understood that the implant **1500** need not include all components.

The implant **1500** may include a port **1591** that is coupled to the valve body **1560** and is configured to receive a diagnostic or therapeutic device, which may comprise a pacemaker lead. The port **1591** as shown in FIG. 39A may include a tube that extends along the height of the implant **1500** to guide the pacemaker lead through the implant **1500.** The tube of the port **1591** may include an entry opening **1592** and an exit opening **1593** with a central lumen **1594** extending between the entry opening **1592** and the exit opening **1593.** The tube may have a cylindrical shape or may have a shape with opposed inverted funnels as shown in FIG. 39A. The tube may be configured to have a pacemaker lead passed from the entry opening **1592,** along the central lumen **1594,** and to the exit opening **1593.**

The port **1591** may be positioned on the outer frame **1540** of the valve body **1560.** The valve body **1560** may form a valve annulus **1595** that the valve leaflets **1562** are positioned in, and the port **1591** may be positioned outside of the valve annulus **1595.** As such, the pacemaker lead passing through the port **1591** may avoid interference with the movement of the valve leaflets **1562.**

The port **1591** may be configured to pass through the outer skirt **1580** of the implant **1500** and may pass within openings of the outer frame **1540** and between struts of the outer frame **1540.** Other locations of the port **1591** may be utilized in other embodiments.

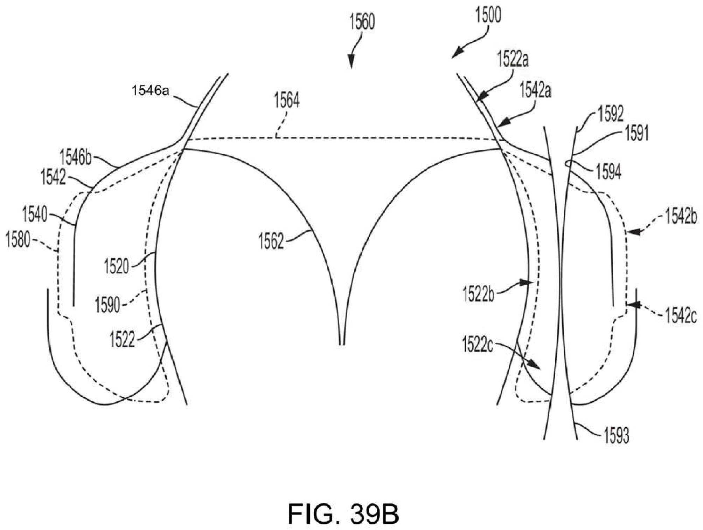

FIG. 39B illustrates an alternate embodiment of FIG. 39A with modifications to the design of the coverings, or skirts (or cloth) **1580/1590.** As shown, the skirts **1580/1590** can contact both the inner frame **1520** and outer frame **1540.** The skirts **1580/1590** can start on the inside of the outer **1540,** transition to the outside of the outer frame **1540,** then attach to the bottom of the outside of the inner frame **1520,** then proceed up along the outside of the inner frame **1520.** By closing the skirts **1580/1590,** this could avoid/reduce clot formation/embolization.

The port **1591** accordingly may pass through both the upper portion of the skirt **1580** and the lower portion of the skirt **1580,** as shown in FIG. 39B.

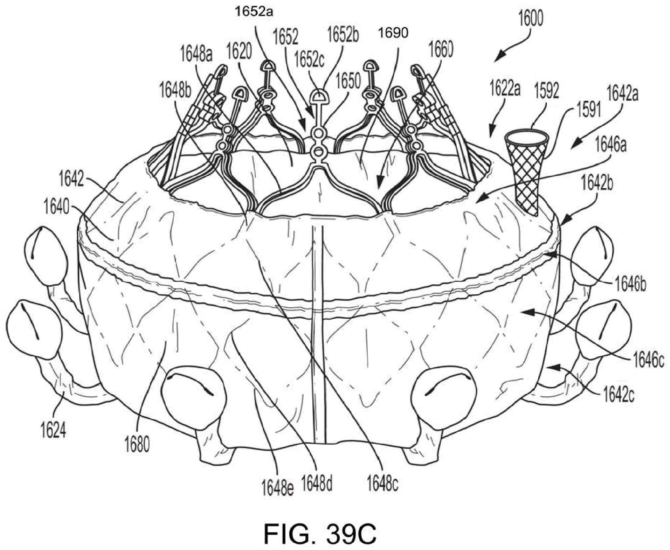

FIGS. 39C-D illustrate a perspective view of an embodiment of an implant **1600** in an expanded configuration. The implant **1600** may be similar in construction to the implant **1500** described above. The implant **1600** can include an inner frame **1620,** an outer frame **1640,** a valve body **1660,** and one or more skirts, such as an outer skirt **1680** and an inner skirt **1690.** A perspective view of the port **1591** is shown extending from an upper surface of the implant **1600.**

With reference first to the outer frame **1640** illustrated in FIGS. 39C-D, the outer frame **1640** can be attached to the inner frame **1620** using any known fasteners and/or techniques. Although the outer frame **1640** is illustrated as a separate component from the inner frame **1620,** it is to be understood that the frames **1620, 1640** can be unitarily or monolithically formed.

As shown in the illustrated embodiment, the outer frame **1640** can include an outer frame body **1642.** The outer frame body **1642** can have an upper region **1642a,** an intermediate region **1642b,** and a lower region **1642c.** At least a portion of the upper region **1642a** of the outer frame body **1642** can be sized and/or shaped to generally match the size and/or shape of an upper region **1622a** of the inner frame **1620.**

When in an expanded configuration such as in a fully expanded configuration, the outer frame body **1642** can have a shape similar to that of outer frame body **1542** described above in connection with FIG. 39A. However, it is to be understood that all or a portion of the outer frame body **1642** can be have a non-circular cross-section such as, but not limited to, a D-shape, an oval or an otherwise ovoid cross-sectional shape.

With continued reference to the implant **1600** illustrated in FIG. 39C, the outer frame body **1642** can include a plurality of struts with at least some of the struts forming cells **1646a-c.**

The upper row of cells **1646a** can have an irregular octagonal shape such as a "heart" shape. This additional space can beneficially allow the outer frame **1640** to retain a smaller profile when crimped. The cell **1646a** can be formed via a combination of struts. As shown in the illustrated embodiment, the upper portion of cells **1646a** can be formed from a set of circumferentially-expansible struts **1648a** having a zig-zag or undulating shape forming a repeating "V" shape.

The middle portion of cells **1646a** can be formed from a set of struts **1648b** extending downwardly from bottom ends of each of the "V" shapes.

The lower portion of cells **1646a** can be formed from a set of circumferentially-expansible struts **1648c** having a zig-zag or undulating shape forming a repeating "V" shape.

The middle and/or lower rows of cells **1646b-c** can have a different shape from the cells **1646a** of the first row. The middle row of cells **1646b** and the lower row of cells **1646c** can have a diamond or generally diamond shape. The diamond or generally diamond shape can be formed via a combination of struts.

The upper portion of cells **1646a** can be formed from the set of circumferentially-expansible struts **1648c** such that cells **1646b** share struts with cells **1646a.** The lower portion of cells **1646b** can be formed from a set of circumferentially-expansible struts **1648d.** As shown in the illustrated embodiment, one or more of the circumferentially-expansible struts **1648d** can extend generally in a downward direction generally parallel to the longitudinal axis of the outer frame **1640.**

The upper portion of cells **1646c** can be formed from the set of circumferentially-expansible struts **1648d** such that cells **1646c** share struts with cells **1646b.** The lower portion of cells **1646c** can be formed from a set of circumferentially-expansible struts **1648e.** Circumferentially-expansible struts **1648e** can extend generally in a downward direction.

As shown in the illustrated embodiment, the implant **1600** can include a set of eyelets **1650.** The upper set of eyelets **1650** can extend from an upper region **1642a** of the outer frame body **1642.** As shown, the upper set of eyelets **1650** can extend from an upper portion of cells **1646a,** such as the upper apices of cells **1646a.** The upper set of eyelets **1650** can be used to attach the outer frame **1640** to the inner frame **1620.**

The outer frame **1640** can include a set of locking tabs **1652** extending from at or proximate an upper end of the upper region **1642a.** As shown, the locking tabs **1652** can extend upwardly from the set of eyelets **1650.** The outer frame **1640** can include twelve locking tabs **1652,** however, it is to be understood that a greater number or lesser number of locking tabs can be used. The locking tabs **1652** can include a longitudinally-extending strut **1652a.** At an upper end of the strut, the locking tab **1652** can include an enlarged head **1652b.** As shown, the enlarged head **1652b** can have a semi-circular or semi-elliptical shape forming a "mushroom" shape with the longitudinal strut **1652a.** The locking tab **1652** can include an eyelet **1652c** which can be positioned through the enlarged head **1652b.** It is to be understood that the locking tab **1652** can include an eyelet at other locations or can include more than a single eyelet.

The locking tab **1652** can be advantageously used with multiple types of delivery systems. For example, the shape of the struts and the enlarged head **1652b** can be used to secure the outer frame **1640** to a "slot" based delivery system, such as the inner retention member **40** described above. The eyelets **1652c** and/or eyelets **1650** can be used to secure the outer frame **1640** to a "tether" based delivery system such as those which utilize sutures, wires, or fingers to control delivery of the outer frame **1640** and the implant **1600.** This can advantageously facilitate recapture and repositioning of the outer frame **1640** and the implant **1600** in situ.

The outer frame **1640,** such as the outer frame body **1642** can be used to attach or secure the implant **1600** to a native valve, such as a native tricuspid valve. For example, the intermediate region **1642b** of the outer frame body **1642** and/or the anchoring feature **1624** can be positioned to contact or engage a native valve annulus, tissue beyond the native valve annulus, native leaflets, and/or other tissue at or around the implantation location during one or more phases of the cardiac cycle, such as systole and/or diastole. As another example, the outer frame body **1642** can be sized and positioned relative to the inner frame anchoring feature **1624** such that tissue of the body cavity positioned between the outer frame body **1642** and the inner frame anchoring feature **1624,** such as native valve leaflets and/or a native valve annulus, can be engaged or pinched to further secure the implant **1600** to the tissue. As shown, the inner frame anchoring feature **1624** includes nine anchors; however, it is to be understood that a fewer or greater number of anchors can be used. In some embodiments, the number of individual anchors can be chosen as a multiple of the number of commissures for the valve body **1660.**

The valve body **1660** can include a plurality of valve leaflets **1662,** for example three leaflets **1662,** which are joined at commissures. The valve body **1660** can include one or more intermediate components **1664.**

With reference next to the outer skirt **1680** illustrated in FIG. 39C, the covering or outer skirt **1680** can be attached to the inner frame **1620** and/or outer frame **1640.** The outer skirt **1680** can be positioned around and secured to a portion of, or the entirety of, the exterior of the outer frame **1640.** The inner skirt **1690** can be attached to the valve body **1660** and the outer skirt **1680.**

Although the implant **1600** has been described as including an inner frame **1620,** an outer frame **1640,** a valve body **1660,** and skirts **1680, 1690,** it is to be understood that the implant **1600** need not include all components. For example, in some embodiments, the implant **1600** can include the inner frame **1620,** the outer frame **1640,** and the valve body **1660** while omitting the skirt **1680.** Moreover, although the components of the implant **1600** have been described and illustrated as separate components, it is to be understood that one or more components of the implant **1600** can be integrally or monolithically formed. For example, in some embodiments, the inner frame **1620** and the outer frame **1640** can be integrally or monolithically formed as a single component.

Referring to FIG. 39C, the upper end of the port **1591** is shown extending from an upper surface of the skirt **1680** and the outer frame **1640.** The opening **1592** may be raised above the upper surface of the skirt **1680** and the outer frame **1640** to allow a user to pass a diagnostic or therapeutic device, which may comprise a pacemaker pacing lead through the opening **1592.**

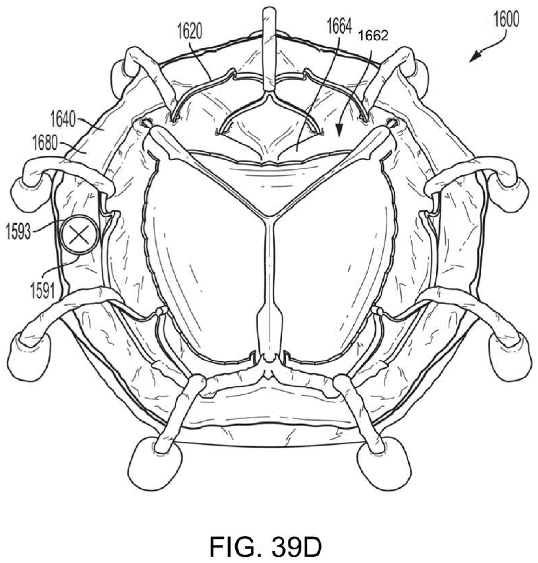

FIG. 39D shows a bottom view of the implant **1600,** illustrating the position of the exit opening 1593.

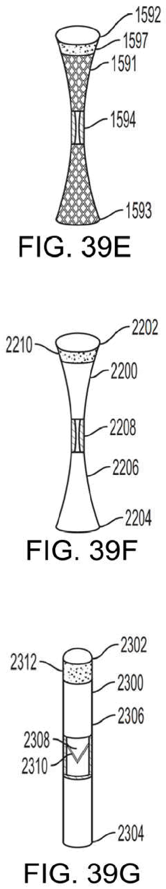

Referring to FIGS. 39E-G, the port may have a variety of forms. The ports are shown isolated from the implants. Referring to FIG. 39E, the body of the tube of the port **1591** may be made of a woven or textile material that exhibits bias to contract the central lumen **1594.** The contraction of the central lumen **1594** may form a seal against the diagnostic or therapeutic device, which may comprise a pacemaker pacing lead, as it is inserted through the port **1591,** to prevent reverse blood flow through the port **1591.** The woven material may be made of wires such as nitinol wires that are interwoven and heat set. Other materials may be utilized as desired.

The port **1591** may include a location marker such as a radiopaque marker **1597** that identifies the location of the port **1591** and particularly the opening **1592** of the port **1591** under imaging.

FIG. 39F illustrates an embodiment of the port **2200** in which the port **2200** includes a tube having an entry opening **2202,** an exit opening **2204** and a body **2206** positioned between the entry opening **2202** and the exit opening **2204.** The body **2206** may surround a central lumen **2208** that the diagnostic or therapeutic device, which may comprise a pacemaker pacing lead, passes through. The body **2260** may be made of a polymer such as an elastomeric material such as a fluoroelastomer or a silicone, configured to be biased towards the central lumen **2208.** The bias of the body **2260** towards the central lumen **2208** may form a seal against the pacemaker pacing lead as it is inserted through the port **2200,** to prevent reverse blood flow through the port **2200.** Other materials may be utilized as desired.

The port **2200** may include a location marker such as a radiopaque marker **2210** that identifies the location of the port **2200** and particularly the opening **2202** of the port **2200** under imaging.

FIG. 39G illustrates an embodiment of the port **2300** in which the port **2300** includes a tube having an entry opening **2302,** an exit opening **2304** and a body **2306** positioned between the entry opening **2302** and the exit opening **2304.** The body **2306** may surround a central lumen **2308** that the pacemaker pacing lead passes through. The central lumen **2308** may include a valve **2310** positioned therein, that may form a seal against the diagnostic or therapeutic device, which may comprise a pacemaker pacing lead, as it is inserted through the port **2300,** to prevent reverse blood flow through the port **2300.** The valve **2310** may comprise a duckbill valve or other form of valve.

The port **2300** may include a location marker such as a radiopaque marker **2312** that identifies the location of the port **2300** and particularly the opening **2302** of the port **2300** under imaging. The body **2306** may be made of a polymer, an elastomer, silicone, or a textile material. Other materials may be utilized as desired.

Any embodiment of port disclosed herein may be impregnated on either an outside surface or inside surface, or both, of a drug coating for release into the patient's body. Further, a coating may be provided on either an outside surface or inside surface, or both, to provide the surface with hydrophilic or hydrophobic properties, or antithrombic properties.

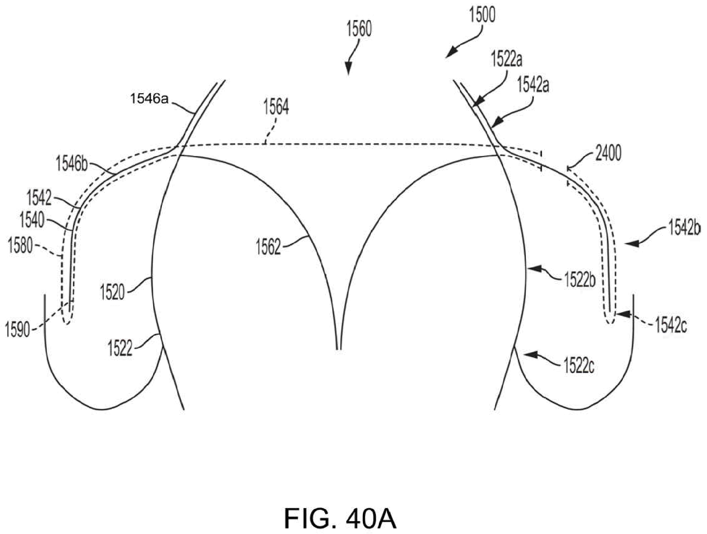

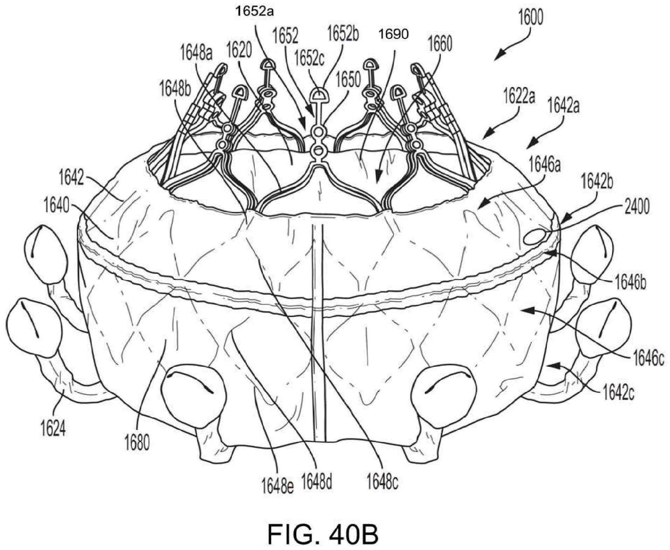

FIGS. 40A-B illustrate an embodiment of a port **2400** in which the port **2400** comprises an opening in the valve body **1560** of the implant **1500.** The port **2400** may extend through a cover or skirt of the implant **1500,** which may include an outer skirt **1580** as shown in FIG. 40A. The opening may be made of a material that is biased towards the center of the opening, such that as a pacemaker pacing lead is passed through the opening, the material may form a seal against the pacemaker pacing lead as it is inserted through the port **2400,** to prevent reverse blood flow through the port **2400.** For example, an elastic material may form a seal against the pacing lead. As shown in FIG. 40B, the opening may be positioned between struts of the outer frame to allow the lead to have a path to pass through. The opening may be surrounded by a location marker such as a radiopaque marker that identifies the location of the port **2400** and particularly the opening of the port **2400** under imaging.

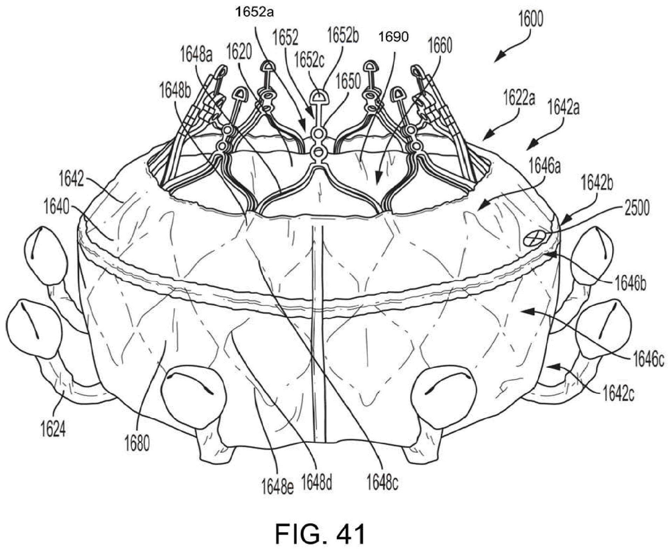

FIG. 41 illustrates an embodiment in which the port **2500** comprises a tearable portion of the valve body **1660.** The tearable portion may be a tearable portion of the outer skirt **1680** as shown in FIG. 41. The tearable portion may pass a diagnostic or therapeutic device therethrough. The tearable portion may be configured to be penetrated by a puncture device or the pacemaker pacing lead to pass through the tearable portion. The material surrounding the resulting opening in the skirt **1680** may be configured to be biased towards the opening, to prevent reverse blood flow through the port **2500.** The tearable portion may form flaps that press against the pacing lead, to seal against the pacing lead as a reverse flow is applied to the flaps against the lead. An elastomer material may be used to form a seal against the lead. As shown in FIG. 41, the opening may be positioned between struts of the outer frame, to allow a path for the pacing lead. The opening may be surrounded by a location marker such as a radiopaque marker that identifies the location of the port **2500** and particularly the opening of the port **2500** under imaging.

In one embodiment, a port may be positioned outside of the outer valve body, for positioning between the outer valve body and the annulus of the heart valve. The port may comprise a loop of material or the like that the diagnostic or therapeutic device is passed through.

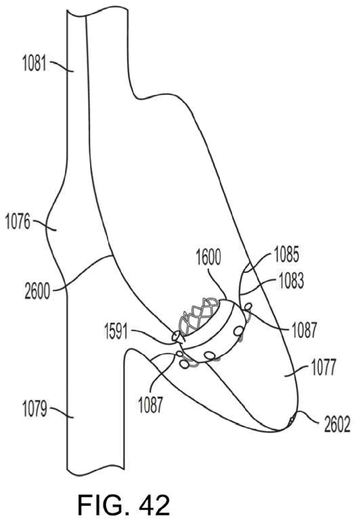

FIG. 42 illustrates use of the port **1591** as shown in FIG. 39C. The diagnostic or therapeutic device, which may comprise a pacemaker pacing lead **2600** may be passed through the port **1591.** The tip **2602** of the pacemaker pacing lead **2600** may be positioned within the right ventricle.

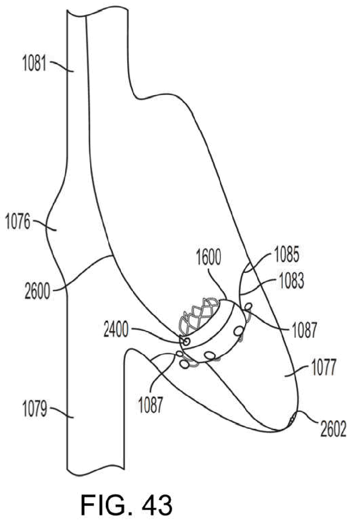

FIG. 43 illustrates use of the port **2400** as shown in FIG. 40B. The diagnostic or therapeutic device, which may comprise a pacemaker pacing lead **2600** may be passed through the port **2400.** The tip **2602** of the pacemaker pacing lead **2600** may be positioned within the right ventricle.

A method may include passing a diagnostic or therapeutic device through a port positioned on a prosthetic heart valve body. The prosthetic heart valve body may form a prosthetic heart valve annulus. The port may include a tube for the diagnostic or therapeutic device to be passed through.

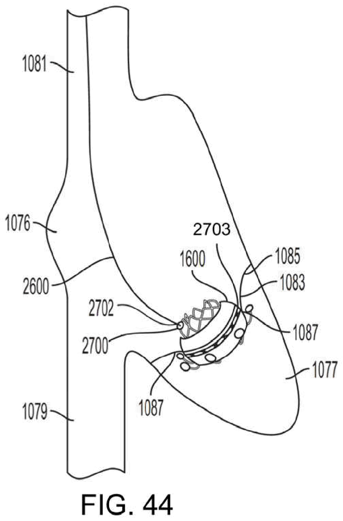

FIG. 44 illustrates an embodiment in which the port **2700** is configured to couple to the pacemaker pacing lead **2600** to form an electrical connection between the implant **1600** and the pacemaker pacing lead **2600.** The tip **2702** of the pacemaker pacing lead **2600** may be configured to couple to the port **2700** and provide electrical energy to the implant **1600.** The frame of the implant **1600** may provide electrical energy to pace functioning of the patient's heart. The pacemaker pacing lead **2600** may couple directly to the implant frame, as shown in FIG. 44. The implant frame may be made of nitinol or another electrically conductive material. The implant may include one or more electrical terminals **2703** that are in contact with the patient's body and may provide the electrical energy to the patient's body to pace functioning of the patient's heart. The terminals, for example, may be on the outside of the body of the implant, or may be positioned on the valve leaflet anchors or other part of the implant in contact with a portion of the patient's heart.

In embodiments disclosed herein, the prosthetic valve body may be deployed to the patient's heart valve annulus utilizing methods disclosed herein. The valve body may be expanded within the heart valve annulus and may be anchored to the heart valve flaps or leaflets of the heart valve. The valve body may be contacted to the patient's heart valve.

A method may include coupling a pacemaker pacing lead to a prosthetic heart valve body positioned within a patient's heart valve annulus to provide electrical energy through the pacemaker pacing lead and through the prosthetic heart valve body to pace functioning of the patient's heart. The method may include providing electrical energy through the frame. The prosthetic valve body may include one or more electrical terminals in contact with a portion of the patient's heart. Electrical energy may be provided through the pacemaker pacing lead and through the prosthetic heart valve body to pace functioning of the patient's heart.

Any embodiments of ports for a pacemaker pacing lead may be utilized acutely, if conduction disturbance is detected at the time of implant, or chronically, if conduction issues develop over time.

The diagnostic or therapeutic device may not only comprise a pacemaker pacing lead, but may comprise other forms of devices, such as catheters or other medical devices to be passed through the implant.

The embodiments of implants disclosed herein may be utilized solely, or across embodiments as desired. Such embodiments may be utilized in a tricuspid or mitral valve, or other valve as desired. Features of the embodiments of implants may be combined across embodiments as desired.

Any and all of the embodiments disclosed herein may be utilized with motorized implant delivery systems. Further, in any and all embodiments, the delivery system may utilize a processor for control of at least one motor for actuating a delivery apparatus. Further, in any and all embodiments, the delivery system may include sensors as disclosed herein. The delivery system may include sensors configured sense one or more of a condition of the patient's body or a condition of the delivery apparatus. The processor may process the signals provided by the sensors, which may comprise feedback signals to the processor.

Features of such systems are disclosed in U.S. Provisional Patent Application No. 62/837,641, filed April 23, 2019, the entire contents of which are incorporated herein by reference. Features of such systems are also disclosed in PCT Application No. PCT/US2020/029138, filed April 21, 2020, the entire contents of which (along with the U.S. National Stage application for PCT Application No. PCT/US2020/029138) are incorporated herein by reference.

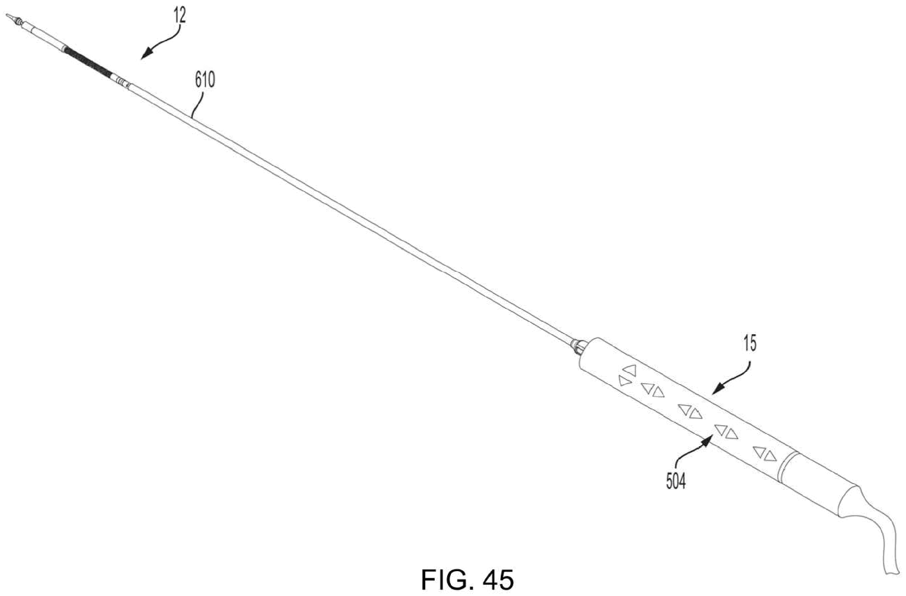

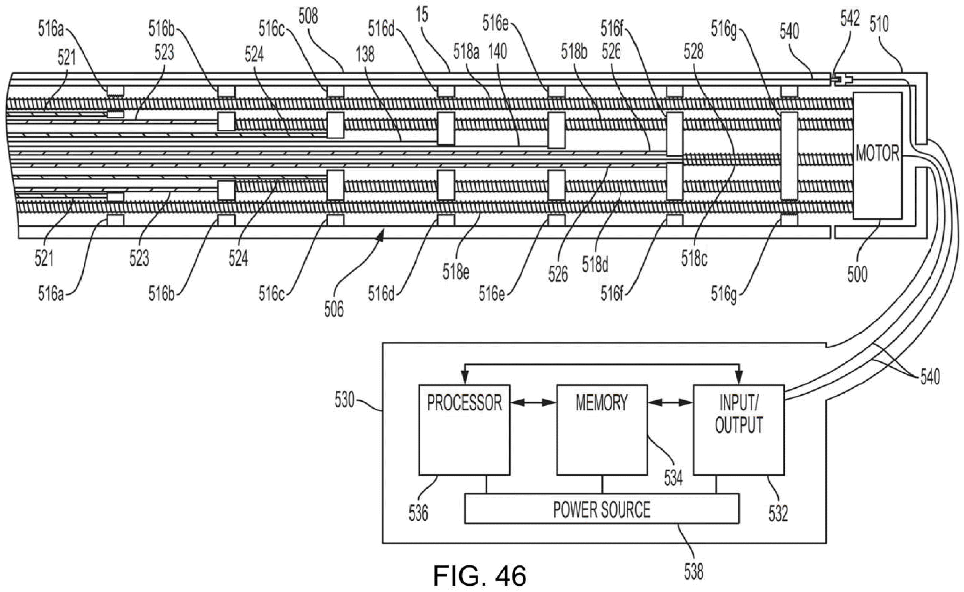

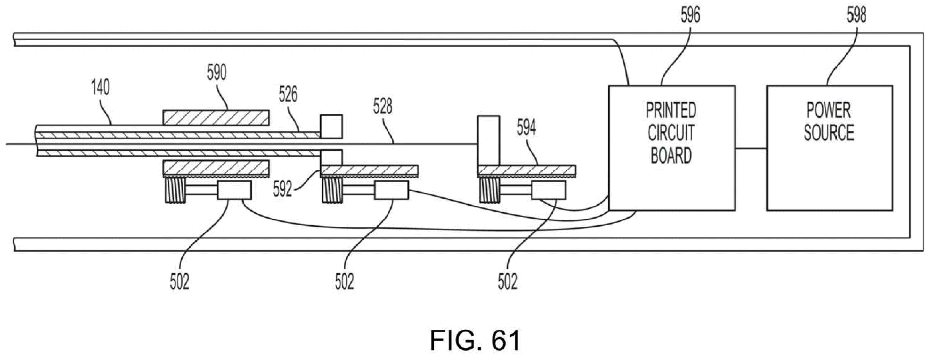

Referring to FIG. 45, the elongate shaft **12** and housing in the form of a handle **15** may form a delivery apparatus that is configured to deliver the implant **70** to a location within a patient's body. The delivery apparatus may also include the deflection mechanisms disclosed herein, which may include use of the sheath **610** as shown in FIG. 45. The delivery system **10** may include at least one motor that is configured to actuate at least a portion of the delivery apparatus. The actuation of at least a portion of the delivery apparatus may include deflection of a portion of the delivery apparatus (including the elongate shaft) or other movement of the delivery apparatus and may include actuation of an operation of the delivery apparatus. The operation may include deployment (whether full or partial) of the implant **70** to the body location, among other operations of the delivery apparatus. The motor may comprise a motor **500** as shown in FIG. 46 or may comprise a plurality of motors **502** shown in FIG. 61 (i.e., at least one motor), among other forms of motors.

As shown in FIG. 45, the housing in the form of the handle **15** may be positioned at the proximal end **11** of the elongate shaft **12.** The proximal end **11** of the elongate shaft **12** may be coupled to the handle **15.** The handle **15** may include a control device **504** configured to control the at least one motor. The control device **504** as shown in FIG. 45 may include a plurality of buttons; however, in other embodiments other forms of control devices may be utilized. The control device **504** may be positioned on the handle **15** as shown in FIG. 45 or may be located remotely.

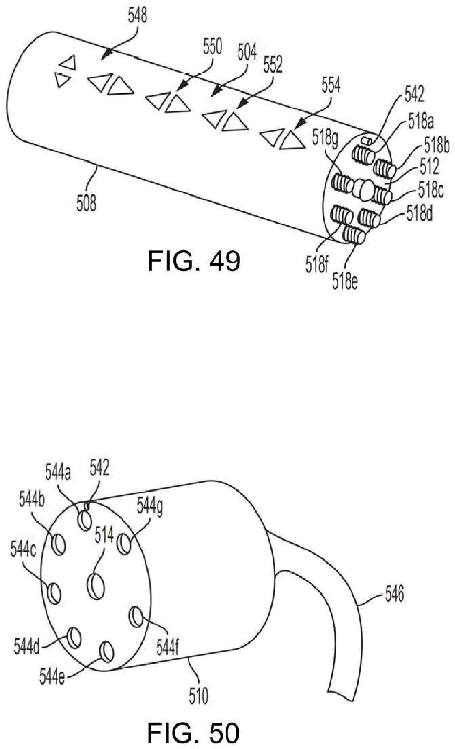

FIG. 46 illustrates a cross section of the handle **15** including the motor **500** and an actuation mechanism **506** that may be utilized to actuate at least a portion of the delivery apparatus. In various embodiments, the motor and actuation mechanism may be used to actuate pull wires during advancement through the vasculature. The motor and actuation mechanism may be used to actuate to actuate shafts/sheaths for deploying and releasing the implant at the treatment site. The body of the handle **15** may include multiple parts, including a distal portion **508** and a proximal portion **510.** The distal portion **508** as shown in FIG. 46 may be configured to retain the actuation mechanism **506** and the proximal portion **510** may be configured to retain the motor **500.** In other embodiments, other components may be positioned in respective distal **508** and proximal portions **510,** and in certain embodiments the handle **15** may include a single body. In the embodiment shown in FIG. 46, the distal portion **508** and proximal portion **510** may be configured to couple together via a coupler **512, 514** (marked in FIGS. 49 and 50), and may be separable from each other in certain embodiments.

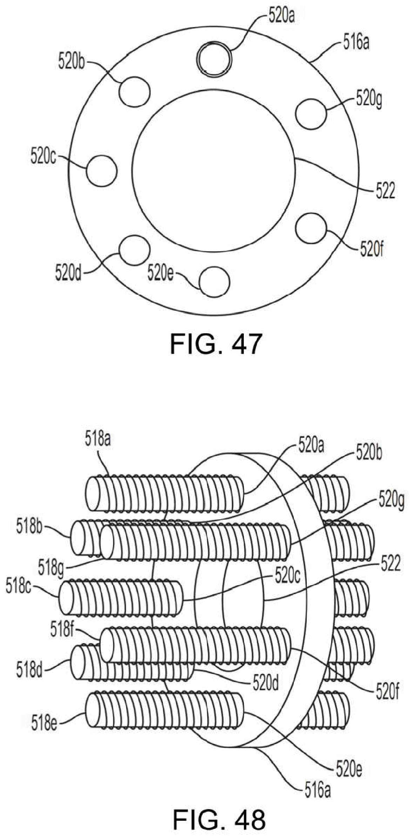

The actuation mechanism **506** may take the form as shown in FIG. 46 and may include a plurality of adaptors **516a-g** configured to engage with a plurality of drive rods **518a-g** (drive rods **518f-g** are marked in FIG. 48). Each adaptor **516a-g** may comprise a plate or other body including a plurality of apertures. FIG. 47 illustrates a front plan view of the adaptor **516a.** The adaptor **516a** as shown in FIG. 47 may include apertures **520a-g** and **522.** The apertures **520a-g** may each be configured to allow a respective drive rod **518a-g** to pass therethrough (as represented in FIG. 48). The apertures **520b-g** may each be configured to be smooth bearing surfaces, that do not engage the respective drive rods **518b-g.** The aperture **520a,** however, may be configured with a threaded surface or other surface that engages the drive rod **518a.** For example, the drive rod **518a** may include a gear threading and the aperture **520a** may include a threading that matches the gear threading. Such a configuration allows the drive rod **518a** to actuate the adaptor **516a** in two directions (distal and proximal) based on the direction that the drive rod **518a** is rotating. In other embodiments, other forms of engagement may be utilized.

The central aperture **522** may allow other components of the actuation mechanism **506** such as assembly connectors to pass through the central aperture to couple to the remaining respective adaptors 516a-g.

FIG. 48 illustrates a perspective view of adaptor **516a** with representative drive rods **518a-g** extending through the apertures **520a-g.**

The other adaptors **516b-g** may be configured similarly as the adaptor **516a,** however, each respective adaptor **516b-g** may have an aperture that is configured to engage the respective drive rods **518b-g,** with the remaining apertures comprising smooth bearing surfaces. For example, for adaptor **516b,** the equivalent aperture to aperture **520b** may be configured to engage drive rod **518b** while the remaining equivalent apertures to apertures **520a, c-g** may comprise smooth bearing surfaces. Adaptors **516c-g** have similar respective apertures configured to engage respective drive rods **518c-g.** In this manner, a single drive rod **518a-g** may be configured to actuate a respective dedicated adaptor **516a-g.** The remaining drive rods may pass through the remaining adaptors without engaging the adaptor.

Referring to FIG. 46, the adaptors **516a-g** may be configured to slide within the interior cavity of the housing comprising the handle **15.** The outer surfaces of the adaptors **516a-g** for example, may be positioned on a track within the handle **15** or otherwise configured to slide or move within the handle **15.**

The drive rods **518a-g** may extend longitudinally along the interior of the handle **15** and may be configured to engage a respective adaptor **516a-g.** For example, FIG. 46 illustrates the adaptor **516a** engaged by drive rod **518a** and the adaptor **516g** engaged by drive rod **518e** (in a configuration in which adaptor **516g** was configured to be engaged by drive rod **518e,** other configurations, e.g., the adaptor **516g** being engaged by drive rod **518g,** may be utilized). Proximal ends of the drive rods **518a-g** may be configured to engage and be actuated by motor **500.**

The adaptors **516a-g** may be coupled to assembly connectors that couple to respective portions of the assemblies (the outer sheath assembly **22**, the mid shaft assembly **21,** the rail assembly **20,** the inner assembly **18,** and the nose cone assembly **31**) including the pull wire assemblies **138, 140.** In certain embodiments, the adaptors **516a-g** may couple to particular components comprising each of the assemblies, for example, the adaptor **516a** may couple directly to the nose cone shaft **27** in certain embodiments. The coupling of the adaptors **516a-g** to the assembly connectors may be such that the adaptor **516a** couples to an assembly connector **521** for the outer sheath assembly **22.** The adaptor **516b** may couple to an assembly connector **523** for the mid shaft assembly **21.** The adaptor **516c** may couple to an assembly connector **524** for the rail assembly **20.** The adaptor **516d** may couple to an assembly connector for the distal pull wires **138** or may couple to the distal pull wires **138** directly. The adaptor **516e** may couple to an assembly connector for the proximal pull wires **140** or may couple to the proximal pull wires **140** directly. The adaptor **516f** may couple to an assembly connector **526** for the inner assembly **18.** The adaptor **516g** may couple to an assembly connector **528** for the nose cone assembly **31.** The assembly connectors **521, 523, 524, 526, 528** may comprise sheaths that extend concentrically over each other, or may comprise rods, wires, or other forms of connectors. The assembly connectors **521, 523, 524, 526, 528** may be configured to pass through the central aperture of the respective adaptors **516a-g** (for example aperture **522** shown in FIG. 47).

The assembly connectors **521, 523, 524, 526, 528** may have a proximal portion coupled to the respective adaptor **516a, b, c, f, g** and a distal portion coupled to a portion of the respective assembly in order to actuate the respective assembly. For example, the assembly connector **521** may couple to the outer sheath assembly **22** such that movement of the assembly connector **521** moves the outer covering, or sheath of the outer sheath assembly **22** to expose the implant **70** in the capsule **106.** The assembly connector **523** may couple to the mid shaft assembly **21** such that movement of the assembly connector **523** moves the outer retention member **42.** The assembly connector **524** may couple to the rail assembly **20** such that movement of the assembly connector **524** moves the rail assembly **20.** The movement of the adaptors **516d** and **516e** may move the respective pull wires **138, 140.** The assembly connector **526** may couple to the inner assembly **18** such that movement of the assembly connector **526** moves the inner retention member **40.** The assembly connector **528** may couple to the nose cone assembly **31** such that movement of the assembly connector **528** moves the nose cone **28.** The respective drive rod **518a-g** may thus be actuated by the motor **500** to selectively move a respective adaptor **516a-g** and accordingly a respective portion of the assemblies (the outer sheath assembly **22,** the mid shaft assembly **21,** the rail assembly **20,** the inner assembly **18,** and the nose cone assembly **31**).

The motion of the assemblies (the outer sheath assembly **22,** the mid shaft assembly **21,** the rail assembly **20,** the inner assembly **18,** and the nose cone assembly **31**) may be a translation of the respective assemblies, which may include the pull wires **138, 140,** to produce the desired movement (e.g., deflection) or operation (e.g., deployment of the implant). For example, the motor **500** may be configured to translate a rail shaft of the rail assembly **20** relative to an inner sheath of the inner assembly **18** and the outer sheath of the outer sheath assembly **22.** The motor **500** may be configured to translate the outer sheath of the outer sheath assembly **22** relative to the inner sheath of the inner assembly **18** in certain embodiments. The motor **500** may be configured to translate any of the assemblies relative to each other to produce a desired result. The motor **500** may be configured to steer the rail assembly **20,** for example, by actuating the pull wires **138, 140.** Other movements may include actuating a depth of the elongate shaft **12,** and actuating an operation of the elongate shaft **12,** for example a full or partial deployment of the implant **70.** The movement may be of a deflection mechanism disclosed herein.

In other embodiments, the actuation of the delivery apparatus with the motor **500** may occur in a different manner than shown in FIG. 46. In one embodiment the configuration of the actuation mechanism **506** may differ from the configuration shown in FIG. 46.

The delivery system **10** may include a controller **530** that is configured to control operation of the motor **500** and thus control actuation of the portion of the delivery apparatus. The controller **530** as shown in FIG. 46 may include an input device and an output device (marked as item **532**). The controller **530** may include a memory **534** and a processor **536.** The controller may include a power source **538.**

The input device and output device **532** may have a plurality of configurations, including electrical ports or terminals that are configured to transmit electrical signals. The input device may be configured to receive signals from the motor **500** as well as from sensors positioned on the delivery system **10.** The output device may be configured to transmit signals to the motor **500** or other components of the system **10** which may be received from the processor **536** or other components of the system **10.** In certain embodiments, the input device and output device **532** may comprise wireless transmission devices, such as a Wi-Fi or Bluetooth device or other device configured for wireless communication. In an embodiment in which the controller **530** is positioned remotely from the delivery apparatus, the input device and output device **532** may be configured to transmit and receive information via the Internet or other form of communication medium. In other embodiments, other forms of input devices and output devices may be utilized.

The memory **534** may be configured to store programs for operation by the processor **536** as well as other data desired to be stored in the controller **530.** The memory **534** may be configured to store and log data regarding the patient and the operation of the delivery apparatus and the motor **500** during a procedure, thereby allowing the system to learn from past events. The learning aspect may be based on an algorithm capable of identifying procedures that have produced positive outcomes in the past, thereby allowing the system to continually refine the procedure to enhance the probability of success. Preferably, data could be pooled from different patients, different clinicians and/or different hospitals. The compilation of data could be used to increase precision and improve outcomes in future procedures. This could be achieved, for example, by comparing characteristics of a new patient with patients who have been treated in the past. Data from procedures on past patients with similar anatomies and/or other parameters, such as the patient's gender, age, and health, would be particularly useful. Other parameters could be incorporated into the algorithm, such as the clinician's skill level and amount of experience and/or the facilities available at the hospital. The data may be used in a machine learning algorithm utilizing data from past implantation procedures or from characteristics of the patient.

The memory **534** may comprise various forms of memory including a hard disk, solid state memory, various forms of RAM or ROM, or other forms of memory. In one embodiment, the memory **534** may be configured to be removable from the controller **530** for storage and/or data analysis. Separate memory **534** may be installed into the controller **530** or swapped into or out of the controller **530** as desired for particular forms of operation.

The processor **536** may be configured to perform processes disclosed herein and may be configured to provide signals to components of the system **10** for example, the motor **500** to perform desired processes. The processor **536** may be configured to operate the motor **500,** or at least one motor **500,** to actuate at least a portion of the delivery apparatus. The processor **536** may be configured to operate at least one motor **500** to move a portion of the delivery apparatus (e.g., deflect or control a depth of the elongate shaft **12**), or perform an operation of the delivery apparatus, which may include deploying the implant **70** from the delivery apparatus. The processor **536** may be configured to execute processes stored in the memory **534.** The processor **536** may be configured to receive signals from components of the system **10** such as a control device (for example control device **504**) or sensors of the system **10.** The processor **536** may be configured to process and perform operations based on those signals. The processor **536** may comprise a microprocessor, or other form of processor as desired. In one embodiment, the processor **536** may comprise a plurality of processors, and in one embodiment may be distributed in a cloud computing environment or the like.

The power source **538** may be configured to provide power to the components of the controller 530, and may be configured to provide power to the motor **500** or other components of the system **10.** The power source **538** may comprise one or more batteries according to certain embodiments, which may be rechargeable and detachable from the controller **530** or other components of the system **10** as desired. In one embodiment, the power source **538** may comprise a power plug such as an AC plug, and may include a power regulator for converting the AC power to a power usable by the system **10.** Other forms of power sources **538** (e.g., super capacitors, solar cells, among others) may be used in other embodiments as desired.

The components of the controller **530** may be positioned together as shown in FIG. 46 or may be distributed as desired. The components of the controller **530** may be positioned in a separate housing, or control box, and may be coupled to the delivery apparatus with a cable or the like. FIG. 46 illustrates a cabled connection of the controller **530** to the delivery apparatus. In other embodiments, wireless communication may be possible between one or more components of the controller **530** and the delivery apparatus. In other embodiments, components of the controller **530** may be positioned within the housing of the delivery apparatus, for example, in a configuration shown in FIG. 61.

Power and signal connectors **540** may extend between the controller **530** and the delivery apparatus. For example, a signal connector **540** is shown extending along a portion of the handle **15** and may couple between the distal portion **508** of the handle **15** and the proximal portion **510** at the electrical coupler **542.** Power connectors **540** may extend to the motor **500** from the power source **538** of the controller **530.**

FIG. 49 illustrates a perspective view of the distal portion **508** of the handle **15.** The distal portion **508** of the handle **15** may be configured to separate from the proximal portion **510** (shown in FIG. 50). Such a configuration may allow a particular portion of the handle **15** of the delivery apparatus to be utilized in delivery of an implant, and then separated from another portion (e.g., proximal portion **510**) of the handle **15** such that sterilization or discard of the distal portion **508** may occur. This process may separate electrical components of the system **10,** which may include the motor **500** positioned within the proximal portion **510,** or may include the controller **530,** from components that are inserted into or contact portions of the patient's body. This may enhance reusability of the system **10** and reduce the overall complexity associated with sterilizing the system **10.** As shown in FIG. 49, proximal portions of the drive rods **518a-g** may extend proximally from the distal portion **508** of the handle **15,** for coupling to respective apertures **544a-g** in the proximal portion **510** of the handle **15.** The proximal portions of the drive rods **518a-g** may couple to the respective apertures **544a-g** to allow the motor **500** to engage the drive rods **518a-g.** The electrical coupler **542** and coupler **512** are also shown protruding from the distal portion **508** of the handle **15.**

FIG. 50 illustrates a perspective view of the proximal portion **510** of the handle **15.** The proximal portion **510** may include a cable **546** or other connector that couples the proximal portion **510** to the controller **530,** which may be contained in a control box or the like.

Referring again to FIG. 49, the control device **504** is shown on the distal portion **508** of the handle **15** as including a plurality of buttons. The control device **504** may be configured to receive an input from a user to operate the motor **500** and thus actuate a portion of the delivery apparatus. The control device **504** may be configured to send a signal directly to the motor **500** or may be sent to the processor **536** of the controller **530** for processing. The control device **504** may be configured to control deflection and movement of the delivery apparatus. The control device **504** may be configured to control an operation of the delivery apparatus such as deployment of the implant **70.** The control device **504** may have a variety of forms, and as shown in FIG. 49 may have portions designated to control certain movements or operations of the delivery apparatus.

The control device **504** of FIG. 49 may include buttons **548** that control the rail assembly **20** and particularly the direction of deflection of the rail assembly **20,** which may be in at least two planes. The buttons **548** may be configured to control steering of the rail assembly **20.** The user may press the desired button **548** to cause the motor to actuate the delivery apparatus to deflect in the desired direction. The control device **504** of FIG. 49 may include buttons **550** that control the depth of the elongate shaft **12,** for example, by sliding the assemblies including the outer sheath assembly **22,** the mid shaft assembly **21,** the inner assembly **18,** and the nose cone assembly **31,** relative to the rail assembly **20.** The buttons **550** may allow the user to increase or decrease the depth. The control device **504** of FIG. 49 may include buttons **552** that actuate deployment of the implant **70.** For example, the buttons **552** may cause the motor to actuate the delivery apparatus to retract the outer sheath assembly **22** and the mid shaft assembly **21** to deploy the implant **70.** The control device **504** of FIG. 49 may include buttons **554** that actuate movement of the nose cone assembly **31,** to advance or retract the nose cone **28.** Various configurations of control may be utilized to deflect the delivery apparatus or to perform operations of the delivery apparatus. The control signals from the control device **504** may be sent directly to the motor **500** for operation, or may be sent to the processor **536** for the processor **536** to operate the motor **500** to actuate at least a portion of the delivery apparatus. The configuration of the control device **504** may be varied in other embodiments. The control signals may be utilized to operate a deflection mechanism as disclosed herein.

Other embodiments of control devices that may be utilized include buttons, joysticks, touchpads, touch screens, knobs, or motion sensing devices, among other forms of control devices.

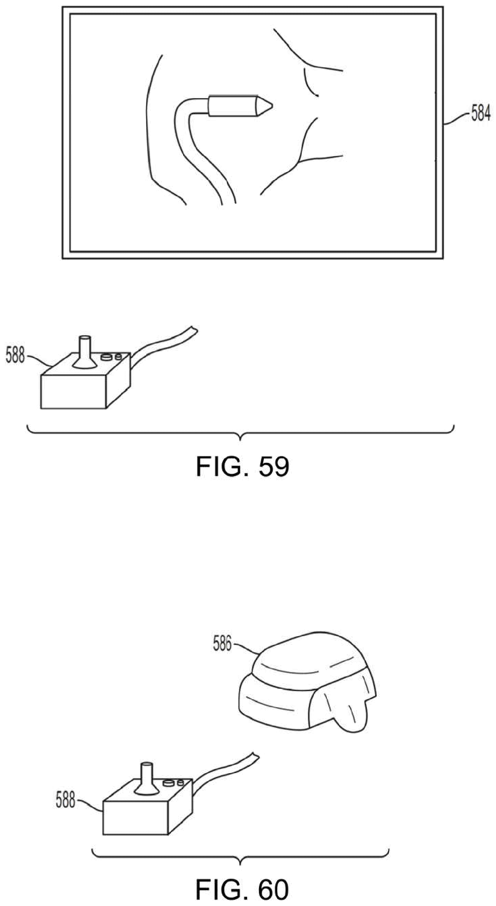

The system **10** may include an output device that may have various forms. The output device may be configured to provide an output to a user that may indicate a condition of the delivery apparatus or of the patient. The output device may be configured to provide an indicator of a condition of the delivery apparatus or of the patient. The output device may include lights that may illuminate to indicate a condition of the delivery apparatus or of the patient. The lights may illuminate to indicate the delivery apparatus has contacted or approached a surface of the patient's body (a condition of the delivery apparatus), or may illuminate to indicate a certain condition of the patient's body, such as a correct or incorrect pressure being sensed in the patient's body. Other forms of output devices may be utilized, including a haptic device , such as a vibrating actuator, which may indicate the condition of the delivery apparatus or of the patient. An output device may include the display screen of the touch screen. An output device may include a display screen **584** as shown in FIG. 59. An output device may include one or more of a display screen, a light, a speaker, or a haptic device, among other forms of output devices. Various forms of output devices may be utilized as desired. An indicator produced on the output device may include one or more of an image, data, a sound, a light, or a haptic signal. The output device may be configured to provide an indicator based on an output provided by the processor **536.**

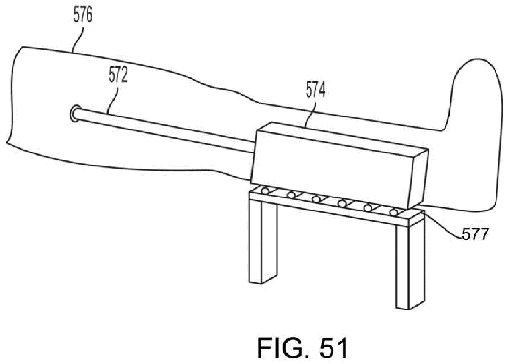

The actuation of the delivery apparatus by at least one motor may include a translation of the elongate shaft **12** and may include a translation of a housing at a proximal end of the elongate shaft **12.** Axial translation of the delivery apparatus may be provided. FIG. 51, for example, illustrates a side perspective view of a delivery apparatus including an elongate shaft **572** and a housing **574.** The delivery apparatus is being passed transfemorally into a patient's body **576.** The elongate shaft **572** may be configured similarly as the elongate shaft **12.** The housing **574** may be configured similarly as the housing forming the handle **15,** however the housing **574** may not comprise a handle for grip by a user. Rather the housing **574** may include a motor or may be configured to move along a motor driven rail **577** or other assembly that actuates axial movement of the delivery apparatus into the patient's body. The axial movement of the delivery apparatus may be controlled by a control device, which may be positioned proximate the housing **574** or may be located remote from the housing **574.**

The motor **500** may be configured to actuate the delivery apparatus by selectively moving one or more of the outer sheath assembly **22,** the mid shaft assembly **21,** the inner assembly **18,** the rail assembly **20,** the assembly including the distal pull wires **138,** the assembly including the proximal pull wires **140,** and the nose cone assembly **31.** The motor may be configured to perform any other method, or may be utilized with any embodiment disclosed herein, including the embodiments of FIGS. 13A-44 and 62A-64C.

In certain embodiments, the processor **536** may be utilized to automatically move the assemblies or other portions of the elongate shaft **12** to perform the operations of the delivery apparatus. For example, if a request is made to increase the depth of the elongate shaft **12** or deploy the implant **70,** then the processor **536** may be configured to operate a program (which may be stored in memory **534)** to control the motor **500** to move the corresponding assemblies or other portions of the elongate shaft **12.** If a request is made that requires compensation of movement, then the processor **536** may be configured to operate a program (which may be stored in memory **534)** to control the motor **500** to move the corresponding assemblies or other portions of the elongate shaft **12** to automatically perform such compensation. The processor **536** may be configured to operate the motor to move one of the assemblies to compensate for a motion of another of the assemblies. Particular movements and combinations of movements of the assemblies or other portion of the elongate shaft **12** may be programmed into the memory **534** and operated by the processor **536.** As discussed above, the programmed movements may be based on data "learned" from previous procedures and, in particular, learned from previous procedures performed on patients with similar anatomies and/or other characteristics. The movements may be based on a machine learning algorithm utilizing data from past implantation procedures or from characteristics of the patient. Therefore, procedural steps performed successfully on patients with similar anatomies could be duplicated, thereby increasing the probability of a successful procedure on the current patient. The processor **536** may be configured to automatically operate the motor **500** to actuate a portion of the delivery apparatus in a desired manner.

The system **10** may include sensors that are configured to sense a condition of the delivery apparatus and may include sensors that are configured to sense a condition of the patient.

In certain embodiments, a sensor may be utilized to sense a condition of the delivery apparatus. The sensor may comprise a position sensor that may be utilized to determine the movement and/or position of one or more of the assemblies. For example, the position sensor may be configured to sense the amount that the motor **500** has moved the assembly to track the position and movement of the assembly. The motor **500** may be wired to track movement of the various assemblies and perform a desired movement (e.g., simultaneous movement of assemblies, or compensatory movement of one or more assemblies) based on the signal from the position sensor. In one embodiment, the signal from the position sensor may be provided to the processor **536** for the processor **536** to perform a desired movement. The signal from the position sensor may be a feedback signal to the processor **536.** For example, the position sensor may sense that a portion of the elongate shaft **12** is moving in response to movement of another portion of the elongate shaft **12,** and the processor **536** may operate the motor **500** to produce compensatory movement based on this signal. An indicator indicating a position of the delivery apparatus may be provided on an output device, as discussed herein. The indicator may be provided based on the position sensed by the position sensor.

A sensor may be utilized to sense a condition of the delivery apparatus in the form of a motor torque sensor. The sensor may be utilized to determine the amount of torque exerted by the motor **500.** The motor torque sensor, for example, may be a current draw sensor able to sense the amount of current drawn by the motor **500.** If the amount of torque exceeds a certain amount, the motor **500** may be configured to automatically shut off or reverse its operation or reduce torque. In one embodiment, the signal from the motor torque sensor may be provided to the processor **536** for the processor **536** to perform a desired movement. The signal from the motor torque sensor may be a feedback signal to the processor **536.** For example, the processor **536** may operate the motor **500** to automatically shut off or reverse its operation or reduce torque based on this signal. An indicator indicating a torque of a motor of the delivery apparatus may be provided on an output device, as discussed herein. The indicator may be provided based on the torque sensed by the motor torque sensor.

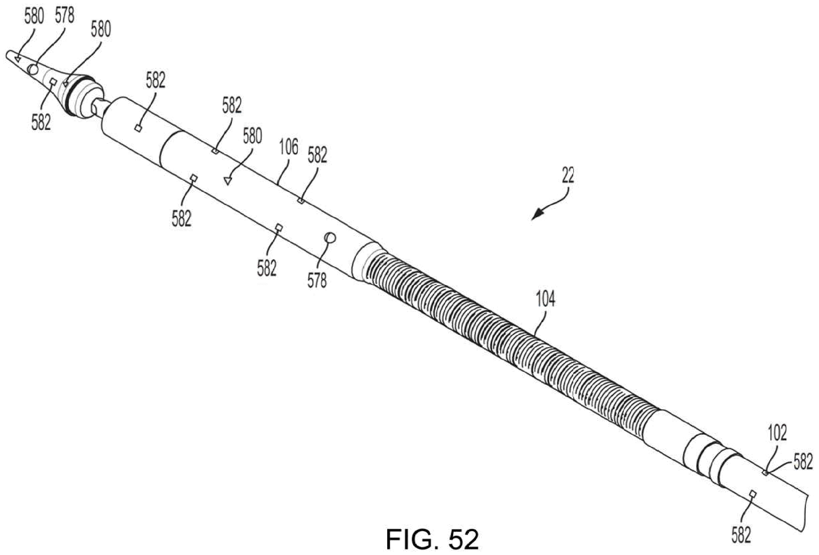

Referring to FIG. 52, sensors configured to sense a condition of the patient may be utilized. Such sensors may be positioned as desired on the delivery apparatus. Sensors configured to sense a condition of the patient may include ambient pressure sensors **578.** Such pressure sensors **578** may be configured to sense a pressure, such as a fluid pressure, within the patient's body. The pressure sensors **578** may be utilized during and following delivery of the implant **70,** to determine whether the deployed implant **70** is operating as desired following implantation, or to generally monitor a condition of the patient before and following implantation. In the embodiment shown in FIG. 52, a pressure sensor **578** may be positioned on the nose cone **28** and a pressure sensor may be positioned on the capsule **106** among other locations. With this particular configuration of pressure sensors **578,** one pressure sensor may be positioned in the right ventricle during implantation of the implant **70,** and one pressure sensor may be positioned in the right atrium during implantation. Thus, following implantation, the pressure gradient across the mitral valve can be determined. A signal from the pressure sensors **578** may be provided to an output device (such as output devices **568, 570,** or other output device) for indication to the user. In one embodiment, the pressure sensed by the pressure sensors **578** may be utilized as feedback to the system **10,** such as the processor **536,** to actuate the delivery apparatus. For example, if an incorrect pressure is read, the processor **536** may actuate the delivery apparatus to redeploy the implant or perform another operation. In other embodiments, other positions of pressure sensors **578** and other pressure readings may be provided.

In one embodiment, a sensor configured to sense a condition of the delivery apparatus may include sensors configured to sense a spatial relationship between the delivery apparatus and a surface of the patient's body. Such a sensor may be positioned on the delivery apparatus. Such a sensor may include a contact sensor **580.** A contact sensor **580** may comprise a force transducer or load cell, or other form of contact sensor **580** that is configured to sense a force applied to the delivery apparatus. As shown, a contact sensor **580** may be positioned in a variety of positions on the elongate shaft **12,** including on the nose cone **28** or other locations (such as generally on the outer surface of the elongate shaft 12). A contact sensor **580** may be configured to provide a signal when the elongate shaft **12** contacts a portion of the patient's body. Such a signal may indicate the possibility of damage to the patient's body due to the elongate shaft **12.** A signal from a contact sensor **580** may be provided to an output device (such as output devices **568, 570,** or other output device) for indication to the user. In one embodiment, the contact sensed by the contact sensor **580** may be utilized as feedback to the system **10,** such as the processor **536,** to actuate the delivery apparatus. For example, if contact is sensed with a surface, then the processor **536** may actuate the delivery apparatus to move away from the surface or stop operation of the motor **500.** In other embodiments, other positions of contact sensors **580** and other contact sensors may be provided.

In one embodiment, a sensor configured to sense a condition of the delivery apparatus may include a proximity sensor **582.** The proximity sensor **582** may be configured to sense a spatial relationship between the delivery apparatus and a surface of the patient's body. Such a sensor may be positioned on the delivery apparatus. A proximity sensor **582** may comprise a device for sensing a distance to a portion of the patient's body, including use of ultrasound, or echo signals, or visual identification. As shown, a proximity sensor **582** may be positioned in a variety of positions on the elongate shaft **12,** including on the nose cone **28** or other locations (such as generally on the outer surface of the elongate shaft **12).** The proximity sensor **582** may be configured to provide a signal when the elongate shaft **12** approaches a portion of the patient's body, and may provide such a signal to an output device (such as output devices **568, 570,** or other output device) for indication to the user. In one embodiment, the proximity sensed by the proximity sensor **582** may be utilized as feedback to the system **10,** such as the processor **536,** to actuate the delivery apparatus. For example, if proximity to a surface (e.g., an inner wall of blood vessel) is sensed, the processor **536** may actuate the delivery apparatus to move away from the surface or stop operation of the motor **500.** As such, the delivery system could be advanced through the patient's vasculature without damaging an inner wall of a blood vessel. This "smart catheter" technology could provide a significant improvement over current "blind catheters." For example, this technology could reduce or eliminate the possibility of vascular dissection, which is a significant and life-threatening risk with current delivery systems. Although embodiments have been described for sake of explanation, it will be understood that other positions of proximity sensors **582** and other proximity readings may be provided.

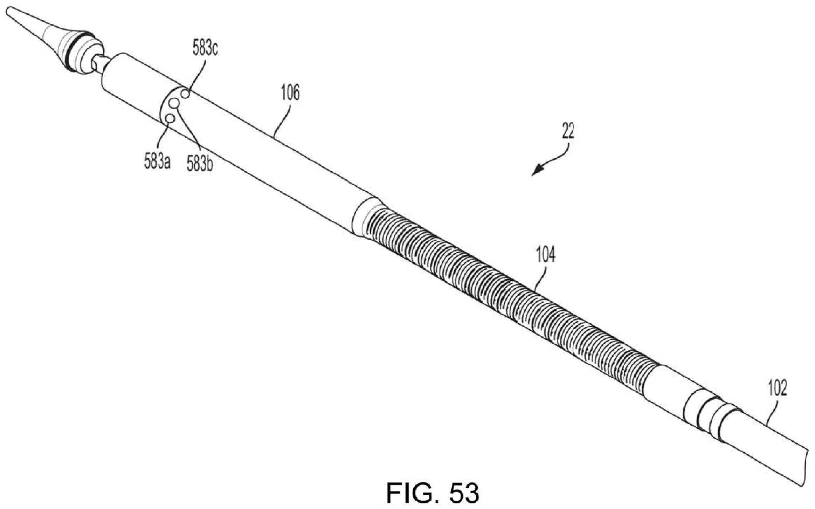

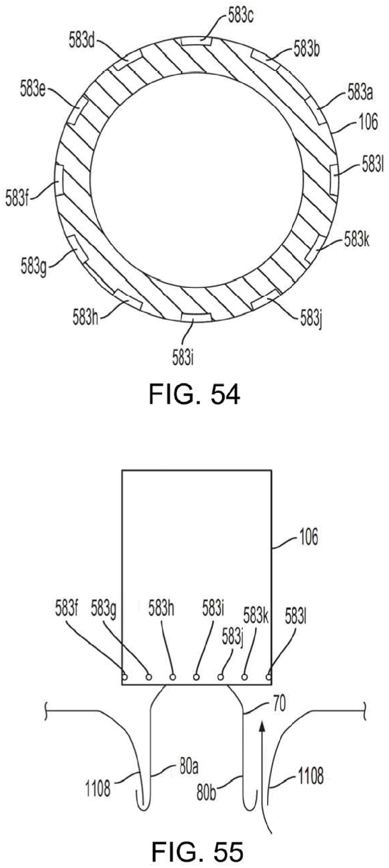

FIGS. 53-55 illustrate an embodiment of a sensor configured to sense a condition of the patient. The sensor comprises a flow sensor that may sense a fluid flow (e.g., blood flow) within the patient's body. A plurality of sensors **583a-l** (as marked in FIG. 54) may be positioned on the delivery apparatus forming a spaced array of sensors **583a-l.** The sensors **583a-l** may be configured to sense a local fluid flow, such that the sensors **583a-l** may sense a fluid flow in a local area in the body that is different from the fluid flow sensed by other sensors **583a-l.** FIG. 53 illustrates a perspective view of the distal end of the elongate shaft **12,** with sensors **583a-c** visible on the capsule **106.** FIG. 54 illustrates a cross sectional view of the capsule **106** showing the spaced array of sensors **583a-l.** The sensors **583a-l** may be positioned on the delivery apparatus to sense fluid flow at a location proximate the deployment location for the implant **70.** Such a location may comprise the capsule **106** or another portion of the delivery apparatus.

FIG. 55 illustrates an exemplary operation of the sensors **583a-l.** The implant **70** may be deployed to the tricuspid valve, with one distal anchor **80a** capturing a leaflet **1108** and another distal anchor **80b** failing to capture a leaflet **1108.** The sensors **583k, 583l** may sense a flow of blood by the mis-captured leaflet **1108** and may provide a signal accordingly. The sensors **583a-l** may be configured to sense a differential flow between the sensors **583f, 583g** proximate the captured leaflet **1108** and the sensors **583k, 583l** proximate the mis-captured leaflet **1108.** The flow sensors **583a-l** may be configured to provide a signal when a flow is sensed, and may provide such a signal to an output device (such as output devices **568, 570,** or other output device) for indication to the user. In one embodiment, the flow sensed by the flow sensors **583a-l** may be utilized as feedback to the system **10,** such as the processor **536,** to actuate the delivery apparatus. For example, if flow is sensed indicated a mis-capture of a leaflet, then the processor **536** may actuate the delivery apparatus to redeploy the implant **70** or perform another operation. In other embodiments, other positions of flow sensors **583a-l** and other flow readings may be provided.

The sensors that are configured to sense the condition of the delivery apparatus and the sensors that are configured to sense a condition of the patient may be coupled to the delivery apparatus. In certain embodiments, however, the sensors that are configured to sense the condition of the delivery apparatus and the sensors that are configured to sense a condition of the patient may not be coupled to the delivery apparatus and may be external to the patient's body.

The signals from the sensors that are configured to sense the condition of the delivery apparatus and the sensors that are configured to sense a condition of the patient, may be utilized in a variety of manners. In one embodiment, the signals may be provided as indicators on an output device (such as output devices **568, 570,** or other output device) for indication to the user. For example, a condition of the delivery apparatus may be indicated to a user in a variety of forms, for example, an output device may include one or more of a display screen, a light, a speaker, or a haptic device, among other forms of output devices. An indicator produced on the output device may include one or more of an image, data, a sound, a light, or a haptic signal. The user may be able to act accordingly based on the indicator. For example, if an indicator indicates that the delivery apparatus has contacted a portion of the patient's body, then the user may act accordingly to move the delivery apparatus away from the body. A condition of the patient's body may similarly be indicated to a user in a variety of forms.

In embodiments, the signals from the sensors that are configured to sense the condition of the delivery apparatus and the sensors that are configured to sense a condition of the patient may be provided to the processor **536.** The processor **536** may provide a variety of outputs based on the one or more of a condition of the patient's body or a condition of the delivery apparatus sensed by the one or more sensors. One such form of output includes a log of data for an implantation procedure with the delivery apparatus. Such a log of data may be stored in the memory **534.** The data may be stored for later retrieval by a user for analysis or may record a log of actions taken by the delivery apparatus. For example, the position sensor signals may be logged to record the movements of the delivery apparatus, among other forms of sensors signals.

The processor **536** may provide an output to an output device based on the condition of the patient's body or a condition of the delivery apparatus sensed by the one or more sensors. The output may result in an indicator on an output device (such as output devices **568, 570,** or other output device) for indication to the user. For example, a condition of the delivery apparatus may be indicated to a user in a variety of forms, for example, an output device may include one or more of a display screen, a light, a speaker, or a haptic device, among other forms of output devices. The processor **536** may process the signals to produce a desired indicator to a user. For example, the sensors **583a-l** may sense a flow of blood during deployment of the implant **70,** and the processor **536** may process these signals to provide an indicator to a user that leaflet mis-capture has occurred.

The processor **536** may provide an output that comprises a control of the motor **500** based on the condition of the patient's body or a condition of the delivery apparatus sensed by the one or more sensors. The processor **536** may be configured to operate the motor **500** to actuate the delivery apparatus based on a signal from the sensors. The signal from the sensors may comprise feedback signals that are input to the processor **536** for the processor to control operation of the motor **500.** For example, a signal from a contact sensor **580** or a proximity sensor **582** may be provided to the processor **536** as feedback that the delivery apparatus has contacted or is proximate a surface of the patient's body. The processor **536** accordingly may provide an output that operates the motor **500** to avoid or retract from the surface of the patient's body. A signal from the flow sensors **583a-l** may cause the processor **536** to provide an output to the motor **500** to redeploy the implant **70** or move the portion of the delivery apparatus to recapture the leaflet **1108.** A signal from a position sensor may provide feedback to the processor **536** regarding whether the delivery apparatus is performing the correct movements, and the processor **536** may operate the motor **500** to perform corrective movements if desired (e.g., deflect the elongate shaft **12** if needed). The processor **536** may be programmed to automatically respond and produce outputs based on the condition of the patient's body or a condition of the delivery apparatus sensed by the one or more sensors. The programming for the processor **536** may be stored in the memory **534** and operated by the processor **536.**

The delivery system can be used in a method for percutaneous delivery of a replacement tricuspid valve to treat patients with moderate to severe tricuspid regurgitation. However, it will be understood that the delivery systems described herein can be used as part of other methods as well, such as implants for repair of valves and delivery of implants to other heart valves and delivery of other implants.

In one embodiment, a method may include extending a delivery apparatus within a portion of the patient's body to deliver an implant to a body location. The delivery system **10** can be placed in the ipsilateral femoral vein and advanced toward the right atrium. Accordingly, it can be advantageous for a user to be able to steer the delivery system **10** through the complex areas of the heart in order to position a replacement tricuspid valve in line with the native tricuspid valve. This task can be performed with or without the use of a guide wire. The distal end of the delivery system can be advanced towards or into the left atrium. The motor **500** may then be operated to actuate the rail assembly **20** or the deflection mechanism to target the distal end of the delivery system **10** to the appropriate area. The motor **500** may be operated by a processor **536** as discussed herein. The motor **500** may be operated to create a variety of bends in the rail assembly **20** and deflect the elongate shaft **12** in a variety of manners to place the implant in the desired location for implantation.

The operation of the motor **500** may be operated by a processor **536.** A user may provide input to the processor **536** with a control device **504.**

Further the sensors discussed herein may be utilized in certain embodiments. The delivery apparatus may include one or more sensors coupled to the delivery apparatus and configured to sense one or more of a condition of the patient's body or a condition of the delivery apparatus. The processor **536** may be configured to provide an output based on the one or more of a condition of the patient's body or a condition of the delivery apparatus sensed by the one or more sensors. For example, the processor may cause at least a portion of the delivery apparatus to avoid or retract from a surface of the patient's body based on a condition of the delivery apparatus.

The use of a processor, one or more sensors, and/or one or more motors with a delivery system, as disclosed herein, may be configured to perform any other method, or may be utilized with any embodiment disclosed herein, including the embodiments of FIGS. 13A-44 and 62A-64C.

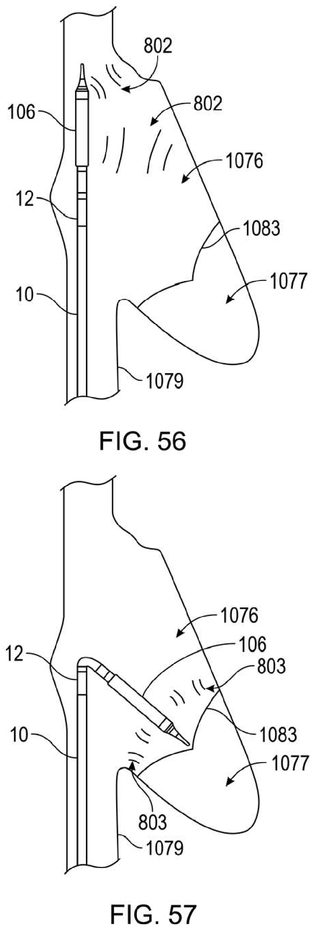

In embodiments, the delivery system **10** can be used in a method for percutaneous delivery of a replacement tricuspid valve that may be used to treat patients with moderate to severe tricuspid regurgitation. Such a method may utilize any of the systems or devices disclosed herein. Referring to FIG. 56, for example, the delivery apparatus may be extended within a portion of a patient's body to deliver an implant to a body location. The portion of the patient's body may be the right atrium **1076** and the body location for delivering the implant may be the native tricuspid heart valve **1083.** The delivery apparatus may be extended within a portion of the patient's body in a similar manner as disclosed herein, for example, the delivery apparatus can be placed in the ipsilateral femoral vein and advanced towards the right atrium **1076.** Other entry methods may be utilized as desired.

The delivery apparatus may be extended within the inferior vena cava **1079** into the right atrium **1076.** One or more motors, which may be operated by a processor **536** as discussed herein, may be utilized to extend the delivery apparatus into the right atrium **1076.**

The delivery apparatus may be steered through the complex areas of the heart in order to position a replacement tricuspid valve in line with the native tricuspid valve. The motor **500** may be operated to actuate the rail assembly **20** to target the distal end of the delivery apparatus to the appropriate area. For example, the motor **500** may be utilized to steer the rail assembly **20** to the desired orientation relative to the tricuspid heart valve **1083.** The motor **500** may be operated by a processor **536** as discussed herein. The rail assembly **20** may form one or more bends such that the distal end of the delivery apparatus is oriented coaxial with the native tricuspid heart valve **1083.**

FIG. 57, for example, shows that the delivery apparatus has been deflected within the right atrium **1076** towards the native tricuspid heart valve **1083.** One or more bends may be formed within the right atrium **1076** and/or the inferior vena cava **1079.** Once the implant **70** is positioned coaxial with the native tricuspid heart valve **1083,** the outer sheath assembly **22,** mid shaft assembly **21,** inner assembly **18,** and nose cone assembly **31** can together be advanced (e.g., using the motor 500) distally relative to the rail assembly **20** towards the right ventricle **1077.** The depth of the elongate shaft **12** may be varied by the operation of the motor **500** disclosed herein, which may be operated by a processor **536.** The proximal/distal translation of the other assemblies over the rail assembly **20** allows for ventricular-atrial motion. Further, a deflection mechanism as disclosed herein may be utilized. Other features from other embodiments disclosed herein may be utilized as desired.

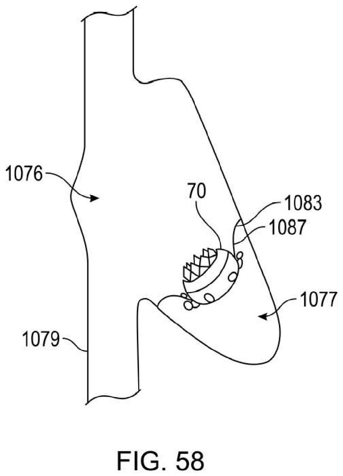

The depth of the elongate shaft **12** may be varied until the capsule **106** is positioned in the desired location relative to the native tricuspid heart valve **1083.** The distal end **303** of the implant **70,** and specifically the distal anchors **80,** may be restrained within the capsule **106** of the outer sheath assembly **22,** thus preventing expansion of the implant **70.** Similar to what is shown in FIG. 2A, the distal anchors **80** can extend distally when positioned in the capsule. The proximal end **301** of the implant **70** is restrained within the capsule **106** and within a portion of the inner retention member **40** and thus is generally constrained between the capsule **106** and the inner retention member **40.** The implant **70** may then be deployed to the native tricuspid heart valve **1082.** FIG. 58, for example, illustrates the implant **70** deployed to the native tricuspid heart valve **1082.** The distal anchors of the implant **70** extend over the leaflets **1087** of the tricuspid heart valve **1083.** The delivery apparatus may then be withdrawn from the patient's right atrium **1076.**

The method may utilize the systems and devices disclosed herein. For example, the motor **500** may deflect a portion of the delivery apparatus or deploy the implant to the body location. The motor may operate a deflection mechanism as disclosed herein, or other feature of an embodiment disclosed herein, including controlling operation of the embodiments of FIGS. 13A-44 and 62A-64C. The operation of the motor **500** may be operated by a processor **536.** A user may provide input to the processor **536** with a control device **504.** The system **10** can be positioned through the use of the steering mechanisms discussed herein or other techniques. The delivery system **10** can be advanced by the user manually moving the handle **15** in an axial direction. In some embodiments, the delivery system **10** can be placed into a stand while operating the handle **15** controls.

The delivery apparatus may be utilized in the form shown in FIG. 1, or other forms of delivery apparatuses may be utilized, for example, delivery apparatuses configured for delivery of an implant to the native tricuspid valve.

In other embodiments, other methods of delivering the implant to the native tricuspid heart valve may be utilized, for example, a transapical, transseptal, or other method may be utilized.

Other locations for valve implant may include the aortic or pulmonary valve, and other valves of a patient's body. Other forms of implants may be delivered to other body locations as desired.

In some embodiments, the implant **70** can be delivered under fluoroscopy so that a user can view certain reference points for proper positioning of the implant **70.** Further, echocardiography can be used for proper positioning of the implant **70.**

In one embodiment, the proximity sensor **582** may be configured to provide a model of the interior of the patient's body and the spatial relationship of the elongate shaft **12** from surfaces of the patient's body. Such a model may be provided on output devices **584, 586** shown in FIGS. 59 and 60 as display screens (on a monitor and on a virtual reality or augmented reality display). Such a model may also be provided by other sensors positioned external to the patient's body if desired. Such a model may be a two-dimensional map or three-dimensional map of the patient's body for view by a user, and for use by the processor **536** as feedback to navigate through the patient's body and deliver an implant **70** to the desired location.

FIG. 59 illustrates an embodiment in which operation of the delivery apparatus may occur remotely by a user. The user may utilize a control device **588** such as a joystick or other form of control device to control movement of the delivery apparatus and elongate shaft **12.** The control device **588** may be configured to sense motion of the control device to control the delivery apparatus. The user may view the position of the elongate shaft **12** on an output device **584** in the form of a display screen. The position may be provided in a variety of manners, including external sensing of the position via sensors using fluoroscopy or echocardiography. The position may also be provided via an image produced by signals from proximity sensors of the elongate shaft **12.** The proximity sensors may be configured to produce an image of the spatial relationship between the elongate shaft **12** and the surfaces of the patient's body. A configuration including a motor for axial movement of the elongate shaft **12,** as shown in FIG. 54, may be utilized as well for remote control of the procedure.

FIG. 60 illustrates an embodiment in which the output device **586** is in the form of a display screen on a virtual reality or augmented reality display. The display may include a helmet (or other headset that allows for enhanced visualization) for wear by the user, wherein the user is able to move his or her head to alter the perspective of the view provided by the display screen. Similar to the embodiment discussed with respect to FIG. 59, the position of the elongate shaft **12** and portions of the patient's heart seen in the output device **586** may be provided in a variety of manners, including external sensing of the position via fluoroscopy or echocardiography. The position may also be provided via an image produced by signals from proximity sensors of the elongate shaft **12.** The proximity sensors may be configured to produce an image of the spatial relationship between the elongate shaft **12** and the surfaces of the patient's body. A configuration including a motor for axial movement of the elongate shaft **12,** as shown in FIG. 51, may be utilized as well for remote control of the procedure.

In an exemplary method, a user (e.g., clinician) may provide input, which may be assisted by use of the components disclosed herein (e.g., the processor, motor, and one or more sensors, among other components). In embodiments, however, an implantation procedure may occur autonomously (i.e., adapts to environment during operation). The processor may perform autonomous control of the delivery apparatus to perform the implantation procedure. A user may provide some input during the procedure, such that the procedure may occur semi-autonomously. As such, a method may occur autonomously or semi-autonomously (or at least semi-autonomously). Other autonomous procedures may include autonomously performing the methods disclosed with respect to the embodiments of FIGS. 13A-44 and 62A-64C.

A method may include extending a delivery apparatus within a portion of a patient's body to deliver an implant to a body location. The delivery apparatus may be configured similarly as any embodiment of delivery apparatus disclosed herein. The delivery apparatus may be extended within a portion of the patient's body as disclosed herein. The implant may be configured similarly as any implant disclosed herein, and the body location may comprise any location disclosed herein.

The delivery apparatus may be extended within the portion of the patient's body by way of a motor advancing the delivery apparatus, such as the elongate shaft of the delivery apparatus within the patient's body. The motor may be controlled by the processor **536.** For example, a motor driven rail 577, or other assembly that actuates axial movement of the delivery apparatus into the patient's body may be utilized. In other embodiments, other methods may be utilized to extend the delivery apparatus within the portion of the patient's body.

The processor **536** may operate a program to actuate the delivery apparatus. The processor **536** may be programmed with a sequence of movements to actuate the delivery apparatus to the desired location and for the desired deployment operation. For example, the processor **536** may be configured to determine the desired delivery location and the path and orientation to be followed to reach the desired delivery location based on external sensing of the position via fluoroscopy or echocardiography and/or the position being determined via signals from proximity sensors of the elongate shaft **12.** The programmed sequence of movement may be provided based on the geometry of the path to the desired implant location, and the orientation of the desired implant location. The movement and deployment of the delivery apparatus may be preprogrammed into the processor **536** and may be individualized based on the particular path to the desired location in the patient's body to be followed. In certain embodiments, a machine learning algorithm may be utilized by the processor **536** to control actuation of the delivery apparatus. For example, the path and orientation also be supplemented by data from previous procedures on patients with similar characteristics. The processor **536** and programming may be utilized to extend the delivery apparatus within a portion of the patient's body as disclosed.

The processor **536** may continue to follow the program and may receive signals from one or more sensors. The processor **536** may receive feedback from sensors (as discussed herein) that cause the processor **536** to produce outputs. The signals from the sensors may be utilized by the processor **536** in a similar manner as disclosed herein. For example, the processor **536** may be configured to produce a log of data. The processor **536** may be configured to produce an indicator. The indicator may be provided for a user to determine whether to intervene in a procedure. For example, if a user (e.g., a clinician) receives an indicator that the autonomously operated delivery apparatus has contacted a surface or has improperly deployed an implant, then the user may intervene to attempt to correct such actuation.

The processor **536** may be configured to produce actuation of the delivery apparatus. The actuation may be provided for the processor **536** to correct the path and operation with minimal or no human interaction using feedback from sensors as discussed herein, to complete the procedure. For example, if the position sensor indicates the delivery apparatus is straying from the intended path, the processor **536** may automatically adjust the path. If the proximity sensor indicated the delivery apparatus is approaching a surface, then the processor **536** may automatically adjust the path. The processor **536** may be used to navigate to any desired location for delivery of the implant. Any of the sensors and feedback operations from the sensors disclosed herein may be utilized in such a method. In certain embodiments, a user may provide some input during the procedure to correct the procedure or otherwise provide input to control the procedure.

The actuation produced by the processor **536** may be based on a machine learning algorithm utilizing data from past implantation procedures or from characteristics of the patient. The actuation may be based on data "learned" from previous procedures and, in particular, learned from previous procedures performed on patients with similar anatomies and/or other characteristics. Therefore, procedural steps performed successfully on patients with similar anatomies could be duplicated, thereby increasing the probability of a successful procedure on the current patient. A machine learning algorithm may be utilized by the processor **536** to control actuation of the delivery apparatus.

The processor **536** may be configured to operate the motor **500** to produce the desired actuation of the delivery apparatus. The processor **536** may be configured to automatically operate the motor to deflect the delivery apparatus to the desired body location. The processor **536** may be configured to automatically operate the motor to deflect the delivery apparatus in at least two planes. The processor **536** may be configured to automatically deploy the implant **70** to the desired location and complete the delivery procedure. The processor **536** may be configured to complete the delivery procedure in certain embodiments without control or intervention by a user. The processor **536** may be configured to provide such a confirmation of implantation as an indicator on an output device, so that the user is notified that the implant has been implanted.

The methods may be utilized for replacement or repair of a heart valve within a patient's body. The heart valve may comprise one or more of an aortic heart valve, a mitral heart valve, a tricuspid heart valve, or a pulmonary heart valve. Other valves or body locations for implantation may be treated in other embodiments.

FIG. 61 illustrates an embodiment of a delivery apparatus configured similarly as the apparatus shown in FIG. 46, however, multiple motors **502** may be utilized to control actuation of the delivery apparatus. The multiple motors **502,** for example, may each be configured to engage respective adaptors **590, 592, 594** configured to actuate portions of the delivery apparatus. The motors **502** may be configured to perform linear movement of the adaptors **590, 592, 594** to cause actuation of the delivery apparatus. Further, in the embodiment of FIG. 61, the processor, memory, and input device and output device of FIG. 61 may be provided on a printed circuit board **596** positioned within the handle. A power source **598** such as a battery pack or other form of power source may also be utilized within the handle. The embodiment of FIG. 61 may comprise a self-contained handle unit including a processor for performing a delivery procedure and receiving feedback from sensors, as well as performing data logging if desired.

The motors disclosed herein may comprise a variety of forms of motors, including electromagnetic, stepper, hydraulic, piezoelectric, among others. The methods, systems, and apparatuses disclosed herein with respect to FIGS. 45-61 may be utilized with any embodiment disclosed herein. For example, actuation and control of any of the systems, apparatuses, or methods of any of the embodiments of FIGS. 13A-44 or 62A-64C may occur under the operation of the systems, apparatuses or methods of the embodiments of FIGS. 45-61.

Although many of the systems and methods disclosed herein have been discussed with respect to implantation of a prosthetic tricuspid valve implant, it is understood that the systems and methods may be utilized to deliver a variety of implants, including implants for repair of a heart valve. For example, other types of heart valve implants that may be utilized than are shown herein, among other types of implants (e.g., aortic valve implants and other repair implants).

The methods and systems disclosed herein may in certain embodiments not be limited to delivery of implants, but may extend to any medical intervention or insertion into a patient's body, which may include performing a medical procedure within the body. The methods and systems disclosed herein may be utilized in general use of a catheter as desired. For example, the handle shown in FIG. 61 and components disclosed therein may comprise a general catheter handle in certain embodiments. Further, the configuration of the delivery apparatus may be modified in other embodiments. For example, for an aortic valve delivery apparatus, the configuration of the implant retention area and other features of the delivery apparatus may be modified.

Although many of the embodiments herein are discussed with respect to a replacement tricuspid valve, the deflection mechanisms and other embodiments disclosed herein may be utilized for a variety of other implementations including delivery of mitral replacement valves, or aortic or pulmonary valves, or for valve repair procedures, including tricuspid or mitral valve repair or aortic or pulmonary valve repair.

From the foregoing description, it will be appreciated that an inventive product and approaches for implant delivery systems are disclosed. While several components, techniques and aspects have been described with a certain degree of particularity, it is manifest that many changes can be made in the specific designs, constructions and methodology herein above described without departing from the spirit and scope of this disclosure.

Certain features that are described in this disclosure in the context of separate implementations can also be implemented in combination in a single implementation. Conversely, various features that are described in the context of a single implementation can also be implemented in multiple implementations separately or in any suitable subcombination. Moreover, although features may be described above as acting in certain combinations, one or more features from a claimed combination can, in some cases, be excised from the combination, and the combination may be claimed as any subcombination or variation of any subcombination.

Moreover, while methods may be depicted in the drawings or described in the specification in a particular order, such methods need not be performed in the particular order shown or in sequential order, and that all methods need not be performed, to achieve desirable results. Other methods that are not depicted or described can be incorporated in the example methods and processes. For example, one or more additional methods can be performed before, after, simultaneously, or between any of the described methods. Further, the methods may be rearranged or reordered in other implementations. Also, the separation of various system components in the implementations described above should not be understood as requiring such separation in all implementations, and it should be understood that the described components and systems can generally be integrated together in a single product or packaged into multiple products. Additionally, other implementations are within the scope of this disclosure.

Conditional language, such as "can," "could," "might," or "may," unless specifically stated otherwise, or otherwise understood within the context as used, is generally intended to convey that certain embodiments include or do not include, certain features, elements, and/or steps. Thus, such conditional language is not generally intended to imply that features, elements, and/or steps are in any way required for one or more embodiments.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than or equal to 10% of, within less than or equal to 5% of, within less than or equal to 1% of, within less than or equal to 0.1% of, and within less than or equal to 0.01% of the stated amount. If the stated amount is 0 (e.g., none, having no), the above recited ranges can be specific ranges, and not within a particular % of the value. For example, within less than or equal to 10 wt./vol. % of, within less than or equal to 5 wt./vol. % of, within less than or equal to 1 wt./vol. % of, within less than or equal to 0.1 wt./vol. % of, and within less than or equal to 0.01 wt./vol. % of the stated amount.

Some embodiments have been described in connection with the accompanying drawings. The figures are drawn to scale, but such scale should not be limiting, since dimensions and proportions other than what are shown are contemplated and are within the scope of the disclosed inventions. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein. Additionally, it will be recognized that any methods described herein may be practiced using any device suitable for performing the recited steps.

While a number of embodiments and variations thereof have been described in detail, other modifications and methods of using the same will be apparent to those of skill in the art. Accordingly, it should be understood that various applications, modifications, materials, and substitutions can be made of equivalents without departing from the unique and inventive disclosure herein or the scope of the claims.

The application further comprises the following embodiments:
1) A delivery system for an implant, the delivery system comprising:
   an elongate shaft having:
      a distal end,
      an implant retention area for retaining the implant,
      a bend portion configured to deflect the distal end of the elongate shaft to a first direction, and
      a portion positioned proximal of the bend portion; and
   a deflection mechanism configured to deflect the portion that is positioned proximal of the bend portion to deflect the bend portion towards a second direction that is opposed to the first direction.
2) The delivery system of embodiment 1, wherein the distal end includes a nose cone.
3) The delivery system of embodiment 1 or embodiment 2, wherein the implant retention area is positioned between the distal end and the bend portion.
4) The delivery system of any of embodiments 1-3, wherein the bend portion is configured to deflect the implant retention area to the first direction.
5) The delivery system of any of embodiments 1-4, wherein the second direction is coplanar with the first direction.
6) The delivery system of any of embodiments 1-5, wherein the deflection mechanism includes a sheath extending over a portion of the elongate shaft.
7) The delivery system of embodiment 6, wherein the deflection mechanism includes at least one pull tether configured to deflect the sheath.
8) The delivery system of embodiment 6 or embodiment 7, wherein the sheath is configured to rotate around the portion of the elongate shaft that the sheath extends over.
9) The delivery system of any of embodiments 1-5, wherein the elongate shaft includes:
   an inner shaft,
   a rail shaft extending over the inner shaft, and
   an outer sheath extending over the rail shaft; and
   the deflection mechanism includes a pull tether coupled to the rail shaft.
10) The delivery system of embodiment 9, wherein the rail shaft includes the bend portion.
11) The delivery system of embodiment 10, wherein the rail shaft includes a second bend portion configured to deflect the distal end of the elongate shaft towards a direction that is perpendicular to the first direction.
12) The delivery system of any of embodiments 1-5, wherein the elongate shaft includes:
   an inner shaft,
   a rail shaft extending over the inner shaft and having one or more cuts thereon, and
   an outer sheath extending over the rail shaft; and
   the deflection mechanism includes a stopper on the rail shaft and a stopper on the inner shaft that is configured to apply a force to the stopper on the rail shaft to cause a deflection of the rail shaft at the one or more cuts.
13) The delivery system of embodiment 12, wherein the inner shaft is configured to be drawn proximally to cause the stopper on the inner shaft to apply the force to the stopper on the rail shaft.
14) The delivery system of any of embodiments 1-13, wherein the deflection mechanism is configured to deflect the portion that is positioned proximal of the bend portion in multiple directions.
15) The delivery system of embodiment 14, wherein at least one of the multiple directions includes a direction towards the first direction.
16) The delivery system of embodiment 14 or embodiment 15, wherein at least one of the multiple directions includes a direction perpendicular to the first direction.
17) The delivery system of any of embodiments 1-16, wherein the bend portion is a first bend portion, and the elongate shaft includes a second bend portion configured to deflect the distal end of the elongate shaft towards a direction that is perpendicular to the first direction.
18) The delivery system of embodiment 17, wherein the second bend portion is positioned between the first bend portion and the portion that is positioned proximal of the bend portion, and the deflection mechanism is configured to deflect the first bend portion and the second bend portion towards the second direction.
19) The delivery system of any of embodiments 1-18, further comprising a capsule surrounding the implant retention area.
20) The delivery system of embodiment 19, wherein the capsule is configured to be retracted to deploy the implant.
21) A delivery system for an implant, the delivery system comprising:
   an elongate shaft having:
      a distal end,
      an implant retention area for retaining the implant,
      a bend portion configured to deflect the distal end of the elongate shaft in a first plane, and
      a portion positioned proximal of the bend portion; and
   a deflection mechanism configured to deflect the portion that is positioned proximal of the bend portion in one or more planes that are not perpendicular to the first plane.
22) The delivery system of embodiment 21, wherein the deflection mechanism includes a sheath extending over at least a portion of the elongate shaft and configured to rotate around the portion of the elongate shaft that the sheath extends over.
23) The delivery system of embodiment 22, further comprising a pull tether coupled to the sheath and configured to deflect the sheath in one or more planes that are not perpendicular to the first plane.
24) The delivery system of embodiment 23, wherein the pull tether is configured to rotate around the portion of the elongate shaft that the sheath extends over to vary a plane that the portion that is positioned proximal of the bend portion is deflected in.
25) The delivery system of embodiment 21, wherein the elongate shaft includes:
   an inner shaft,
   a rail shaft extending over the inner shaft, and
   an outer sheath extending over the rail shaft; and
   the deflection mechanism includes a pull tether coupled to the rail shaft.
26) The delivery system of embodiment 25, wherein the rail shaft includes the bend portion.
27) The delivery system of embodiment 25 or embodiment 26, wherein the bend portion is a first bend portion and the rail shaft includes a second bend portion configured to deflect the distal end of the elongate shaft towards a direction that is perpendicular to the first plane.
28) The delivery system of embodiment 21, wherein the elongate shaft includes:
   an inner shaft,
   a rail shaft extending over the inner shaft and having one or more cuts thereon, and
   an outer sheath extending over the rail shaft; and
   the deflection mechanism includes a stopper on the rail shaft and a stopper on the inner shaft that is configured to apply a force to the stopper on the rail shaft to cause a deflection of the rail shaft at the one or more cuts.
29) The delivery system of embodiment 28, wherein the inner shaft is configured to be drawn proximally to cause the stopper on the inner shaft to apply the force to the stopper on the rail shaft.
30) The delivery system of any of embodiments 21-29, wherein the deflection mechanism is configured to deflect the portion that is positioned proximal of the bend portion in multiple directions.
31) A delivery system for an implant, the delivery system comprising:
   an elongate shaft having:
      a distal end,
      an implant retention area for retaining the implant,
      a first bend portion configured to deflect the distal end of the elongate shaft to a first direction,
      a second bend portion positioned proximal of the first bend portion and configured to deflect the distal end of the elongate shaft to a second direction, and
      a portion positioned proximal of the second bend portion; and
   a deflection mechanism configured to deflect the first bend portion and the second bend portion and the portion that is positioned proximal of the second bend portion.
32) The delivery system of embodiment 31, wherein the deflection mechanism includes a sheath extending over a portion of the elongate shaft.
33) The delivery system of embodiment 32, wherein the deflection mechanism includes at least one pull tether configured to deflect the sheath.
34) The delivery system of embodiment 33, wherein the at least one pull tether is configured to rotate around the portion of the elongate shaft that the sheath extends over to vary a plane that first bend portion, the second bend portion, and the portion that is positioned proximal of the second bend portion are deflected in.
35) The delivery system of embodiment 31, wherein the elongate shaft includes:
   an inner shaft,
   a rail shaft extending over the inner shaft and including the first bend portion and the second bend portion, and
   an outer sheath extending over the rail shaft; and
   the deflection mechanism includes a pull tether coupled to the rail shaft.
36) The delivery system of embodiment 31, wherein the elongate shaft includes:
   an inner shaft,
   a rail shaft extending over the inner shaft and including the first bend portion and the second bend portion and having one or more cuts thereon, and
   an outer sheath extending over the rail shaft; and
   the deflection mechanism includes a stopper on the rail shaft and a stopper on the inner shaft that is configured to apply a force to the stopper on the rail shaft to cause a deflection of the rail shaft at the one or more cuts.
37) The delivery system of embodiment 36, wherein the inner shaft is configured to be drawn proximally to cause the stopper on the inner shaft to apply the force to the stopper on the rail shaft.
38) The delivery system of any of embodiments 31-37, wherein the second direction is perpendicular to the first direction.
39) The delivery system of any of embodiments 31-38, wherein the deflection mechanism is configured to deflect the first bend portion and the second bend portion and the portion that is positioned proximal of the second bend portion towards a direction that is opposed to the first direction.
40) The delivery system of any of embodiments 31-39, wherein the deflection mechanism is configured to deflect the portion that is positioned proximal of the bend portion in multiple directions.
41) A delivery system for an implant, the delivery system comprising:
   an elongate shaft having:
   an implant retention area for retaining the implant, and
   a capsule having a distal end and surrounding the implant retention area, and the distal end of the capsule forming a distal tip of the elongate shaft.
42) The delivery system of embodiment 41, wherein the distal end of the capsule has a planar profile.
43) The delivery system of embodiment 41 or embodiment 42, wherein at least a portion of the distal end of the capsule is rounded.
44) The delivery system of any of embodiments 41-43, wherein the distal end of the capsule is configured to surround a distal portion of the implant when the implant is positioned in the implant retention area.
45) The delivery system of any of embodiments 41-44, wherein the distal end of the capsule is elastic.
46) The delivery system of any of embodiments 41-45, wherein the distal end of the capsule forms an opening for the implant to be passed through.
47) The delivery system of any of embodiments 41-46, wherein the distal end of the capsule is configured to conform to the implant when the implant is positioned in the implant retention area.
48) A delivery system for an implant, the delivery system comprising:
   an elongate shaft having:
   an implant retention area for retaining the implant, and
   a distal tip including a flexible sheath extending distally and configured to bend about a portion of a guide wire.
49) The delivery system of embodiment 48, wherein the distal tip includes a proximal portion and a distal portion, and the distal tip tapers in a direction from the proximal portion to the distal portion.
50) The delivery system of embodiment 48 or embodiment 49, wherein the elongate shaft includes a capsule extending over the implant retention area, and a tip body forms the distal tip, and the tip body is movable relative to the capsule.
51) The delivery system of embodiment 50, wherein the tip body is positioned at a distal end of the capsule.
52) The delivery system of embodiment 51, wherein an outer surface of the tip body tapers from a proximal portion of the tip body to a proximal portion of the flexible sheath.
53) The delivery system of embodiment 52, wherein the flexible sheath has a cylindrical shape from the proximal portion of the flexible sheath to a distal end of the flexible sheath.
54) The delivery system of any of embodiments 50-53, wherein the capsule is configured to retract proximally to deploy the implant from the implant retention area.
55) A delivery system for an implant, the delivery system comprising:
   an elongate shaft having:
   an implant retention area for retaining the implant, and
   a distal tip having a dome shape or a parabolic shape.
56) The delivery system of embodiment 55, wherein a distal end of the distal tip has a convex profile.
57) The delivery system of embodiment 55 or embodiment 56, wherein the elongate shaft includes a capsule extending over the implant retention area, and a tip body forms the distal tip, and the tip body is movable relative to the capsule.
58) The delivery system of embodiment 57, wherein the tip body is positioned at a distal end of the capsule.
59) The delivery system of embodiment 57 or embodiment 58, wherein an outer surface of the tip body is convex from a proximal portion of the tip body to a distal end of the tip body.
60) The delivery system of any of embodiments 57-59, wherein the capsule is configured to retract proximally to deploy the implant from the implant retention area.
61) The delivery system of any of embodiments 55-60, wherein the distal tip is configured for a guide wire to pass through.
62) A delivery system for an implant, the delivery system comprising:
   an elongate shaft having:
   a wall surrounding a channel for the implant to be passed through for deployment of the implant, the wall configured to have a bend defining a bend in the channel during deployment of the implant.
63) The delivery system of embodiment 62, wherein the channel comprises an implant retention area for retaining the implant.
64) The delivery system of embodiment 62 or embodiment 63, wherein the wall is steerable, and the system further comprises a control mechanism for steering the wall.
65) The delivery system of embodiment 64, wherein the control mechanism is configured to control a direction of the bend of the wall.
66) The delivery system of any of embodiments 62-65, wherein a port is positioned at a distal end of the channel for the implant to be passed through for the deployment of the implant.
67) The delivery system of any of embodiments 62-66, further comprising a flexible implant configured to bend within the channel transverse to an axial dimension of the flexible implant.
68) The delivery system of embodiment 67, wherein a distal end of the channel has an opening for the flexible implant to be passed through in the axial dimension for the deployment of the flexible implant.
69) A delivery system for an implant, the delivery system comprising:
   an elongate shaft having an axial dimension and having:
   an implant retention area for retaining the implant, and
   a port for the implant to be deployed from the elongate shaft in a direction transverse to the axial dimension.
70) The delivery system of embodiment 69, wherein the port is positioned on a side wall of the elongate shaft.
71) The delivery system of embodiment 69 or embodiment 70, wherein the port is adjacent the implant retention area.
72) The delivery system of any of embodiments 69-71, further comprising a deployment mechanism for deploying the implant through the port.
73) The delivery system of embodiment 72, wherein the deployment mechanism includes an inflatable body.
74) The delivery system of any of embodiments 69-73, wherein the implant retention area is configured to retain the implant with an axial dimension of the implant extending transverse to the axial dimension of the elongate shaft.
75) The delivery system of embodiment 74, further comprising an implant positioned within the implant retention area with the axial dimension of the implant extending transverse to the axial dimension of the elongate shaft.
76) A delivery system for an implant, the delivery system comprising:
   an elongate shaft having an implant retention area for retaining the implant, the elongate shaft configured to bend more than 180 degrees to form a loop.
77) The delivery system of embodiment 76, wherein the elongate shaft is configured to bend more than 200 degrees to form the loop.
78) The delivery system of embodiment 76, wherein the elongate shaft is configured to bend more than 230 degrees to form the loop.
79) The delivery system of embodiment 76, wherein the elongate shaft is configured to bend more than 250 degrees to form the loop.
80) The delivery system of embodiment 76, wherein the elongate shaft is configured to bend more than 270 degrees to form the loop.
81) The delivery system of embodiment 76, wherein the elongate shaft is configured to bend more than 180 degrees at a bend portion of the elongate shaft, the implant retention area being positioned distal of the bend portion.
82) The delivery system of any of embodiments 76-81, wherein the elongate shaft includes a port positioned distal of the implant retention area for the implant to be deployed from.
83) A delivery system for an implant, the delivery system comprising:
   an elongate shaft having:
   a capsule surrounding an implant retention area for retaining the implant, and
   a hinge coupling the capsule to a portion of the elongate shaft.
84) The delivery system of embodiment 83, wherein the capsule includes a proximal portion and a distal portion, and the proximal portion of the capsule is coupled to the hinge.
85) The delivery system of embodiment 83 or embodiment 84, wherein the capsule includes a proximal portion and a distal portion and a central portion positioned between the proximal portion and the distal portion, and the central portion of the capsule is coupled to the hinge.
86) The delivery system of any of embodiments 83-85, wherein the capsule is configured to rotate about the hinge to a rotated position, and the capsule is configured such that the implant deploys from the capsule when the capsule is in the rotated position.
87) The delivery system of any of embodiments 83-86, wherein the capsule includes a port for the implant to be deployed from when the capsule is in the rotated position.
88) The delivery system of embodiment 86 or embodiment 87, further comprising a deployment mechanism for deploying the implant from the capsule when the capsule is in the rotated position.
89) The delivery system of any of embodiments 83-88, further comprising a control mechanism for controlling rotation of the capsule about the hinge.
90) A delivery system for an implant, the delivery system comprising:
   an elongate shaft extending along an axis and having an outer surface and an implant retention area for retaining the implant; and
   one or more support bodies configured to extend radially outward from the outer surface of the elongate shaft and contact an external surface to resist deflection of the elongate shaft transverse to the axis.
91) The delivery system of embodiment 90, further comprising a sheath extending over the outer surface of the elongate shaft, and the one or more support bodies are configured to extend radially outward from the sheath.
92) The delivery system of embodiment 90 or embodiment 91, wherein the elongate shaft includes a bend portion configured to deflect the implant retention area to a first direction, and the one or more support bodies are configured to extend radially outward from the outer surface proximal of the bend portion.
93) The delivery system of embodiment 92, wherein the bend portion is a first bend portion, and the elongate shaft includes a second bend portion positioned proximal of the first bend portion and configured to deflect the implant retention area to a second direction that is transverse to the first direction, and the one or more support bodies are configured to extend radially outward from the outer surface proximal of the second bend portion.
94) The delivery system of any of embodiments 90-93, wherein the one or more support bodies are configured to move to an expanded state from an unexpanded state.
95) The delivery system of any of embodiments 90-94, wherein the one or more support bodies include one or more inflatable bodies.
96) The delivery system of any of embodiments 90-95, wherein the one or more support bodies include one or more mesh bodies.
97) The delivery system of any of embodiments 90-96, wherein the one or more support bodies include one or more disks.
98) The delivery system of any of embodiments 90-97, wherein the one or more support bodies include one or more occluders.
99) The delivery system of any of embodiments 90-94, wherein the one or more support bodies include one or more arms.
100) Any of the foregoing delivery systems, further comprising:
   at least one motor configured to actuate at least a portion of the delivery system; and
   a processor configured to operate the at least one motor to actuate at least the portion of the delivery system.
101) The delivery system of embodiment 100, further comprising one or more sensors configured to sense one or more of a condition of the patient's body or a condition of the delivery system, and wherein the processor is configured to operate the at least one motor to actuate at least the portion of the delivery system based on a signal from the one or more sensors.
102) The delivery system of embodiment 101, wherein the processor is configured to provide an output based on the one or more of the condition of the patient's body or the condition of the delivery system sensed by the one or more sensors.
103) A system comprising:
   a prosthetic heart valve configured for implantation within a patient's valve annulus;
   an anchor configured to be secured within a portion of the patient's body; and
   a tether configured to couple the prosthetic heart valve to the anchor.
104) The system of embodiment 103, wherein the prosthetic heart valve includes prosthetic valve flaps.
105) The system of embodiment 103 or embodiment 104, wherein the prosthetic heart valve includes a plurality of anchors configured to extend over heart valve flaps.
106) The system of any of embodiments 103-105, wherein the anchor comprises a stent.
107) The system of embodiment 106, wherein the stent is configured to be secured in one or more of an inferior vena cava or a superior vena cava.
108) The system of any of embodiments 103-105, wherein the anchor is configured to be secured to a wall of the patient's right ventricle.
109) The system of any of embodiments 103-105, wherein the anchor is configured to be secured to a moderator band of the patient's right ventricle.
110) The system of embodiment 109, wherein the anchor comprises one or more of a hook, a barb, a cover, a loop, or an expandable body.
111) The system of any of embodiments 103-110, wherein the prosthetic heart valve comprises a prosthetic tricuspid heart valve.
112) A prosthetic valve for replacement of a patient's native valve, the prosthetic valve comprising:
   a prosthetic heart valve body configured to be anchored within an annulus of the patient's native valve and forming a prosthetic valve annulus; and
   a port coupled to the prosthetic heart valve body and configured to receive a diagnostic or therapeutic device.
113) The prosthetic valve of embodiment 112, wherein the port includes a tube for the diagnostic or therapeutic device to be passed through.
114) The prosthetic valve of embodiment 113, wherein the tube is made of one or more of a textile, a woven material, or a polymer.
115) The prosthetic valve of any of embodiments 113-114, wherein the tube includes a valve for the diagnostic or therapeutic device to be passed through.
116) The prosthetic valve of any of embodiments 112-115, wherein the prosthetic heart valve body includes an outer frame body and an inner frame body, and the port is positioned on the outer frame body.
117) The prosthetic valve of embodiment 116, wherein the port is positioned on a covering extending over an outer frame of the outer frame body.
118) The prosthetic valve of embodiment 116 or embodiment 117, wherein the port comprises a tearable portion of the prosthetic heart valve body.
119) The prosthetic valve of any of embodiments 112-118, wherein the port is configured to form a seal with the diagnostic or therapeutic device.
120) The prosthetic valve of any of embodiments 112-119, wherein the port is positioned exterior of the prosthetic valve annulus.
121) The prosthetic valve of any of embodiments 112-120, wherein an imaging marker identifies a location of the port.
122) The prosthetic valve of any of embodiments 112-121, wherein the diagnostic or therapeutic device comprises a pacemaker pacing lead.
123) A method for treating a patient's tricuspid valve, comprising:
   passing a delivery apparatus for an implant into the patient's right atrium; and
   deploying the implant to the patient's tricuspid valve.
124) The method of embodiment 123, wherein the delivery apparatus includes an elongate shaft having an implant retention area for retaining the implant.
125) The method of embodiment 124, wherein the delivery apparatus includes a deployment port for the implant to be deployed from, and the method further comprises deflecting the delivery apparatus within an inferior vena cava or a superior vena cava to vary a height of the deployment port from the patient's tricuspid valve.
126) The method of embodiment 124 or embodiment 125, wherein the elongate shaft includes a bend portion configured to direct a distal portion of the elongate shaft towards the patient's tricuspid valve.
127) The method of embodiment 126, further comprising varying a height of the bend portion from the patient's tricuspid valve.
128) The method of embodiment 126 or embodiment 127, further comprising deflecting the bend portion and a portion of the elongate shaft that is proximal the bend portion in a direction away from the patient's tricuspid valve.
129) The method of any of embodiments 124-128, wherein a sheath is positioned over the elongate shaft and the method further comprises deflecting a portion of the elongate shaft using the sheath.
130) The method of embodiment 129, further comprising rotating the sheath to vary a direction of deflection of the elongate shaft.
131) The method of embodiment 124, wherein the elongate shaft includes an outer sheath extending over a rail shaft, with the outer sheath configured to slide relative to the rail shaft to vary a distance of the implant retention area from the patient's tricuspid valve.
132) The method of any of embodiments 124-131, wherein the elongate shaft includes a capsule having a distal end and that surrounds the implant retention area, and the distal end of the capsule forms a distal tip of the elongate shaft.
133) The method of any of embodiments 124-131, wherein the elongate shaft includes a distal tip including a flexible sheath extending distally and configured to bend about a portion of a guide wire.
134) The method of embodiment 133, further comprising bending the flexible sheath about the portion of the guide wire when the guide wire is positioned in the patient's right ventricle.
135) The method of any of embodiments 124-132, wherein the elongate shaft includes a distal tip having a dome shape or a parabolic shape.
136) The method of any of embodiments 124-135, wherein the implant is a prosthetic tricuspid valve, and the method further comprises passing the prosthetic tricuspid valve through a bent deployment channel of the elongate shaft to deploy the prosthetic tricuspid valve.
137) The method of embodiment 136, wherein the prosthetic tricuspid valve has an axial dimension and the prosthetic tricuspid valve bends transverse to the axial dimension when passing through the bent deployment channel.
138) The method of any of embodiments 124-137, wherein the elongate shaft has an axial dimension, and the method further comprises deploying the implant through a port of the elongate shaft in a direction transverse to the axial dimension.
139) The method of any of embodiments 124-138, further comprising bending the elongate shaft more than 180 degrees to form a loop.
140) The method of embodiment 139, wherein the loop is at least partially positioned within the patient's right atrium.
141) The method of any of embodiments 124-140, wherein the elongate shaft includes a capsule surrounding an implant retention area for retaining the implant and a hinge coupling the capsule to a portion of the elongate shaft.
142) The method of embodiment 141, further comprising pivoting the capsule about the hinge.
143) The method of any of embodiments 123-142, wherein at least a portion of the delivery apparatus is actuated by at least one motor operated by a processor.
144) The method of any of embodiments 123-143, wherein the delivery apparatus includes one or more sensors coupled to the delivery apparatus and configured to sense one or more of a condition of a patient's body or a condition of the delivery apparatus.
145) A method for treating a patient's tricuspid valve, the method comprising:
   deploying a prosthetic heart valve within a patient's tricuspid valve annulus;
   deploying an anchor to a portion within a patient's body; and
   providing a tether coupling the prosthetic heart valve to the anchor.
146) The method of embodiment 145, further comprising coupling the anchor to the prosthetic heart valve with the tether.
147) The method of embodiment 145 or embodiment 146, wherein the anchor comprises a stent.
148) The method of embodiment 147, further comprising securing the stent within an inferior vena cava or a superior vena cava of the patient.
149) The method of embodiment 145 or embodiment 146, further comprising securing the anchor to a moderator band of the patient's right ventricle.
150) The method of embodiment 149, wherein the anchor comprises one or more of a hook, a barb, a cover, a loop, or an expandable body.
151) The method of embodiment 145 or embodiment 146, wherein the anchor is configured to be secured to a wall of the patient's right ventricle.
152) The method of any of embodiments 145-151, further comprising anchoring the prosthetic heart valve to heart valve flaps of the patient's heart valve.
153) A method comprising:
   passing a diagnostic or therapeutic device through a port positioned on a prosthetic heart valve body, the prosthetic heart valve body forming a prosthetic valve annulus.
154) The method of embodiment 153, wherein the prosthetic heart valve body is positioned in a tricuspid heart valve annulus of a patient, and the diagnostic or therapeutic device is passed through the port to the patient's right ventricle.
155) The method of embodiment 153 or embodiment 154, wherein the port includes a tube for the diagnostic or therapeutic device to be passed through.
156) The method of embodiment 155, wherein the tube is made of one or more of a textile, a woven material, or a polymer.
157) The method of embodiment 155, wherein the tube includes a valve for the diagnostic or therapeutic device to be passed through.
158) The method of embodiment 153, wherein the port comprises a tearable portion of the prosthetic heart valve body.
159) The method of any of embodiments 153-158, wherein the prosthetic heart valve body includes an outer frame body and an inner frame body, and the port is positioned on the outer frame body.
160) The method of embodiment 159, wherein the port is positioned on a covering extending over an outer frame of the outer frame body.
161) The method of any of embodiments 153-160, wherein the port forms a seal with the diagnostic or therapeutic device.
162) The method of embodiment 153-161, wherein the port is positioned exterior of the prosthetic valve annulus.
163) The method of embodiment 153-162, further comprising imaging an imaging marker on the prosthetic heart valve body to identify a location of the port.
164) The method of embodiment 153-163, wherein the diagnostic or therapeutic device comprises a pacemaker pacing lead.
165) A method comprising:
   coupling a pacemaker pacing lead to a prosthetic heart valve body positioned within a patient's heart valve annulus to provide electrical energy through the pacemaker pacing lead and through the prosthetic heart valve body to pace functioning of the patient's heart.
166) The method of embodiment 165, wherein the prosthetic heart valve body includes a frame, and the method includes providing electrical energy through the frame.
167) The method of embodiment 166, wherein the frame is in contact with the patient's heart valve.
168) The method of any of embodiments 165-167, wherein the prosthetic heart valve body includes one or more electrical terminals in contact with a portion of the patient's heart.
169) The method of any of embodiments 165-168, further comprising providing the electrical energy through the pacemaker pacing lead and through the prosthetic heart valve body to pace functioning of the patient's heart.
170) The method of any of embodiments 165-169, further comprising deploying the prosthetic heart valve body to the patient's heart valve annulus.
171) The method of any of embodiments 165-170, further comprising expanding the prosthetic heart valve body within the patient's heart valve annulus.
172) The method of any of embodiments 165-171, further comprising anchoring the prosthetic heart valve body to heart valve flaps of the patient's heart valve.
173) The method of any of embodiments 165-172, further comprising contacting the prosthetic heart valve body to the patient's heart valve.
174) The method of any of embodiments 165-173, wherein the prosthetic heart valve body is positioned within a tricuspid heart valve annulus or a mitral heart valve annulus.
175) A method comprising:
   delivering a delivery apparatus for an implant into a portion of a patient's heart, the delivery apparatus including an elongate shaft extending along an axis and having an outer surface;
   expanding one or support bodies radially outward from the outer surface of the elongate shaft; and
   contacting the one or more support bodies to a surface external of the delivery apparatus to resist deflection of the elongate shaft transverse to the axis.
176) The method of embodiment 175, wherein the surface is an atrial wall.
177) The method of embodiment 175 or embodiment 176, wherein the surface is a wall of an interatrial septum.
178) The method of any of embodiments 175-177, wherein the delivery apparatus is positioned within an atrium of the patient's heart.
179) The method of any of embodiments 175-178, further comprising deploying the implant to a mitral valve or a tricuspid valve of the patient's heart.
180) The method of any of embodiments 175-179, wherein the delivery apparatus includes an implant retention area and a bend portion, and the method further comprises expanding the one or more support bodies proximal of the bend portion and deflecting the implant retention area at the bend portion to a first direction.
181) The method of embodiment 180, wherein the bend portion is a first bend portion, and the method further comprising deflecting the implant retention area at a second bend portion positioned proximal of the first bend portion to a second direction that is transverse to the first direction.
182) The method of any of embodiments 175-181, wherein the one or more support bodies include one or more inflatable bodies.
183) The method of any of embodiments 175-182, wherein the one or more support bodies include one or more mesh bodies.
184) The method of any of embodiments 175-181, wherein the one or more support bodies include one or more arms.

## Claims

1. A prosthetic valve (1500, 1600) for replacement of a patient's native valve, the prosthetic valve (1500, 1600) comprising:
a prosthetic heart valve body configured to be anchored within an annulus of the patient's native valve and forming a prosthetic valve annulus; and
a port (1591) coupled to the prosthetic heart valve body and configured to receive a diagnostic or therapeutic device.

2. The prosthetic valve (1500, 1600) of claim 1, wherein the port (1591) includes a tube for the diagnostic or therapeutic device to be passed through.

3. The prosthetic valve (1500, 1600) of claim 2, wherein the tube is made of one or more of a textile, a woven material, or a polymer.

4. The prosthetic valve (1500, 1600) of any of claims 2 or 3, wherein the tube includes a valve (2310) for the diagnostic or therapeutic device to be passed through.

5. The prosthetic valve (1500, 1600) of any of claims 2 to 4, wherein the tube extends along a height of the prosthetic valve (1500, 1600) to guide the diagnostic or therapeutic device through the prosthetic valve (1500, 1600).

6. The prosthetic valve (1500, 1600) of any of claims 2 to 5, wherein the tube has a cylindrical shape.

7. The prosthetic valve (1500, 1600) of any of claims 2 to 6, wherein the tube has a shape with opposed inverted funnels.

8. The prosthetic valve (1500, 1600) of any of claims 2 to 7, wherein the tube includes an entry opening (1592) and an exit opening (1593) with a central lumen (1594) extending between the entry opening (1592) and the exit opening (1593),
wherein the exit opening (1593) is preferably positioned on a bottom of the prosthetic valve (1600).

9. The prosthetic valve (1500, 1600) of any of claims 1 to 8, wherein the port (1591) extends from an upper surface of the valve (1500, 1600).

10. The prosthetic valve (1500, 1600) of any of claims 1 to 9, wherein the prosthetic heart valve body includes an outer frame body (1542) and an inner frame body (1522), and the port (1591) is positioned on the outer frame body (1542).

11. The prosthetic valve (1500, 1600) of claim 9, wherein the port (1591) is positioned on a covering (1580) extending over an outer frame (1540) of the outer frame body (1542).

12. The prosthetic valve (1500, 1600) of claim 10 or claim 11, wherein the port (1591) comprises a tearable portion of the prosthetic heart valve body.

13. The prosthetic valve (1500, 1600) of any of claims 10 to 12, wherein the outer frame body (1642) includes a plurality of struts with at least some of the struts forming cells (1646a, 1646c),
wherein the port (1591) passes within opening of the outer frame (1540) and between struts of the outer frame (1540).

14. The prosthetic valve (1500, 1600) of any of claims 1 to 13, wherein the port (1591) is configured to form a seal with the diagnostic or therapeutic device.

15. The prosthetic valve (1500, 1600) of any of claims 1 to 14, wherein the port (1591) is positioned exterior of the prosthetic valve annulus, and/or
wherein an imaging marker (1597) identifies a location of the port (1591).
